# EUROPEAN PATENT APPLICATION

(11) **EP 4 180 429 A1**
(43) Date of publication of application: **17.05.2023**
(21) Application number: 21837472.6
(22) Date of filing: 25.06.2021
(51) Int. Cl.: C07D 471/04, A61K 31/519, A61P 31/06

(54) **PYRIMIDINONE DERIVATIVE, AND PREPARATION METHOD THEREFOR AND USE THEREOF AGAINST MYCOBACTERIUM TUBERCULOSIS INFECTION**

(30) Priority: 04.07.2020 CN 202010633523
(71) Applicant: Institute of Medicinal Biotechnology, Chinese Academy of Medical Sciences, Beijing 100050 (CN)
(72) Inventor: SUN, Lianqi, Beijing 100050 (CN); MENG, Jianzhou, Beijing 100050 (CN); LI, Zhuorong, Beijing 100050 (CN); LIU, Yishuang, Beijing 100050 (CN)
(74) Representative: Michalski Hüttermann & Partner Patentanwälte mbB
(86) International application number: PCT/CN2021/000132
(87) International publication number: WO 2022/007372

(57) **Abstract**

Provided are a pyrimidinone derivative, and a preparation method therefor and the use thereof against Mycobacterium tuberculosis infection. The structure of the pyrimidinone derivative is represented by formula I. Experiments prove that the pyrimidinone derivative has obvious anti-Mycobacterium tuberculosis activity and is particularly suitable for preparing a drug for preventing and/or treating related diseases caused by Mycobacterium tuberculosis.

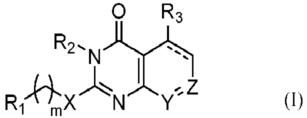

## Description

### CROSS-REFERENCE TO RELATED APPLICATIONS

This application claims the benefit of priority of Chinese Patent Application No. 202010633523.5 filed on July 04, 2020, which is incorporated herein by reference in its entirety for all purposes.

### TECHNICAL FIELD

The present invention belongs to the field of pharmaceutical compounds. Specifically, the present invention relates to a novel pyrimidinone derivative, or an enantiomer, diastereomer, raceme or a mixture thereof, and a pharmaceutically acceptable salt thereof, to a preparation method for the above compound, and to the use of the above-mentioned compound in the preparation of an anti-mycobacterium tuberculosis drug.

### BACKGROUND

Tuberculosis (TB) is a very old infectious disease caused by mycobacterium tuberculosis (MTB) infection. This disease is usually caused by mycobacterium tuberculosis infection, and destroys the lungs and other systems and organs of the human body, forming tubercle, infiltrates, caseation or cavities. Common clinical manifestations include prolonged low-grade fever, expectoration and hemoptysis, etc. [Wang Y, Luan S, Zhou X, Fan S, Research progress on anti-tuberculosis drugs, Clinical Medication Journal, 2018, 16(4), 9-13].

Tuberculosis is one of the most widespread, infectious and fatal diseases at present, which leads to tens of millions of new TB-infected cases and death of more than one million people every year [Campanico A, Moreira R, Lopes F, Drug discovery in tuberculosis. New drug targets and antimycobacterial agents, Eur. J. Med. Chem. 2018, 150, 525-545]. The emergence and wide spread of drug-resistant TB (DR-TB), multidrug-resistant TB (MDR-TB, cure rate: 40% to 80%), extensively drug-resistant TB (XDR-TB), totally drug-resistant TB(TDR-TB) and TB and HIV co-infection have led to the increasingly severe situation of TB prevention and control in the world.

On September 18, 2018, the World Health Organization said in Global Tuberculosis Report 2018 announced at the United Nations headquarters in New York that in 2017, an estimated 10 million people had been infected with tuberculosis and 1.6 million of them died from tuberculosis around the world. From 2013 to 2017, the number of new TB cases in the world decreased by 2% annually. In 2017, the number of patients and deaths was the lowest in recent years. However, TB remains the deadliest infectious disease in the world. A report pointed out that among 10 million patients estimated by the WHO, only 6.4 million patients had been recorded in the national reporting system. The report also said that drug-resistant tuberculosis is a major problem in the global health field, and the cure rate of MDR-TB is only 55% [World Health Organization, Global Tuberculosis Report, 2018, http://www.who.int/tb/].

The global situation of tuberculosis is very serious. According to the current trend, it is difficult to achieve the goal set by of 2030 Agenda for Sustainable Development of the United Nations, that is, to eliminate tuberculosis by 2030. In order to change this situation, in addition to improving the tuberculosis reporting mechanism, the main measure is to enhance the treatment of drug-resistant TB and increase the investment in research and development so as to produce more effective anti-tuberculosis drugs.

In the past half century, only three novel drugs have been developed. Compared with the fiercely competitive race of anti-tumor drugs such as PD-1, the research and development pipeline for anti-tuberculosis drugs is almost exhausted, the accessibility of novel drugs is low, and the enthusiasm of generic drug enterprises is not high. This is a public health challenge faced by countries with high burden of tuberculosis including China. Tuberculosis has become a global public health issue, so it is urgent to develop a novel anti-tuberculosis drug with definite curative effect and better compliance for the treatment of tuberculosis. In view of the current severe situation of drug resistance of tuberculosis, it is also urgent to develop an anti-tuberculosis drug with a novel structural type or a new mechanism of action.

### SUMMARY OF THE INVENTION

The object of the present invention is to provide a novel anti-tuberculosis drug and its preparation method and use thereof.

In order to achieve the above object, the present invention adopts the following technical means:

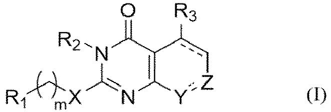

The present invention provides a compound shown as formula I, an enantiomer, diastereomer, raceme or their mixture thereof, or a pharmaceutically acceptable salt thereof,

Wherein, R₁ is a saturated hydrocarbyl group, an unsaturated hydrocarbyl group, a carboxyl group, a hydroxyl group, a C₁-C₆ alkoxy group, a substituted or unsubstituted amino group, halogen, a nitro group, a cyano group, or a substituted or unsubstituted amide group, wherein a substituent in the substituted amino group is selected from hydrogen, a C₁-C₆ alkyl group, a carboxyl group, a hydroxyl group, an amino group, a C₁-C₆ alkoxy group, a nitro group, a cyano group, or a trifluoromethyl group;
m is 0, 1, or 2;
X is sulfur or oxygen;
if there is a single bond between Y and Z, Y is -CH₂- or -N(R)-, and R is selected from hydrogen, a saturated hydrocarbyl group, an unsaturated hydrocarbyl group, a carboxyl group, a hydroxyl group, a C₁-C₆ alkoxy group, a substituted or unsubstituted amino group, halogen, a nitro group, a cyano group, or a substituted or unsubstituted amide group; Z is 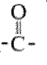 or -CH₂-; wherein the substituent in the substituted amino group is selected from hydrogen, a C₁-C₆ alkyl group, a carboxyl group, a hydroxyl group, an amino group, a C₁-C₆ alkoxy group, a nitro group, a cyano group, or a trifluoromethyl group; if there is a double bond between Y and Z, Y is N and Z is CH;
R₂ is hydrogen, a saturated hydrocarbyl group, an unsaturated hydrocarbyl group, a carboxyl group, a hydroxyl group, a C₁-C₆ alkoxy group, a substituted or unsubstituted amino group, halogen, a nitro group, a cyano group, or a substituted or unsubstituted amide group; wherein a substituent in the substituted amino group is selected from hydrogen, a C₁-C₆ alkyl group, a carboxyl group, a hydroxyl group, an amino group, a C₁-C₆ alkoxy group, a nitro group, a cyano group, or a trifluoromethyl group;
R₃ is hydrogen, a substituted or unsubstituted C₁-C₆ alkyl group, a C₁-C₆ alkoxy group, a carboxyl group, a nitro group, halogen, a trifluoromethyl group, a cyano group, a protecting group-protected or -unprotected hydroxyl group, a protecting group-protected or -unprotected amino group, a substituted or unsubstituted phenyl group or fused ring group, or a substituted or unsubstituted heterocyclyl group; wherein a substituent in the substituted alkyl group is selected from a carboxyl group, a hydroxyl group, an amino group, halogen, a nitro group, a cyano group, or a mercapto group; wherein the substituted phenyl or fused ring group has 1, 2, 3, 4 or 5 substituents, the substituent being selected from hydrogen, a C₁-C₆ alkyl group, a carboxyl group, a hydroxyl group, an amino group, a C₁-C₆ alkoxy group, a nitro group, a cyano group, or a trifluoromethyl group; wherein the heterocyclyl group is a 3-membered, 4-membered or 5-membered heterocyclyl group; a heteroatom is O, S, or N; if the heterocyclyl group has one or more substituents, the substituent is selected from hydrogen, a C₁-C₆ alkyl group, a carboxyl group, a hydroxyl group, an amino group, a C₁-C₆ alkoxy group, a nitro group, a cyano group, or a trifluoromethyl group; wherein the protecting group is selected from a *tert*-butoxycarbonyl group, a p-methoxybenzyl group, a dibenzyl group, a benzyl group, a *tert*-butyldimethylsilyl group, a tert-butyldiphenylsilyl group, an allyl group, a methoxymethyl group, a methylthiomethyl group, a methoxyethoxymethyl group, or a benzyloxymethyl group.

Wherein, preferably, the configuration of each chiral carbon in the compound shown as formula I is independently an R configuration or an S configuration.

Wherein, preferably, the pharmaceutically acceptable salt is hydrochloride, hydrobromide, sulfate, nitrate, phosphate, citrate, mesylate, trifluoroacetate, acetate, oxalate, succinate, malate, tosylate, tartrate, fumarate, glutamate, glucuronate, lactate, glutarate, arginine salt, or maleate.

Wherein, preferably, in the formula, R₁ is a methyl group or a vinyl group;
m is 0, 1, or 2;
X is sulfur;
if there is a single bond between Y and Z, Y is -NH-; Z is 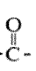 or -CH₂-; if there is a double bond between Y and Z, Y is N; Z is CH;
R₂ is hydrogen; and
R₃ is a 4-propoxyphenyl group, a 3-hydroxy-4-methoxyphenyl group, a 3,4-dihydroxyphenyl group, a 3,4,5-trimethoxyphenyl group, a 3,4-dimethoxyphenyl group, a 3-nitro-4-chlorophenyl group, a 4-nitrophenyl group, a 2-hydroxy-4-methoxyphenyl group, a 4-hydroxyphenyl group, a 2,6-dimethylphenyl group, a 3-nitrophenyl group, a 2,4-dihydroxyphenyl group, a 4-propoxyphenyl group, a 4-iodophenyl group, a 4-methoxyphenyl group, a 2,3-dihydroxy-4-methoxyphenyl group, a 3-hydroxy-4-propoxyphenyl group, a 4-bromophenyl group, a 4-fluorophenyl group, a 2-hydroxy-4-propoxyphenyl group, a 4-benzyloxyphenyl group, a 4-phenoxyphenyl group, a 3,4,5-trihydroxyphenyl group, a 4-trifluoromethoxyphenyl group, a 4-ethoxyphenyl group, a 4-methoxy-3-nitrophenyl group, a 4-methoxy-2-nitrophenyl group, a 3-methoxyphenyl group, a 2-methoxyphenyl group, a 3-hydroxyphenyl group, a 3-cyanophenyl group, a 2-nitrophenyl group, a 4-hydroxy-3-nitrophenyl group, a phenyl group, a quinolin-4-yl group, a quinolin-5-yl group, a quinolin-6-yl group, or a 4-methoxyphenyl group.

Wherein, preferably, the compound shown as formula I is:
2-propylmercapto-5-(4-propoxyphenyl)-5,8-dihydropyrido[2,3-*d*]pyrimidin-4,7(*3H*,*6H*)-dione;
2-methylmercapto-5-(4-propoxyphenyl)-5,8-dihydropyrido[2,3-*d*]pyrimidin-4,7(*3H*,*6H*)-dione;
2-propylmercapto-5-(3-hydroxy-4-methoxyphenyl)-5,8-dihydropyrido[2,3-*d*]pyrimidin-4,7(*3H*,*6H*)-dione;
2-methylmercapto-5-(3-hydroxy-4-methoxyphenyl)-5,8-dihydropyrido[2,3-*d*]pyrimidin-4,7(*3H*,*6H*)-dione;
2-propylmercapto-5-(3,4-dihydroxyphenyl)-5,8-dihydropyrido[2,3-*d*]pyrimidin-4,7(*3H*,*6H*)-dione;
2-methylmercapto-5-(3,4,5-trimethoxyphenyl)-5,8-dihydropyrido[2,3-*d*]pyrimidin-4,7(*3H*,*6H*)-dione;
2-propylmercapto-5-(3,4,5-trimethoxyphenyl)-5,8-dihydropyrido[2,3-*d*]pyrimidin-4,7(*3H*,*6H*)-dione;
2-propylmercapto-5-(3,4-dimethoxyphenyl)-5,8-dihydropyrido[2,3-*d*]pyrimidin-4,7(*3H*,*6H*)-dione;
2-propylmercapto-5-(3-nitro-4-chlorophenyl)-5,8-dihydropyrido[2,3-*d*]pyrimidin-4,7(*3H*,*6H*)-dione;
2-methylmercapto-5-(4-nitrophenyl)-5,8-dihydropyrido[2,3-*d*]pyrimidin-4,7(*3H*,*6H*)-dione;
2-propylmercapto-5-(4-nitrophenyl)-5,8-dihydropyrido[2,3-*d*]pyrimidin-4,7(*3H*,*6H*)-dione;
2-methylmercapto-5-(2-hydroxy-4-methoxyphenyl)-5,8-dihydropyrido[2,3-*d*]pyrimidin-4,7(*3H*,*6H*)-dione;
2-propylmercapto-5-(2-hydroxy-4-methoxyphenyl)-5,8-dihydropyrido[2,3-*d*]pyrimidin-4,7(*3H*,*6H*)-dione;
2-propylmercapto-5-(4-hydroxyphenyl)-5,8-dihydropyrido[2,3-*d*]pyrimidin-4,7(*3H*,*6H*)-dione;
2-methylmercapto-5-(2,6-dimethylphenyl)-5,8-dihydropyrido[2,3-*d*]pyrimidin-4,7(*3H*,*6H*)-dione;
2-propylmercapto-5-(2,6-dimethylphenyl)-5,8-dihydropyrido[2,3-*d*]pyrimidin-4,7(*3H*,*6H*)-dione;
2-methylmercapto-5-(3-nitrophenyl)-5,8-dihydropyrido[2,3-*d*]pyrimidin-4,7(*3H*,*6H*)-dione;
2-propylmercapto-5-(3-nitrophenyl)-5,8-dihydropyrido[2,3-*d*]pyrimidin-4,7(*3H*,*6H*)-dione;
2-propylmercapto-5-(2,4-dihydroxyphenyl)-5,8-dihydropyrido[2,3-*d*]pyrimidin-4,7(*3H*,*6H*)-dione;
2-allylmercapto-5-(4-propoxyphenyl)-5,8-dihydropyrido[2,3-*d*]pyrimidin-4,7(*3H*,*6H*)-dione;
2-allylmercapto-5-(3-hydroxy-4-oxyphenyl)-5,8-dihydropyrido[2,3-*d*]pyrimidin-4,7(3H,6H)-dione;
2-allylmercapto-5-(2-hydroxy-4-propoxyphenyl)-5,8-dihydropyrido[2,3-*d*]pyrimidin-4,7(*3H*,*6H*)-dione;
2-allylmercapto-5-(3,4-dimethoxyphenyl)-5,8-dihydropyrido[2,3-*d*]pyrimidin-4,7(*3H*,*6H*)-dione;
2-propylmercapto-5-(4-iodophenyl)-5,8-dihydropyrido[2,3-*d*]pyrimidin-4,7(*3H*,*6H*)-dione;
2-propylmercapto-5-(4-methoxyphenyl)-5,8-dihydropyrido[2,3-*d*]pyrimidin-4,7(*3H*,*6H*)-dione;
2-propylmercapto-5-(2,3-dihydroxy-4-methoxyphenyl)-5,8-dihydropyrido[2,3-*d*]pyrimidin-4,7(*3H*,*6H*)-dione;
2-propylmercapto-5-(3-hydroxy-4-propoxyphenyl)-5,8-dihydropyrido[2,3-*d*]pyrimidin-4,7(*3H*,*6H*)-dione;
2-propylmercapto-5-(4-bromophenyl)-5,8-dihydropyrido[2,3-*d*]pyrimidin-4,7(*3H*,*6H*)-dione;
2-propylmercapto-5-(4-fluorophenyl)-5,8-dihydropyrido[2,3-*d*]pyrimidin-4,7(*3H*,*6H*)-dione;
2-propylmercapto-5-(2-hydroxy-4-propoxyphenyl)-5,8-dihydropyrido[2,3-*d*]pyrimidin-4,7(*3H*,*6H*)-dione;
2-propylmercapto-5-(4-benzyloxyphenyl)-5,8-dihydropyrido[2,3-*d*]pyrimidin-4,7(*3H*,*6H*)-dione;
2-propylmercapto-5-(4-phenoxyphenyl)-5,8-dihydropyrido[2,3-*d*]pyrimidin-4,7(*3H*,*6H*)-dione;
2-propylmercapto-5-(3,4,5-trihydroxyphenyl)-5,8-dihydropyrido[2,3-*d*]pyrimidin-4,7(*3H*,*6H*)-dione;
2-propylmercapto-5-(4-trifluoromethoxyphenyl)-5,8-dihydropyrido[2,3-*d*]pyrimidin-4,7(*3H*,*6H*)-dione;
2-propylmercapto-5-(quinolin-6-yl)-5,8-dihydropyrido[2,3-*d*]pyrimidin-4,7(*3H*,*6H*)-dione;
2-propylmercapto-5-(4-methylphenyl)-5,8-dihydropyrido[2,3-*d*]pyrimidin-4,7(*3H*,*6H*)-dione;
2-propylmercapto-5-(4-trifluoromethylphenyl)-5,8-dihydropyrido[2,3-*d*]pyrimidin-4,7(*3H*,*6H*)-dione;
2-propylmercapto-5-(4-ethoxyphenyl)-5,8-dihydropyrido[2,3-*d*]pyrimidin-4,7(*3H*,*6H*)-dione;
2-propylmercapto-5-(3-nitro-4-methoxyphenyl)-5,8-dihydropyrido[2,3-*d*]pyrimidin-4,7(*3H*,*6H*)-dione;
2-propylmercapto-5-(2-nitro-4-methoxyphenyl)-5,8-dihydropyrido[2,3-*d*]pyrimidin-4,7(*3H*,*6H*)-dione;
2-propylmercapto-5-(3-methoxyphenyl)-5,8-dihydropyrido[2,3-*d*]pyrimidin-4,7(*3H*,*6H*)-dione;
2-propylmercapto-5-(2-methoxyphenyl)-5,8-dihydropyrido[2,3-*d*]pyrimidin-4,7(*3H*,*6H*)-dione;
2-propylmercapto-5-(3-hydroxyphenyl)-5,8-dihydropyrido[2,3-*d*]pyrimidin-4,7(*3H*,*6H*)-dione;
2-propylmercapto-5-(3-cyanophenyl)-5,8-dihydropyrido[2,3-*d*]pyrimidin-4,7(*3H*,*6H*)-dione;
2-propylmercapto-5-(2-nitrophenyl)-5,8-dihydropyrido[2,3-*d*]pyrimidin-4,7(*3H*,*6H*)-dione;
2-propylmercapto-5-(3-nitro-4-hydroxyphenyl)-5,8-dihydropyrido[2,3-*d*]pyrimidin-4,7(*3H*,*6H*)-dione;
2-propylmercapto-5-phenyl-5,8-dihydropyrido[2,3-*d*]pyrimidin-4,7(*3H*,*6H*)-dione;
2-propylmercapto-5-(quinolin-4-yl)-5,8-dihydropyrido[2,3-*d*]pyrimidin-4,7(*3H*,*6H*)-dione;
2-propylmercapto-5-(quinolin-5-yl)-5,8-dihydropyrido[2,3-*d*]pyrimidin-4,7(*3H*,*6H*)-dione;
2-propylmercapto-5-(4-methoxyphenyl)-pyrido[2,3-*d*]pyrimidin-4(*3H*)-one;
2-propylmercapto-5-(quinolin-6-yl)-pyrido[2,3-*d*]pyrimidin-4(*3H*)-one;
2-propylmercapto-5-(4-methoxyphenyl)-pyrido[2,3-*d*]pyrimidin-4,7(*3H*,*6H*)-dione;
2-propylmercapto-5-(3-amino-4-methoxyphenyl)-5,8-dihydropyrido[2,3-*d*]pyrimidin-4,7(*3H*,*6H*)-dione;
2-propylmercapto-5-(3-amino-4-chlorophenyl)-5,8-dihydropyrido[2,3-*d*]pyrimidin-4,7(*3H*,*6H*)-dione;
2-propylmercapto-5-(4-aminophenyl)-5,8-dihydropyrido[2,3-*d*]pyrimidin-4,7(*3H*,*6H*)-dione;
2-propylmercapto-5-(3-aminophenyl)-5,8-dihydropyrido[2,3-*d*]pyrimidin-4,7(*3H*,*6H*)-dione;
2-propylmercapto-5-(2-amino-3-methoxyphenyl)-5,8-dihydropyrido[2,3-*d*]pyrimidin-4,7(*3H*,*6H*)-dione;
2-propylmercapto-5-(2-cyanophenyl)-5,8-dihydropyrido[2,3-*d*]pyrimidin-4,7(*3H*,*6H*)-dione;
2-propylmercapto-5-(4-cyanophenyl)-5,8-dihydropyrido[2,3-*d*]pyrimidin-4,7(*3H*,*6H*)-dione;
2-methylmercapto-5-(4-methoxyphenyl)-5,8-dihydropyrido[2,3-*d*]pyrimidin-4,7(*3H*,*6H*)-dione;
2-butylmercapto-5-(4-methoxyphenyl)-5,8-dihydropyrido[2,3-*d*]pyrimidin-4,7(*3H*,*6H*)-dione;
2-butylmercapto-5-(3-nitro-4-hydroxyphenyl)-5,8-dihydropyrido[2,3-*d*]pyrimidin-4,7(*3H*,*6H*)-dione;
2-butylmercapto-5-(4-propoxyphenyl)-5,8-dihydropyrido[2,3-*d*]pyrimidin-4,7(*3H*,*6H*)-dione;
2-butylmercapto-5-(3-hydroxy-4-methoxyphenyl)-5,8-dihydropyrido[2,3-*d*]pyrimidin-4,7(*3H*,*6H*)-dione;
2-butylmercapto-5-(quinolin-6-yl)-5,8-dihydropyrido[2,3-*d*]pyrimidin-4,7(*3H*,*6H*)-dione;
2-isopropylmercapto-5-(4-methoxyphenyl)-5,8-dihydropyrido[2,3-*d*]pyrimidin-4,7(*3H*,*6H*)-dione;
2-propylmercapto-5-(4-morpholinophenyl)-5,8-dihydropyrido[2,3-*d*]pyrimidin-4,7(*3H*,*6H*)-dione;
2-propylmercapto-5-(4-(4-methylpiperazine)phenyl)-5,8-dihydropyrido[2,3-*d*]pyrimidin-4,7(*3H*,*6H*)-dione;
2-propylmercapto-5-(4-(piperidin-1-yl)phenyl)-5, 8-dihydropyrido[2,3-*d*]pyrimidin-4,7(*3H*,*6H*)-dione;
2-propylmercapto-5-(4-thiomorpholinophenyl)-5,8-dihydropyrido[2,3-*d*]pyrimidin-4,7(*3H*,*6H*)-dione;
2-propylmercapto-5-(benzofuran-5-yl)-5,8-dihydropyrido[2,3-*d*]pyrimidin-4,7(*3H*,*6H*)-dione;
2-propylmercapto-5-(benzothiazol-2-yl)-5,8-dihydropyrido[2,3-*d*]pyrimidin-4,7(*3H*,*6H*)-dione;
2-propylmercapto-5-(4-phenylthiophenyl)-5,8-dihydropyrido[2,3-*d*]pyrimidin-4,7(*3H*,*6H*)-dione;
2-propylmercapto-5-(4-methanesulfonylphenyl)-5,8-dihydropyrido[2,3-*d*]pyrimidin-4,7(*3H*,*6H*)-dione;
2-propylmercapto-5-(4-methylthiophenyl)-5,8-dihydropyrido[2,3-*d*]pyrimidin-4,7(*3H*,*6H*)-dione;
2-propylmercapto-5-(3-nitro-4-bromophenyl)-5,8-dihydropyrido[2,3-*d*]pyrimidin-4,7(*3H*,*6H*)-dione;
2-propylmercapto-5-(4-carboxyphenyl)-5,8-dihydropyrido[2,3-*d*]pyrimidin-4,7(*3H*,*6H*)-dione;
2-propylmercapto-5-(2-nitro-4-bromophenyl)-5,8-dihydropyrido[2,3-*d*]pyrimidin-4,7(*3H*,*6H*)-dione;
2-propylmercapto-5-(pyridin-2-yl)-5,8-dihydropyrido[2,3-*d*]pyrimidin-4,7(*3H*,*6H*)-dione;
2-propylmercapto-5-(4-isobutoxyphenyl)-5,8-dihydropyrido[2,3-*d*]pyrimidin-4,7(*3H*,*6H*)-dione;
2-propylmercapto-5-(pyridin-3-yl)-5,8-dihydropyrido[2,3-*d*]pyrimidin-4,7(*3H*,*6H*)-dione;
2-propylmercapto-5-(pyridin-4-yl)-5,8-dihydropyrido[2,3-*d*]pyrimidin-4,7(*3H*,*6H*)-dione;
2-propylmercapto-5-cyclohexyl-5,8-dihydropyrido[2,3-*d*]pyrimidin-4,7(*3H*,*6H*)-dione;
2-propylmercapto-5-cyclopentyl-5,8-dihydropyrido[2,3-*d*]pyrimidin-4,7(3*H*,6*H*)-dione;
2-propylmercapto-5-(1,4-benzodioxan-6-yl)-5,8-dihydropyrido[2,3-*d*]pyrimidin-4,7(*3H*,*6H*)-dione;
2-propylmercapto-5-(2,3-dihydrobenzofuran-5-yl)-5,8-dihydropyrido[2,3-*d*]pyrimidin-4,7(*3H*,*6H*)-dione;
2-propylmercapto-5-(quinoxalin-6-yl)-5,8-dihydropyrido[2,3-*d*]pyrimidin-4,7(*3H*,*6H*)-dione;
2-propylmercapto-5-(6-methoxypyridin-3-yl)-5,8-dihydropyrido[2,3-*d*]pyrimidin-4,7(*3H*,*6H*)-dione;
2-propylmercapto-5-(quinolin-3-yl)-5,8-dihydropyrido[2,3-*d*]pyrimidin-4,7(*3H*,*6H*)-dione;
2-propylmercapto-5-(furan-3-yl)-5,8-dihydropyrido[2,3-*d*]pyrimidin-4,7(*3H*,*6H*)-dione; or
2-propylmercapto-5-(4-heptoxyphenyl)-5,8-dihydropyrido[2,3-*d*]pyrimidin-4,7(*3H*,*6H*)-dione.

Further, the present invention also provides a preparation method for the compound. The compound according to the present invention may be prepared by the following method, but the conditions of the method, e.g., reactants, solvents, acids, alkalis, amounts of compounds used, reaction temperature, reaction time, etc., are not limited to the following description. Optionally, the compound according to the present invention may also be conveniently prepared by a combination of the various synthesis methods described in the present specification or known to those skilled in the art, and those skilled in the art to which the present invention belongs can easily implement the above combination.

When Y is -N(R)- and Z is 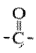 the compound shown as formula I is:

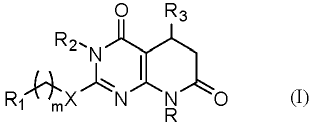

The method includes the following steps: 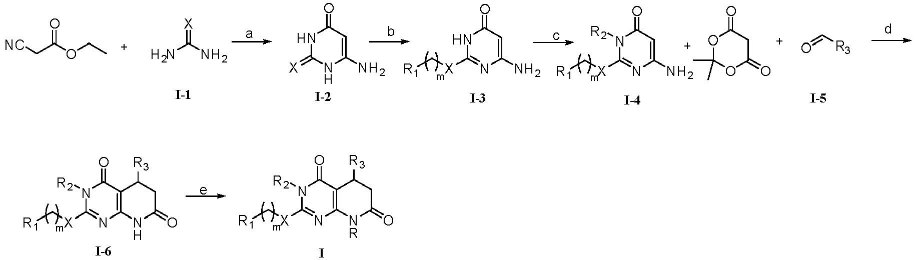
(a) ethyl cyanoacetate reacts with a compound of formula I-1 to give a compound of formula I-2;
(b) the compound of formula I-2 reacts with an R₁-substituted halide to give a compound of formula I-3;
(c) the compound of formula I-3 reacts with methanol, ethanol, haloalkane, unsaturated haloalkane, cyanogen bromide, *N*-chlorosuccinimide, *N*-bromosuccinimide, 1-cyano-1-methylethyl nitrate, urea, methyl isocyanate and chloramine to give a compound of formula I-4;
(d) the compound of formula I-4 reacts with 2,2-dimethyl-1,3-dioxane-4,6-dione and a compound of formula I-5 by a "one-pot" method to give a compound of formula I-6, wherein R₃ is hydrogen, a substituted or unsubstituted C₁-C₆ alkyl group, a carboxyl group, a nitro group, halogen, a trifluoromethyl group, a substituted or unsubstituted phenyl group or fused ring group, or a substituted or unsubstituted heterocyclyl group;
   the compound obtained in step (d) is further subjected to hydroxylation, reduction, cyanation and cyan-hydrolysis reaction in sequence to give the compound of formula I-6, wherein R₃ is a hydroxyl group, an amino group, a cyano group, or an amide group;
(e) the compound of formula I-6 is subjected to substitution reaction to give the compound shown as formula I;

in the formula, X, m, R₁, R₂, R and substituents are defined as above; or
the method includes the following steps: 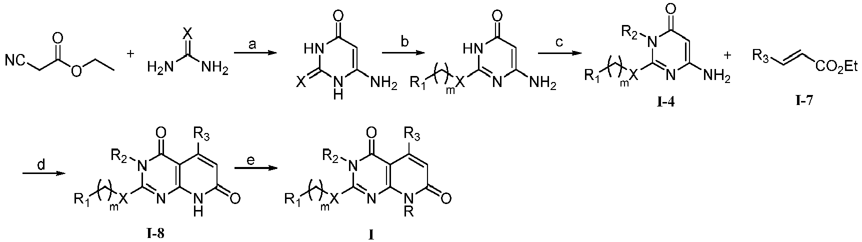
steps (a), (b) and (c) are the same as shown above, obtaining the compound of formula I-4 by reaction;
(d) the compound of formula I-4 reacts with a compound of formula I-7 to give a compound of formula I-8, wherein R₃ is hydrogen, a substituted or unsubstituted C₁-C₆ alkyl group, a carboxyl group, a nitro group, halogen, a trifluoromethyl group, a substituted or unsubstituted phenyl group or fused ring group, a substituted or unsubstituted heterocyclyl group;
the compound obtained in step (d) is further subjected to hydroxylation, reduction, cyanation and cyanohydrolysis reaction in sequence to give the compound of formula I-8, wherein R₃ is a hydroxyl group, an amino group, a cyano group, or an amide group;
(e) the compound of formula I-8 is subjected to substitution reaction to give the compound shown as formula I;
in the formula, X, m, R₁, R₂, R and the substituents are defined as above.

If Y is N and Z is CH, the compound shown as formula I is:

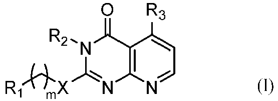

The method includes the following steps: 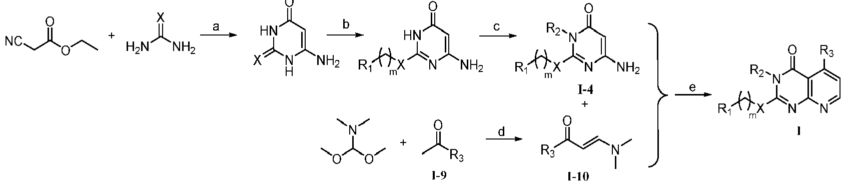
steps (a), (b) and (c) are described as above, obtaining the compound of formula I-4 by reaction;
(d) N,N-dimethylformamide dimethyl acetal (DMFDMA) reacts with a compound of formula I-9 to give a compound of formula I-10;
(e) the compound of formula I-4 reacts with the compound of formula I-10 to give a compound of formula I, wherein R₃ is hydrogen, a substituted or unsubstituted C₁-C₆ alkyl group, a carboxyl group, a nitro group, halogen, a trifluoromethyl group, a substituted or unsubstituted phenyl ring group or fused ring group, or a substituted or unsubstituted heterocyclyl group;
the compound obtained in step (e) is further subjected to hydroxylation, reduction, cyanation and cyan-hydrolysis reaction in sequence to give the compound shown in formula I, wherein R₃ is a hydroxyl group, an amino group, a cyano group, or an amide group; and
in the formula, X, m, R₁, R₂, R and the substituents are defined as above.

In a preferred embodiment, ethyl cyanoacetate and the compound of formula I-1 are heated to reflux in a polar solvent under an alkaline condition for 2 to 5 hours to give the compound of formula I-2. The alkali can be sodium, sodium methoxide, sodium ethoxide, or sodium hydride, the pH value is regulated to 7 to 9 through post-treatment.

In a preferred embodiment, the compound of formula I-2 and an R₁-substituted halide are heated to reflux in a polar solvent under an alkaline condition for 5 to 10 hours to give the compound of formula I-3. The alkali may be sodium hydroxide or potassium hydroxide.

In a preferred embodiment, the compound of formula I-3 and a corresponding reagent containing R₂ group react at room temperature or are heated to react in a polar solvent under an alkaline condition for 2 to 5 hours to give the compound of formula I-4. The alkali may be sodium hydroxide, potassium hydroxide, ammonia, triethylamine, diethylamine, sodium hydride, or *N*,*N*-diisopropylethylamine.

In a preferred embodiment, the compound of formula I-4, isoproplidene malonate and the compound of formula I-5 are heated to react in a high-boiling point solvent at a temperature of 170°C to 210°C for 1 to 8 hours to give the compound of formula I-6. The high-boiling point solvent may be nitrobenzene or ethylene glycol.

In a preferred embodiment, the compound of formula I-6 and a corresponding reagent containing R group react at room temperature or are heated to react in a polar solvent under an alkaline condition for 2 to 5 hours to give the compound (dihydropyrido[2,3-*d*]pyrimidin-4,7(*3H*,*6H*)-dione) of formula I. The alkali may be sodium hydroxide, potassium hydroxide, ammonia, triethylamine, diethylamine, sodium hydride, or *N*,*N-*diisopropylethylamine.

In a preferred embodiment, the compound of formula I-4 and R₃-substituted ethyl acrylate the compound of formula I-7 are heated to reflux in a toluene solvent under the catalysis of a palladium catalyst, *p*-toluenesulfonic acid hydrate, sodium persulfate and acetic anhydride for 12 to 36 hours to give the compound of formula I-8. The palladium catalyst may be palladium acetate or the like.

In a preferred embodiment, the compound of formula I-8 and a corresponding reagent containing R group react at room temperature or are heated to react in a polar solvent under an alkaline condition for 2 to 5 hours to give the compound (pyrido[2,3-*d*]pyrimidin-4,7(*3H*,*8H*)-dione) of formula I. The alkali may be sodium hydroxide, potassium hydroxide, ammonia, triethylamine, diethylamine, sodium hydride, or *N*,*N*-diisopropylethylamine.

In a preferred embodiment, *N*,*N-*dimethylformamide dimethyl acetal (DMFDMA) and R₃-substituted methyl ketone the compound of formula I-9 are heated to reflux in a polar solvent for 1 to 5 hours to give the compound of formula I-10. The polar solvent is usually dioxane or benzene.

In a preferred embodiment, the compound of formula I-4 and the compound of formula I-10 are heated in glacial acetic acid for 1 to 5 hours to give the compound (pyrido[2,3-*d*]pyrimidin-4(*3H*)-one) of formula I.

Further, the present invention also provides a pharmaceutical composition, which contains:
The compound, the enantiomer, diastereomer, raceme or their mixture thereof or the pharmaceutically acceptable salt thereof according to the present invention; and a pharmaceutically acceptable carrier.

Further more, the present invention also provides the use of the compound and the enantiomer, diastereomer, raceme or their mixture thereof or pharmaceutically acceptable salt thereof in:
(1) the preparation of a drug for preventing and/or treating tuberculosis infection;
(2) the preparation of a drug for preventing and/or treating drug-resistant tuberculosis infection; or
(3) the preparation of a drug for inhibiting the growth of mycobacterium tuberculosis.

A method for decreasing the pathogenicity or toxicity of mycobacterium tuberculosis, comprising the following steps:
mycobacterium tuberculosis is brought into contact with a safe and effective dose of the compound, enantiomer, diastereomer, raceme or their mixture thereof or pharmaceutically acceptable salt thereof according to the present invention or pharmaceutical composition according to the present invention, so as to reduce the pathogenicity or toxicity of mycobacterium tuberculosis.

The mode of administration to the subject in the present invention is not specifically limited, including but not limited to oral administration, injection, inhalation, and topical administration.

In the present invention, "safe and effective dose" means that the amount of the active ingredient (the compound of formula I) is enough to significantly improve the condition without causing severe side effects.

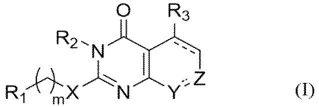

In the present invention, the pyrimidinone derivative, the compound of formula I and the compound shown as formula I have the same meaning, all referring to the compound with the following structure:
wherein R₁, m, X, Y, Z, R₂ and R₃ are defined as above.

In the present invention, the term "C₁-C₆ alkyl group" refers to a linear or branched alkyl group having 1 to 6 carbon atoms, including, without limitation, a methyl group, an ethyl group, a propyl group, an isopropyl group, a butyl group, and the like. In the present invention, the term "halogen" refers to fluorine, chlorine, bromine, and iodine.

In general, the pharmaceutical composition contains 1 mg to 2000 mg of active ingredient/dose, preferably 10 mg to 200 mg of active ingredient/dose. Preferably, the "one dose" is one tablet.

"Pharmaceutically acceptable carrier" refers to one or more compatible solid or liquid fillers or gel substances, which are suitable for human use and must have enough purity and sufficiently low toxicity. "Compatible" here means that the components of the composition can be blended with the active ingredient of the present invention and with each other, without significantly decreasing the efficacy of the active ingredient. Part of examples of the pharmaceutically acceptable carrier include cellulose and derivatives thereof (e.g., sodium carboxymethylcellulose, sodium ethylcellulose, cellulose acetate, etc.), gelatin, talc, solid lubricants (e.g., stearic acid and magnesium stearate), calcium sulfate, vegetable oils (e.g., soybean oil, sesame oil, peanut oil, olive oil, etc.), polyols (e.g., propylene glycol, glycerol, mannitol, sorbitol, etc.), emulsifiers (e.g., Tween), lubricants (e.g., sodium lauryl sulfate), colorants, flavoring agents, stabilizers, antioxidants, preservatives, pyrogen-free water, etc.

The mode of administration of the active ingredient or pharmaceutical composition according to the present invention is not specifically limited, and representative modes of administration include (but are not limited to): oral administration, intratumoral administration, rectal administration, parenteral (intravenous, intramuscular or subcutaneous) administration, etc.

Solid dosage forms for oral administration include capsules, tablets, pills, powders, and granules.

In these solid dosage forms, the active ingredient is mixed with at least one conventional inert excipient (or carrier), such as sodium citrate or calcium phosphate, or with: (a) fillers or solubilizers, such as starch, lactose, sucrose, glucose, mannitol, and silicic acid; (b) binders, such as hydroxymethyl cellulose, alginate, gelatin, polyvinylpyrrolidone, sucrose, and acacia; (c) humectants, such as glycerol; (d) disintegrants, such as agar, calcium carbonate, potato starch or tapioca starch, alginic acid, certain complex silicates, and sodium carbonate; (e) slow-dissolving agents, such as paraffin; (f) absorption accelerators, such as a quaternary amine compound; (g) wetting agents, such as cetyl alcohol and glycerol monostearate; (h) adsorbents, such as kaolin; (i) lubricants, such as talc, calcium stearate, magnesium stearate, solid polyethylene glycol, sodium lauryl sulfate, or mixtures thereof. In capsules, tablets and pills, the dosage form may also contain buffering agents.

The solid dosage forms may also be prepared using coatings and shell materials, such as casings and other materials known in the art. They may contain an opacifying agent, and the release of the active ingredient in such a composition may be in a certain part of the digestive tract in a delayed manner. Examples of embedding components that can be used are polymeric substances and wax substances.

Liquid dosage forms for oral administration include pharmaceutically acceptable emulsions, solutions, suspensions, syrups or tinctures. Besides the active ingredient, the liquid dosage forms may contain inert diluents conventionally used in the art, such as water or other solvents, solubilizers and emulsifiers, e.g., ethanol, isopropanol, ethyl carbonate, ethyl acetate, propylene glycol, 1,3-butanediol, dimethylformamide, and oils, particularly cottonseed oil, peanut oil, corn germ oil, olive oil, castor oil, sesame oil or mixtures of these substances, etc. Besides these inert diluents, the composition may also contain adjuvants, such as a wetting agent, an emulsifier and suspending agent, a sweetening agent, a flavoring agent, and a perfume.

The suspension, in addition to the active ingredient, may contain a suspending agent, e.g., ethoxylated isostearyl alcohol, polyoxyethylene sorbitol and sorbitan ester, microcrystalline cellulose, aluminum methoxide, agar or mixtures of these substances, or the like.

The composition for parenteral injection may contain a physiologically acceptable sterile aqueous or anhydrous solution, a dispersion, a suspension or emulsion, and a sterile powder for re-dissolution into a sterile injectable solution or dispersion. Suitable aqueous and non-aqueous carriers, diluents, solvents or excipients include water, ethanol, polyols, and suitable mixtures thereof.

The compound according to the present invention may be administered alone or in combination with other therapeutic drugs.

When using the pharmaceutical composition, a safe and effective amount of the compound of formula I according to the present invention is administered to a mammal (e.g., a human) in need of treatment, wherein the dose administered is a pharmaceutically considered effective dose, and for a human with a body weight of 60 kg, the daily dose administered is generally 1 mg to 2000 mg, preferably 20 mg to 500 mg. Of course, for the specific dose, factors, such as the route of administration, a patient's health condition, etc., should also be taken into consideration, which are all within the scope of skill of the skilled physician.

The features mentioned above in the present invention or the features mentioned in the examples may be combined arbitrarily. All of the features disclosed in the present specification may be used in combination with any composition form, and each feature disclosed in the present specification may be replaced by any alternative feature that serves the same, equivalent or similar purpose. Therefore, unless specifically stated otherwise, the disclosed features are merely general examples of equivalent or similar features.

The advantages of the present invention are as follows:
(1) The present invention provides a derivative with a novel pyrimidinone structure.
(2) The present invention provides a preparation method for the pyrimidinone derivative, and the process is simple and efficient.
(3) The present invention discovers for the first time a new use of the pyrimidinone derivative, that is, the pyrimidinone derivative can be used in the preparation of an anti-tuberculosis drug.

### DETAILED DESCRIPTION

In order to make the objectives, technical solutions and advantages of the present invention clearer, the technical solutions in the examples of the present invention will be clearly and completely described. Obviously, the described examples are merely part of the examples of the present invention, rather than all the examples. Based on the examples of the present invention, all other examples obtained by those of ordinary skilled in the art without creative efforts shall fall within the protection scope of the present invention.

In all the examples, thin-layer silica gel plate was used for thin-layer analysis (Merck, TLC Silica gel 60 F₂₅₄); silica gel (200 to 300 meshes) used for column chromatography was purchased from Qingdao Haiyang Chemical Co., Ltd., and the boiling range of petroleum ether used in column chromatography was 60°C to 90°C; and unless otherwise specified, all reagents in the experiment were purchased from J&K Scientific (Beijing) and Shanghai Bide Pharmatech Co., Ltd., and were analytically pure or chemically pure without being further processed. Each compound was analyzed for purity by HPLC using a chromatographically pure organic solvent and Watson's purified water prior to bioactivity testing. ¹H-NMR and ¹³C-NMR were recorded using a 400M or 600M nuclear magnetic resonance (NMR) apparatus, and chemical shifts were expressed in δ (ppm).

### Preparation Examples

### Preparation of 6-amino-2-thiouracil:

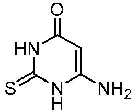

Ethyl cyanoacetate (6.40 mL, 60.0 mmol) and thiourea (4.56 g, 60.0 mmol) were added to a solution of sodium ethoxide (4.29 g, 63.0 mmoL) in ethanol (44 mL) and heated to reflux under an oil bath for 2 hours to give a brown insoluble precipitate. After being concentrated, the reaction solution was added into an appropriate amount of ice water, and the pH value was adjusted with 10% glacial acetic acid, and a white-like solid was gradually precipitated. At room temperature, the mixture was stirred, left standing overnight, and filtered to obtain 6.0 g of white solid with a yield of 70%.

### Preparation of 2-propylmercapto-6-aminopyrimidin-4(3H)-one:

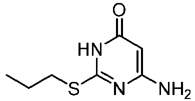

6-amino-2-thiouracil (1.43 g, 10.0 mmol), 1-bromopropane (0.93 mL, 10.0 mmol) and sodium hydroxide (0.4 g, 10.0 mmol) were dissolved in a mixed solution of 50 mL of water and 10 mL of isopropanol and heated to reflux for 10 hours, and TLC detected that the reaction of the raw materials was almost complete. After cooling to room temperature, a large amount of white precipitate was separated out. The reaction solution was filtered, washed with a large amount of water, and the filter cake was the product, which was dried to give 1.10 g of white powder with a yield of 59.5%.
**¹HNMR (DMSO-*d*₆, 400 MHz) δ (ppm)** 11.46 (1H, s), 6.42 (2H, s), 4.88 (1H, s), 3.04 (2H, t, *J* = 7.2 Hz), 1.58-1.68 (2H, m), 0.96 (3H, t, *J=* 7.2 Hz);
**¹³CNMR (DMSO-*d*₆, 101 MHz) δ (ppm)** 164.60, 163.96, 162.70, 81.70, 31.65, 22.79, 13.65;
**MS (ESI+) m/z** 186.2 [M+H] +.

### Preparation of 2-methylmercapto-6-aminopyrimidin-4(3H)-one:

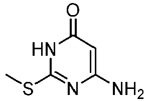

6-amino-2-thiouracil (1.43 g, 10.0 mmol), Iodomethane (0.62 mL, 10.0 mmol) and sodium hydroxide (0.4 g, 10.0 mmol) were dissolved in a mixed solution of 50 mL of water and 10 mL of isopropanol and heated to reflux for 8 hours, and TLC detected that the reaction of the raw materials was almost complete. After cooling to room temperature, a large amount of white precipitate was separated out. The reaction solution was filtered, washed with a large amount of water, and the filter cake was the product, which was dried to give 1.16 g of white powder with a yield of 73.9%.
**¹HNMR (DMSO-*d*₆, 400 MHz) δ (ppm)** 11.49 (1H, s), 6.44 (2H, s), 4.90 (1H, s), 2.42 (3H, s);
**¹³CNMR (DMSO-*d*₆, 101 MHz) δ (ppm)** 164.68, 163.99, 163.33, 81.63, 13.02;
**MS (ESI+) m/z** 158.0 [M+H] +.

### Preparation of 2-allylmercapto-6-aminopyrimidin-4(3H)-one:

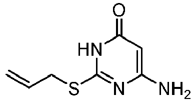

6-amino-2-thiouracil (1.43 g, 10.0 mmol), 3-bromopropene (0.87 mL, 10.0 mmol) and sodium hydroxide (0.4 g, 10.0 mmol) were dissolved in a mixed solution of 50 mL of water and 10 mL of isopropanol and heated to reflux for 5 hours, and TLC detected that the reaction of the raw materials was almost complete. After cooling to room temperature, a large amount of white precipitate was separated out. The reaction solution was filtered, washed with a large amount of water, and the filter cake was the product, which was dried to give 0.74 g of white powder with a yield of 40.4%.
**¹HNMR (DMSO-*d*₆, 400 MHz) δ (ppm)** 11.52(1H, s), 6.46(2H, s), 5.87-5.94(1H, m), 5.31(1H, dd, *J* = 16.8, 1.8 Hz), 5.10(1H, dd, *J* = 10.2, 1.2 Hz), 4.93 (1H, s), 3.74 (2H, d, *J* = 7.2 Hz);
**¹³CNMR (DMSO-*d*₆, 101 MHz) δ (ppm)** 134.28, 118.47, 81.78, 32.56;
**MS (ESI+) m/z** 184.0 [M+H] +.

### Preparation of 3-dimethylamino-1-(4-methoxyphenyl)-2-propylene-1-one:

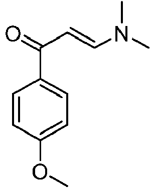

p-methoxyacetophenone (150 mg, 1.0 mmol) and N,N-dimethylformamide dimethyl acetal (DMFDMA) (476 mg, 4.0 mmol) were dissolved in approximately 5.0 mL of DMF and heated to reflux for about 3.5 hours, and TLC detected that the reaction of the raw materials was almost complete. The reaction solution was concentrated to give 205 mg of yellow solid with a yield of 100%.
**¹HNMR (DMSO-*d*₆, 600 MHz) δ (ppm)** 7.87(2H, d, *J* = 9.0 Hz), 7.66(1H, d, *J* = 12 Hz), 6.95(2H, d, *J* = 9.0 Hz), 5.80(1H, d, *J=* 12 Hz), 3.80(3H, s), 3.12(3H, s), 2.90(3H, s);
**¹³CNMR (DMSO-*d*₆, 150 MHz) δ (ppm)** 185.20, 161.90, 154.12, 133.26, 129.60 × 2, 113.78 × 2, 55.74, 44.88, 37.55;
**MS (ESI+) m/z** 206.1 [M+H] +.

### Preparation of 3-dimethylamino-1-(quinolin-6-yl)-2-propylene-1-one:

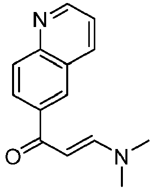

6-acetylquinoline (171 mg, 1.0 mmol) and *N*,*N*-dimethylformamide dimethyl acetal (DMFDMA) (476 mg, 4.0 mmol) were dissolved in approximately 5.0 mL of DMF and heated to reflux under an oil bath for about 3.5 hours, and TLC detected that the reaction of the raw materials was almost complete. The reaction solution was concentrated to give 226 mg of yellow solid with a yield of 100%.
¹HNMR (DMSO-*d*₆, 600 MHz) δ (ppm) 8.95(1H, dd, *J* = 4.2, 1.8 Hz), 8.57(1H, d, *J* = 1.2 Hz), 8.49(1H, d, *J=* 7.8 Hz), 8.24(1H, dd, *J=* 8.4, 1.8 Hz), 8.04(1H, d, *J=* 8.4 Hz), 7.80(1H, d, *J* = 12 Hz), 7.59(1H, q), 6.01(1H, d, *J=* 12 Hz), 3.18(3H, s), 2.98(3H, s);
MS (ESI+) m/z 227.1 [M+H] +.

### Example 1:

### Preparation of 2-propylmercapto-5-(4-propoxyphenyl)-5,8-dihydropyrido[2,3-d]pyrimidin-4,7(3H,6H)-dione (LSL-7-30)

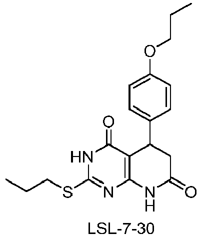

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-propoxybenzaldehyde (0.158 mL, 1.0 mmol) were dissolved in about 5 mL of ethylene glycol, and heated to react under an oil bath at 200°C for about 2 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 40%), 50 mg of pure product was obtained with a yield of 13.4%.
**¹HNMR (DMSO-*d*₆, 400 MHz) δ (ppm)** 12.57(1H, s), 10.52(1H, s), 7.05(2H, dd, *J* = 8.4, 2.0 Hz), 6.83(2H, dd, *J =* 8.4, 1.8 Hz), 4.14(1H, d, *J* = 7.2 Hz), 3.87(2H, t, *J* = 6.8 Hz), 3.05-3.17(2H, m), 3.01(1H, q), 2.49(1H, d, *J* = 16.2 Hz), 1.63-1.74(4H, m), 0.97(3H, t, *J* = 7.2 Hz), 0.95(3H, t, *J* = 7.2 Hz);
**¹³CNMR (DMSO-*d*₆, 101 MHz) δ (ppm)** 171.04, 157.93, 134.70, 127.92 × 2, 114.97 × 2, 69.33, 38.71, 32.63, 31.95, 22.83, 22.48, 13.63, 10.87;
**MS (ESI+) m/z** 374.1 [M+H] +.

### Example 2:

### Preparation of 2-methylmercapto-5-(4-propoxyphenyl)-5,8-dihydropyrido[2,3-d]pyrimidin-4,7(3H,6H)-dione (LSL-7-36)

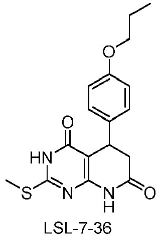

2-methylmercapto-6-aminopyrimidin-4(*3H*)-one (157 mg, 1.0 mmol), 2,2-dimethyl-1,3-dioxane-4,6-dione (173 mg, 1.2 mmol) and 4-propoxybenzaldehyde (0.158 mL, 1.0 mmol) were dissolved in about 5 mL of ethylene glycol, and heated to react under an oil bath at 200°C for about 1.5 hours, and TLC detected (petroleum ether/ethyl acetate = 2/3) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 55%), 43 mg of pure product was obtained with a yield of 12.4%.
**¹HNMR (DMSO-*d*₆, 400 MHz) δ (ppm)** 12.59(1H, s), 10.52(1H, s), 7.05(2H, d, *J =* 8.4Hz), 6.83(2H, d, *J* = 8.4 Hz), 4.14(1H, d, *J* = 7.2 Hz), 3.86(2H, t, *J* = 6.8 Hz), 2.99(1H, q), 2.50(3H, s), 2.49(1H, d, *J =* 16.0 Hz), 1.64-1.74(2H, m), 0.95(3H, t, *J =* 7.6 Hz);**¹³CNMR (DMSO-*d*₆, 101 MHz) δ (ppm)** 171.00, 157.93, 134.65, 127.93 × 2, 114.96 × 2, 69.34, 38.75, 32.62, 22.48, 13.17, 10.87;
**MS (ESI+) m/z** 368.1 [M+Na] +.

### Example 3:

### Preparation of 2-propylmercapto-5-(3-hydroxy-4-methoxyphenyl)-5,8-dihydropyrido[2,3-d]pyrimidin-4,7(3H,6H)-dione (LSL-7-38)

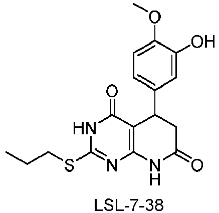

2-propylmercapto-6-aminopyrimidin-4(*3H*)-one (185 mg, 1.0 mmol), 2,2-dimethyl-1,3-dioxane-4,6-dione (173 mg, 1.2 mmol) and 3-hydroxy-4-methoxybenzaldehyde (152 mg, 1.0 mmol) were dissolved in about 5 mL of ethylene glycol, and heated to react under an oil bath at 200°C for about 2.5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 50%), 70 mg of pure product was obtained with a yield of 19.3%.
**¹HNMR (DMSO-*d*₆, 400 MHz) δ (ppm)** 12.56(1H, s), 10.50(1H, s), 8.91(1H, s), 6.80(1H, d, *J* = 8.4 Hz), 6.58(1H, d, *J* = 2.0 Hz), 6.53(1H, dd, *J* = 8.4, 2.0 Hz), 4.06(1H, d, *J* = 7.6 Hz), 3.70(3H, s), 3.87(2H, t, *J* = 6.8 Hz), 3.05-3.17(2H, m), 2.97(1H, q), 2.47(1H, d, *J* = 16.4 Hz), 1.63-1.72(2H, m), 0.98(3H, t, *J=* 7.2 Hz);
**¹³CNMR (DMSO-*d*₆, 101 MHz) δ (ppm)** 171.05, 146.93, 146.89, 135.57, 117.42, 114.30, 112.99, 56.15, 38.76, 32.78, 31.94, 22.84, 13.63;
**MS (ESI+) m/z** 362.1 [M+H] +.

### Example 4:

### Preparation of 2-methylmercapto-5-(3-hydroxy-4-methoxyphenyl)-5,8-dihydropyrido[2,3-d]pyrimidin-4,7(3H,6H)-dione (LSL-7-39)

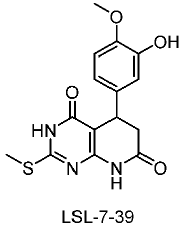

2-methylmercapto-6-aminopyrimidin-4(*3H*)-one (157 mg, 1.0 mmol), 2,2-dimethyl-1,3-dioxane-4,6-dione (173 mg, 1.2 mmol) and 3-hydroxy-4-methoxybenzaldehyde (152 mg, 1.0 mmol) were dissolved in about 5 mL of ethylene glycol, and heated to react under an oil bath at 200°C for about 3 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 50%), 85 mg of pure product was obtained with a yield of 25.5%.
**¹HNMR (DMSO-*d*₆, 600 MHz) δ (ppm)** 12.58(1H, s), 10.48(1H, s), 8.88(1H, s), 6.80(1H, d, *J =* 8.4 Hz), 6.57(1H, d, *J =* 1.8 Hz), 6.53(1H, dd, *J =* 8.4, 1.8 Hz), 4.06(1H, d, *J =* 7.8 Hz), 3.70(3H, s), 2.96(1H, q), 2.50(3H, s), 2.46(1H, d, *J=* 16.8 Hz);
**¹³CNMR (DMSO-*d*₆, 150 MHz) δ (ppm)** 171.00, 146.96, 146.91, 135.55, 117.45, 114.33, 113.04, 56.19, 38.81, 32.80, 13.17;
**MS (ESI+) m/z** 334.1 [M+H] +.

### Example 5:

### Preparation of 2-propylmercapto-5-(3,4-dihydroxyphenyl)-5,8-dihydropyrido[2,3-d]pyrimidin-4,7(3H,6H)-dione (LSL-7-42)

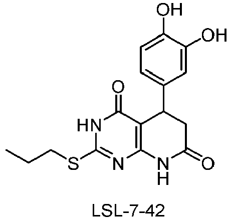

2-propylmercapto-6-aminopyrimidin-4(*3H*)-one (185 mg, 1.0 mmol), 2,2-dimethyl-1,3-dioxane-4,6-dione (173 mg, 1.2 mmol) and 3,4-dihydroxybenzaldehyde (138 mg, 1.0 mmol) were dissolved in about 5 mL of ethylene glycol, and heated to react under an oil bath at 170°C for about 5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 60%), 95 mg of pure product was obtained with a yield of 27.4%.
**¹HNMR (DMSO-*d*₆, 400 MHz) δ (ppm)** 12.53(1H, s), 10.46(1H, s), 8.81(1H, s), 8.68(1H, s), 6.61(1H, d, *J=* 8.4 Hz), 6.52(1H, d, *J=* 1.8 Hz), 6.41(1H, dd, *J=* 8.4, 1.8 Hz), 4.02(1H, d, *J* = 7.2 Hz), 3.05-3.10(2H, m), 2.94(1H, q), 2.44(1H, d, *J=* 16.8 Hz),1.63-1.70(2H, m), 0.97(3H, t, *J* = 7.8 Hz);
**¹³CNMR (DMSO-*d*₆, 101 MHz) δ (ppm)** 171.10, 145.56, 144.48, 133.76, 117.63, 116.04, 114.33, 38.90, 32.77, 31.96, 22.85, 13.63;
**MS (ESI+) m/z** 348.1 [M+H] +.

### Example 6:

### Preparation of 2-methylmercapto-5-(3,4,5-trimethoxyphenyl)-5,8-dihydropyrido[2,3-d]pyrimidin-4,7(3H,6H)-dione (LSL-7-43)

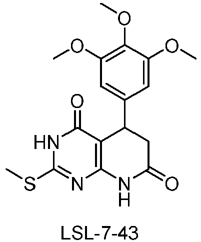

2-methylmercapto-6-aminopyrimidin-4(3H)-one (157 mg, 1.0 mmol), 2,2-dimethyl-1,3-dioxane-4,6-dione (173 mg, 1.2 mmol) and 3,4,5-trimethoxybenzaldehyde (196 mg, 1.0 mmol) were dissolved in about 5 mL of ethylene glycol, and heated to react under an oil bath at 170°C for about 3 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 70%), 78 mg of pure product was obtained with a yield of 20.7%.
**¹HNMR (DMSO-*d*₆, 400 MHz) δ (ppm)** 12.61 (1H, s), 10.52(1H, s), 6.47(2H, s), 4.16(1H, d, *J=* 7.8 Hz), 3.69(6H, s), 3.61(3H, s), 3.00(1H, q), 2.56(1H, d, *J=* 16.8 Hz), 2.51(3H, s);
**¹³CNMR (DMSO-d6, 101 MHz) δ (ppm)** 171.14, 153.34 × 2, 138.78, 136.96, 104.44 × 2, 60.42, 56.34 × 2, 38.40, 33.63, 14.02;
**MS (ESI+) m/z** 378.1 [M+H] +.

### Example 7:

### Preparation of 2-propylmercapto-5-(3,4,5-trimethoxyphenyl)-5,8-dihydropyrido[2,3-d]pyrimidin-4,7(3H,6H)-dione (LSL-7-44)

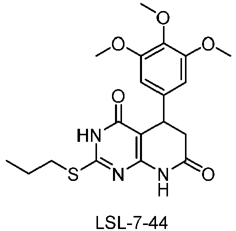

2-propylmercapto-6-aminopyrimidin-4(*3H*)-one (185 mg, 1.0 mmol), 2,2-dimethyl-1,3-dioxane-4,6-dione (173 mg, 1.2 mmol) and 3,4,5-trimethoxybenzaldehyde (196 mg, 1.0 mmol) were dissolved in about 5 mL of ethylene glycol, and heated to react under an oil bath at 170°C for about 2.5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 50%), 102 mg of pure product was obtained with a yield of 25.2%.
**¹HNMR (DMSO-*d*₆, 600 MHz) δ (ppm)** 12.58 (1H, s), 10.52(1H, s), 6.48(2H, s), 4.16(1H, d, *J =* 7.8 Hz), 3.69(6H, s), 3.61(3H, s), 3.06-3.16(2H, m), 3.00(1H, q), 2.56(1H, d, *J =* 16.2 Hz),1.63-1.69(2H, m), 0.96(3H, t, *J* = 7.8 Hz);
**¹³CNMR (DMSO-*d*₆, 150 MHz) δ (ppm)** 171.17, 153.37 × 2, 138.80, 136.96, 104.43 × 2, 60.42, 56.31 × 2, 38.34, 33.60, 31.99, 22.84, 13.57;
**MS (ESI+) m/z** 406.1 [M+H] +.

### Example 8:

### Preparation of 2-propylmercapto-5-(3,4-dimethoxyphenyl)-5,8-dihydropyrido[2,3-d]pyrimidin-4,7(3H,6H)-dione (LSL-7-46)

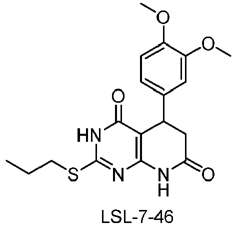

2-propylmercapto-6-aminopyrimidin-4(*3H*)-one (185 mg, 1.0 mmol), 2,2-dimethyl-1,3-dioxane-4,6-dione (173 mg, 1.2 mmol) and 3,4-dimethoxybenzaldehyde (166 mg, 1.0 mmol) were dissolved in about 5 mL of ethylene glycol, and heated to react under an oil bath at 170°C for about 3 hours, and TLC detected (petroleum ether/ethyl acetate = 1/3) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 50%), 100 mg of pure product was obtained with a yield of 26.7%.
**¹HNMR (DMSO-*d*₆, 600 MHz) δ (ppm)** 12.57(1H, s), 10.49(1H, s), 6.88(1H, d, *J =* 1.8 Hz), 6.82(1H, d, *J* = 8.4 Hz), 6.54(1H, dd, *J* = 8.4, 1.8 Hz), 4.15(1H, d, *J* = 7.2 Hz), 3.70(3H, s), 3.69(3H, s), 3.06-3.15(2H, m), 2.98(1H, q), 2.54(1H, d, *J* = 16.8 Hz), 1.63-1.69(2H, m), 0.97(3H, t, *J* = 7.2 Hz);
**¹³CNMR (DMSO-*d*₆, 150 MHz) δ (ppm)** 171.11, 149.30, 148.18, 135.38, 118.03, 112.36, 111.66, 56.01, 55.90, 38.60, 32.99, 31.97, 22.84, 13.61;
**MS (ESI+) m/z** 376.1 [M+H] +.

### Example 9:

### Preparation of 2-propylmercapto-5-(3-nitro-4-chlorophenyl)-5,8-dihydropyrido[2,3-d]pyrimidin-4,7(3H,6H)-dione (LSL-7-48)

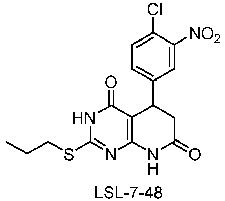

2-propylmercapto-6-aminopyrimidin-4(*3H*)-one (185 mg, 1.0 mmol), 2,2-dimethyl-1,3-dioxane-4,6-dione (173 mg, 1.2 mmol) and 3-nitro-4-chlorobenzaldehyde (185 mg, 1.0 mmol) were dissolved in about 5 mL of ethylene glycol, and heated to react under an oil bath at 170°C for about 3 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 50%), 125 mg of pure product was obtained with a yield of 31.7%.
**¹HNMR (DMSO-*d*₆, 600 MHz) δ (ppm)** 12.66(1H, s), 10.56(1H, s), 7.88(1H, d, *J =* 1.8 Hz), 7.69(1H, d, *J =* 8.4 Hz), 7.45(1H, dd, *J =* 8.4, 1.8 Hz), 4.31(1H, d, *J =* 7.8 Hz), 3.07-3.14(2H, m), 3.06(1H, q), 2.58(1H, d, *J=* 16.2 Hz), 1.63-1.70(2H, m), 0.97(3H, t, *J=* 7.2 Hz);
**¹³CNMR (DMSO-*d*₆, 150 MHz) δ (ppm)** 170.40, 148.10, 132.37, 132.30, 124.31, 113.56, 100.00, 37.93, 33.03, 32.01, 22.86, 13.66;
**MS (ESI+) m/z** 395.1 [M+H] +.

### Example 10:

### Preparation of 2-methylmercapto-5-(4-nitrophenyl)-5,8-dihydropyrido[2,3-d]pyrimidin-4,7(3H,6H)-dione (LSL-7-49)

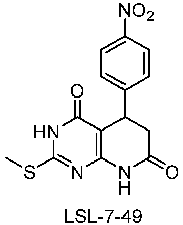

2-methylmercapto-6-aminopyrimidin-4(*3H*)-one (157 mg, 1.0 mmol), 2,2-dimethyl-1,3-dioxane-4,6-dione (173 mg, 1.2 mmol) and 4-nitrobenzaldehyde (151 mg, 1.0 mmol) were dissolved in about 5 mL of ethylene glycol, and heated to react under an oil bath at 200°C for about 2.5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 50%), 85 mg of pure product was obtained with a yield of 25.6%.
**¹HNMR (DMSO-*d*₆, 600 MHz) δ (ppm)** 12.68 (1H, s), 10.65(1H, s), 8.16(2H, d, *J* = 8.4 Hz), 7.44(2H, d, *J =* 8.4 Hz), 4.36(1H, d, *J* = 7.8 Hz), 3.12(1H, q), 2.57(1H, d, *J* = 16.8 Hz), 2.52(3H, s);
**¹³CNMR (DMSO-*d*₆, 150 MHz) δ (ppm)** 170.43, 146.90, 128.40 × 2, 124.36 × 2, 88.48, 38.01, 33.63, 13.19;
**MS (ESI+) m/z** 333.1 [M+H] +.

### Example 11:

### Preparation of 2-propylmercapto-5-(4-nitrophenyl)-5,8-dihydropyrido[2,3-d]pyrimidin-4,7(3H,6H)-dione (LSL-7-50)

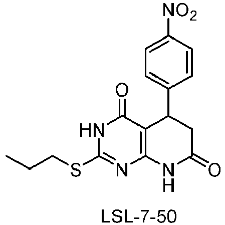

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-nitrobenzaldehyde (151 mg, 1.0 mmol) were dissolved in about 5 mL of ethylene glycol, and heated to react under an oil bath at 210°C for about 1 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 60%), 92 mg of pure product was obtained with a yield of 25.6%.
**¹HNMR (DMSO-*d*₆, 600 MHz) δ (ppm)** 12.67(1H, s), 10.58(1H, s), 8.16(2H, d, *J =* 9.0 Hz), 7.44(2H, d, *J=* 8.4 Hz), 4.35(1H, d, *J=* 7.8 Hz), 3.08-3.15(2H, m), 3.07(1H, q), 2.56(1H, d, *J=* 16.2 Hz), 1.63-1.70(2H, m), 0.97(3H, t, *J=* 7.2 Hz);
**¹³CNMR (DMSO-*d*₆, 150 MHz) δ (ppm)** 168.36, 149.05, 144.74, 126.28 × 2, 122.21 × 2, 35.91, 31.59, 29.89, 20.74, 11.53;
**MS (ESI+) m/z** 361.1 [M+H] +.

### Example 12:

### Preparation of 2-methylmercapto-5-(2-hydroxy-4-methoxyphenyl)-5,8-dihydropyrido[2,3-d]pyrimidin-4,7(3H,6H)-dione (LSL-9-1)

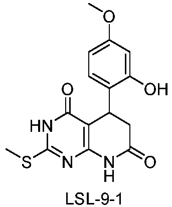

2-methylmercapto-6-aminopyrimidin-4(*3H*)-one (157 mg, 1.0 mmol), 2,2-dimethyl-1,3-dioxane-4,6-dione (173 mg, 1.2 mmol) and 2-hydroxy-4-methoxybenzaldehyde (152 mg, 1.0 mmol) were dissolved in about 5 mL of ethylene glycol, and heated to react under an oil bath at 210°C for about 2.5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 60%), 60 mg of pure product was obtained with a yield of 18.0%.
**¹HNMR (DMSO-*d*₆, 600 MHz) δ (ppm)** 12.33(1H, s), 10.31(1H, s), 6.57(1H, d, *J =* 8.4 Hz), 6.37(1H, d, *J=* 1.8 Hz), 6.23(1H, dd, *J* = 8.4, 1.8 Hz), 4.28(1H, d, *J=* 8.4 Hz), 3.64(3H, s), 2.88(1H, q), 2.47(3H, s), 2.45(1H, d, *J=* 16.8 Hz);
**¹³CNMR (DMSO-*d*₆, 150 MHz) δ (ppm)** 171.34, 134.59, 129.16, 127.04, 104.52, 102.61, 100.00, 55.35, 37.56, 27.97, 13.26;
**MS (ESI+) m/z** 356.1 [M+Na] +.

### Example 13:

### Preparation of 2-propylmercapto-5-(2-hydroxy-4-methoxyphenyl)-5,8-dihydropyrido[2,3-d]pyrimidin-4,7(3H,6H)-dione (LSL-9-2)

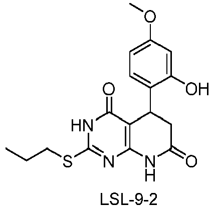

2-propylmercapto-6-aminopyrimidin-4(*3H*)-one (185 mg, 1.0 mmol), 2,2-dimethyl-1,3-dioxane-4,6-dione (173 mg, 1.2 mmol) and 2-hydroxy-4-methoxybenzaldehyde (152 mg, 1.0 mmol) were dissolved in about 5 mL of ethylene glycol, and heated to react under an oil bath at 210°C for about 3.5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 50%), 96 mg of pure product was obtained with a yield of 26.6%.
**¹HNMR (DMSO-*d*₆, 600 MHz) δ (ppm)** 12.57(1H, s), 10.44(1H, s), 9.71(1H, s), 6.52(1H, d, *J* = 8.4 Hz), 6.40(1H, d, *J* = 2.4 Hz), 6.25(1H, dd, *J* = 8.4, 2.4 Hz), 4.30(1H, d, *J* = 7.8 Hz), 3.64(3H, s), 3.08-3.17(2H, m), 2.88(1H, q), 2.46(1H, d, *J* = 16.2 Hz), 1.65-1.71(2H, m), 0.98(3H, t, *J* = 7.8 Hz);
**¹³CNMR (DMSO-*d*₆, 150 MHz) δ (ppm)** 171.21, 159.53, 156.09, 127.28, 120.55, 104.51, 102.32, 55.35, 37.56, 31.99, 27.89, 22.86, 13.64;
**MS (ESI+) m/z** 362.1 [M+H] +.

### Example 14:

### Preparaiton of 2-propylmercapto-5-(4-hydroxyphenyl)-5,8-dihydropyrido[2,3-d]pyrimidin-4,7(3H,6H)-dione (LSL-9-6)

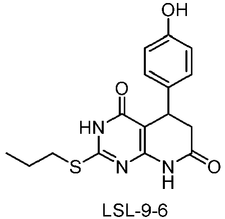

2-propylmercapto-6-aminopyrimidin-4(*3H*)-one (185 mg, 1.0 mmol), 2,2-dimethyl-1,3-dioxane-4,6-dione (173 mg, 1.2 mmol) and p-hydroxybenzaldehyde (122 mg, 1.0 mmol) were dissolved in about 5 mL of ethylene glycol, and heated to react under an oil bath at 200°C for about 2 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (dichloromethane/methanol: 1.5%), 130 mg of pure product was obtained with a yield of 39.3%.
**¹HNMR (DMSO-*d*₆, 600 MHz) δ (ppm)** 12.61(1H, s), 10.37(1H, s), 9.26(1H, s), 6.93(2H, dt, *J* = 8.4, 1.8 Hz), 6.65(1H, dt, *J =* 8.4, 1.8 Hz), 4.08(1H, d, *J =* 7.8 Hz), 3.03-3.12(2H, m), 2.94(1H, q), 2.46(1H, d, *J=* 16.2 Hz), 1.62-1.69(2H, m), 0.97(3H, t, *J=* 7.2 Hz);
**¹³CNMR (DMSO-*d*₆, 150 MHz) δ (ppm)** 171.10, 156.52, 133.26, 127.88 × 2, 115.69 × 2, 38.92, 32.71, 31.96, 22.90, 13.65;
**MS (ESI+) m/z** 332.1 [M+H] +.

### Example 15:

### Preparation of 2-methylmercapto-5-(2,6-dimethylphenyl)-5,8-dihydropyrido[2,3-d]pyrimidin-4,7(3H,6H)-dione (LSL-9-7)

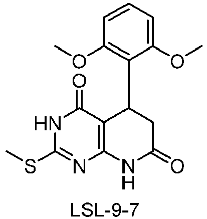

2-methylmercapto-6-aminopyrimidin-4(*3H*)-one (157mg, 1.0 mmol), 2,2-dimethyl-1,3-dioxane-4,6-dione (173 mg, 1.2 mmol) and 2,6-dimethoxybenzaldehyde (166 mg, 1.0 mmol) were dissolved in about 5 mL of ethylene glycol, and heated to react under an oil bath at 200°C for about 2.5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 60%), 78 mg of pure product was obtained with a yield of 22.5%.
**¹HNMR (DMSO-*d*₆, 600 MHz) δ (ppm)** 12.16(1H, s), 10.21(1H, s), 7.11(1H, t, *J =* 8.4 Hz), 6.57(2H, d, *J =* 8.4 Hz), 4.78(1H, d, *J =* 9.0 Hz), 3.66(6H, s), 2.93(1H, q), 2.46(3H, s), 2.06(1H, d, *J* = 16.8 Hz);
**¹³CNMR (DMSO-*d*₆, 150 MHz) δ (ppm)** 170.47, 158.44, 128.01 × 2, 121.24, 105.15 × 2, 56.06 × 2, 36.74, 24.22, 13.17;
**MS (ESI+) m/z** 370.1 [M+Na] +.

### Example 16:

### Preparation of 2-propylmercapto-5-(2,6-dimethylphenyl)-5,8-dihydropyrido[2,3-d]pyrimidin-4,7(3H,6H)-dione (LSL-9-8)

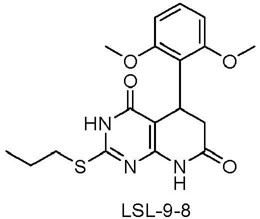

2-propylmercapto-6-aminopyrimidin-4(*3H*)-one (185 mg, 1.0 mmol), 2,2-dimethyl-1,3-dioxane-4,6-dione (173 mg, 1.2 mmol) and 2,6-dimethoxybenzaldehyde (166 mg, 1.0 mmol) were dissolved in about 5 mL of ethylene glycol, and heated to react under an oil bath at 200°C for about 2 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 60%), 130 mg of pure product was obtained with a yield of 34.7%.
**¹HNMR (DMSO-*d*₆, 600 MHz) δ (ppm)** 12.10(1H, s), 10.23(1H, s), 7.11(1H, t, *J =* 8.4 Hz), 6.57(2H, d, *J=* 8.4 Hz), 4.77(1H, d, *J=* 9.6 Hz), 3.66(6H, s), 3.02-3.13(2H, m), 2.94(1H, q), 2.06(1H, d, *J=* 16.8 Hz), 1.61-1.68(2H, m), 0.96(3H, t, *J=* 7.2 Hz);
**¹³CNMR (DMSO-*d*₆, 150 MHz) δ (ppm)** 170.50, 158.44, 128.02 × 2, 121.22, 105.18 × 2, 56.04 × 2, 38.75, 31.91, 24.24, 22.94, 13.61;
**MS (ESI+) m/z** 398.1 [M+Na] +.

### Example 17:

### Preparation of 2-methylmercapto-5-(3-nitrophenyl)-5,8-dihydropyrido[2,3-d]pyrimidin-4,7(3H,6H)-dione (LSL-9-9)

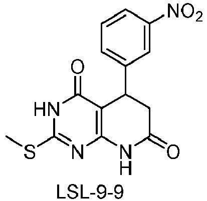

2-methylmercapto-6-aminopyrimidin-4(*3H*)-one (157 mg, 1.0 mmol), 2,2-dimethyl-1,3-dioxane-4,6-dione (173 mg, 1.2 mmol) and 3-nitrobenzaldehyde (151 mg, 1.0 mmol) were dissolved in about 5 mL of ethylene glycol, and heated to react under an oil bath at 200°C for about 2.5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 55%), 110 mg of pure product was obtained with a yield of 33.1%.
**¹HNMR (DMSO-*d*₆, 600 MHz) δ (ppm)** 12.74(1H, s), 10.56(1H, s), 8.09(1H, dd, *J* = 7.8, 1.8 Hz), 8.03(1H, d, *J=* 1.8 Hz), 7.64(1H, dd, *J=* 7.8, 1.8 Hz), 7.60(1H, t, *J=* 7.8 Hz), 4.37(1H, d, *J* = 7.8 Hz), 3.06-3.11(1H, q), 2.61(1H, d, *J* = 16.2 Hz), 2.49(3H, s);
**¹³CNMR (DMSO-*d*₆, 150 MHz) δ (ppm)** 170.57, 148.45, 145.61, 133.82, 130.69, 122.27, 121.68, 38.06, 33.39, 13.23;
**MS (ESI+) m/z** 333.1 [M+H] +.

### Example 18:

### Preparation of 2-propylmercapto-5-(3-nitrophenyl)-5,8-dihydropyrido[2,3-d]pyrimidin-4,7(3H,6H)-dione (LSL-9-10)

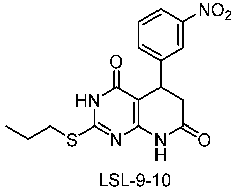

2-propylmercapto-6-aminopyrimidin-4(*3H*)-one (185 mg, 1.0 mmol), 2,2-dimethyl-1,3-dioxane-4,6-dione (173 mg, 1.2 mmol) and 3-nitrobenzaldehyde (151 mg, 1.0 mmol) were dissolved in about 5 mL of ethylene glycol, and heated to react under an oil bath at 210°C for about 1 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 60%), 138 mg of pure product was obtained with a yield of 38.3%.
**¹HNMR (DMSO-*d*₆, 600 MHz) δ (ppm)** 12.68(1H, s), 10.59(1H, s), 8.08(1H, dd, *J* = 8.4, 1.8 Hz), 8.03(1H, d, *J* = 1.8 Hz), 7.59-7.65(2H, m), 4.37(1H, d, *J=* 7.8 Hz), 3.07-3.15(2H, m), 3.07(1H, q), 2.61(1H, d, *J* = 16.2 Hz), 1.63-1.70(2H, m), 0.97(3H, t, *J=* 7.2 Hz);
**¹³CNMR (DMSO-*d*₆, 150 MHz) δ (ppm)** 170.60, 148.46, 145.55, 133.79, 130.72, 122.29, 121.69, 37.99, 33.36, 32.01, 22.86, 13.64;
**MS (ESI+) m/z** 361.1 [M+H] +.

### Example 19:

### Preparation of 2-propylmercapto-5-(2,4-dihydroxyphenyl)-5,8-dihydropyrido[2,3-d]pyrimidin-4,7(3H,6H)-dione (LSL-9-12)

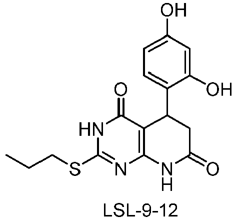

2-propylmercapto-6-aminopyrimidin-4(*3H*)-one (185 mg, 1.0 mmol), 2,2-dimethyl-1,3-dioxane-4,6-dione (173 mg, 1.2 mmol) and 2,4-dihydroxybenzaldehyde (138 mg, 1.0 mmol) were dissolved in about 5 mL of ethylene glycol, and heated to react under an oil bath at 210°C for about 1.5 hours, and TLC detected (petroleum ether/ethyl acetate = 1/3) that the raw material points disappeared. The reaction solution was poured into an appropriate amount of ice water, and a flocculent precipitate was produced, which was filtered to obtain a crude product was obtained. After separation and purification by a flash column (dichloromethane/methanol: 1.5-2.0%), 100 mg of pure product was obtained with a yield of 28.8%.
**¹HNMR (DMSO-*d*₆, 600 MHz) δ (ppm)** 12.50(1H, s), 10.26(1H, s), 9.00(1H, s), 6.44(1H, d, *J =* 8.4 Hz), 6.24(1H, d, *J =* 3.0 Hz), 6.06(1H, dd, *J =* 8.4, 3.0 Hz), 4.22(1H, d, *J =* 8.4 Hz), 3.02-3.11(2H, m), 2.85(1H, q), 2.45(1H, d, *J* = 16.2 Hz), 1.62-1.69(2H, m), 0.97(3H, t, *J* = 7.2 Hz);
**¹³CNMR (DMSO-*d*₆, 150 MHz) δ (ppm)** 171.47, 163.22, 157.43, 126.89, 122.18, 106.20, 100.00, 37.69, 31.95, 27.89, 22.97, 13.68;
**MS (ESI+) m/z** 348.1 [M+H] +.

### Example 20:

### Preparation of 2-allylmercapto-5-(4-propoxyphenyl)-5,8-dihydropyrido[2,3-d]pyrimidin-4,7(3H,6H)-dione (LSL-9-14)

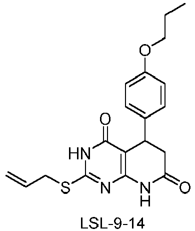

2-allylmercapto-6-aminopyrimidin-4(3H)-one (183 mg, 1.0 mmol), 2,2-dimethyl-1,3-dioxane-4,6-dione (173 mg, 1.2 mmol) and 4-propoxybenzaldehyde (164 mg, 1.0 mmol) were dissolved in about 5 mL of ethylene glycol, and heated to react under an oil bath at 210°C for about 3 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 65%), 70 mg of pure product was obtained with a yield of 18.9%.
**¹HNMR (DMSO-*d*₆, 600 MHz) δ (ppm)** 12.56(1H, s), 10.53(1H, s), 7.04(2H, d, *J =* 8.4 Hz), 6.83(2H, d, *J=* 8.4 Hz), 5.89-5.97(1H, m), 5.39(1H, d, *J=* 16.8 Hz), 5.13(1H, d, *J=* 9.6 Hz), 4.14(1H, d, *J* = 7.8 Hz), 3.86(2H, t, *J* = 6.6 Hz), 3.77-3.84(2H, m), 3.00(1H, q), 2.49(1H, d, *J*= 16.8 Hz), 1.65-1.72(2H, m), 0.95(3H, t, *J=* 7.2 Hz);
**¹³CNMR (DMSO-*d*₆, 150 MHz) δ (ppm)** 170.03, 157.96, 134.69, 133.77, 127.92 × 2, 119.22, 115.00 × 2, 69.37, 38.68, 32.79, 32.65, 22.49, 10.86;
**MS (ESI+) m/z** 372.1 [M+H] +.

### Example 21:

### Preparation of 2-allylmercapto-5-(3-hydroxy-4-methoxyphenyl)-5,8-dihydropyrido[2,3-d]pyrimidin-4,7(3H,6H)-dione (LSL-9-15)

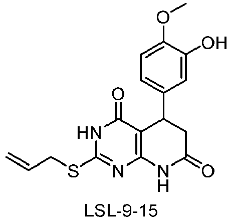

2-allylmercapto-6-aminopyrimidin-4(*3H*)-one (183 mg, 1.0 mmol), 2,2-dimethyl-1,3-dioxane-4,6-dione (173 mg, 1.2 mmol) and 3-hydroxy-4-methoxybenzaldehyde (152 mg, 1.0 mmol) were dissolved in about 5 mL of ethylene glycol, and heated to react under an oil bath at 210°C for about 2 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product was obtained. After separation and purification by a flash column (dichloromethane/methanol: 1.5%), 95 mg of pure product was obtained with a yield of 26.5%.
**¹HNMR (DMSO-*d*₆, 600 MHz) δ (ppm)** 12.58(1H, s), 10.50(1H, s), 8.89(1H, s), 6.80(1H, d, *J=* 8.4 Hz), 6.57(1H, d, *J=* 1.8 Hz), 6.52(1H, dd, *J=* 8.4, 1.8 Hz), 5.89-5.97(1H, m), 5.39(1H, d, *J* = 16.8 Hz), 5.13(1H, d, *J* = 9.6 Hz), 4.06(1H, d, *J* = 7.8 Hz), 3.77-3.85(2H, m), 3.70(3H, s), 2.97(1H, q), 2.47(1H, d, *J* = 16.8 Hz).
**¹³CNMR (DMSO-*d*₆, 150 MHz) δ (ppm)** 169.96, 145.88, 145.85, 134.48, 130.91, 128.05, 116.34, 113.25, 111.95, 55.10, 39.47, 37.65, 31.72;
**MS (ESI+) m/z** 360.1 [M+H] +.

### Example 22:

### Preparation of 2-allylmercapto-5-(2-hydroxy-4-propoxyphenyl)-5,8-dihydropyrido[2,3-d]pyrimidin-4,7(3H,6H)-dione (LSL-9-15)

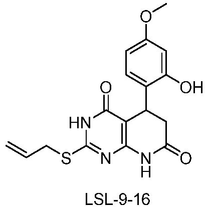

2-allylmercapto-6-aminopyrimidin-4(*3H*)-one (183 mg, 1.0 mmol), 2,2-dimethyl-1,3-dioxane-4,6-dione (173 mg, 1.2 mmol) and 2-hydroxy-4-methoxybenzaldehyde (152 mg, 1.0 mmol) were dissolved in about 5 mL of ethylene glycol, and heated to react under an oil bath at 200°C for about 2.5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (dichloromethane/methanol: 1.5%), 103 mg of pure product was obtained with a yield of 28.7%.
**¹HNMR (DMSO-*d*₆, 600 MHz) δ (ppm)** 12.60(1H, s), 10.46(1H, s), 9.71(1H, s), 6.51(1H, d, *J* = 7.8 Hz), 6.40(1H, d, *J* = 2.4 Hz), 6.24(1H, dd, *J* = 8.4, 2.4 Hz), 5.91-5.98(1H, m), 5.40(1H, d, *J=* 16.8 Hz), 5.14(1H, d, *J=* 10.2 Hz), 4.30(1H, d, *J=* 7.2 Hz), 3.79-3.87(2H, m), 3.64(3H, s), 2.89(1H, q), 2.47(1H, d, *J=* 16.2 Hz);
**¹³CNMR (DMSO-*d*₆, 150 MHz) δ (ppm)** 170.13, 158.46, 155.01, 130.92, 128.06, 126.21, 119.42, 103.42, 101.23, 54.28, 39.52, 36.47, 31.73;
**MS (ESI+) m/z** 360.1 [M+H] +.

### Example 23:

### Preparation of 2-allylmercapto-5-(3,4-dimethoxyphenyl)-5,8-dihydropyrido[2,3-d]pyrimidin-4,7(3H,6H)-dione (LSL-9-17)

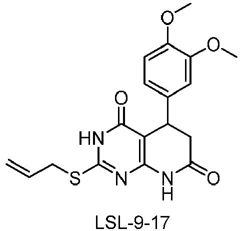

2-allylmercapto-6-aminopyrimidin-4(*3H*)-one (183 mg, 1.0 mmol), 2,2-dimethyl-1,3-dioxane-4,6-dione (173 mg, 1.2 mmol) and 3,4-dimethoxybenzaldehyde (166 mg, 1.0 mmol) were dissolved in about 5 mL of ethylene glycol, and heated to react under an oil bath at 170°C for about 2.5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 65%), 108 mg of pure product was obtained with a yield of 29.0%.
**¹HNMR (DMSO-*d*₆, 600 MHz) δ (ppm)** 12.60(1H, s), 10.52(1H, s), 6.87(1H, d, *J =* 1.8 Hz), 6.82(1H, d, *J* = 8.4 Hz), 6.53(1H, dd, *J* = 7.8, 1.8 Hz), 5.89-5.96(1H, m), 5.39(1H, d, *J* = 16.2 Hz), 5.13(1H, d, *J =* 10.2 Hz), 4.15(1H, d, *J =* 7.8 Hz), 3.78-3.85(2H, m), 3.70(3H, s), 3.69(3H, s), 2.99(1H, q), 2.54(1H, d, *J=* 16.8 Hz);
**¹³CNMR (DMSO-*d*₆, 150 MHz) δ (ppm)** 170.03, 148.23, 147.12, 134.25, 132.69, 128.06, 116.96, 111.29, 110.55, 54.93, 54.83, 39.47, 37.47, 31.92;
**MS (ESI+) m/z** 374.1 [M+H] +.

### Example 24:

### Preparation of 2-propylmercapto-5-(4-iodophenyl)-5,8-dihydropyrido[2,3-d]pyrimidin-4,7(3H,6H)-dione (LSL-9-19)

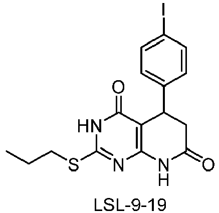

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-iodobenzaldehyde (233 mg, 1.0 mmol) were dissolved in about 5 mL of ethylene glycol, and heated to react under an oil bath at 210°C for about 2.5 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 65%), 235 mg of pure product was obtained with a yield of 53.2%.
**¹HNMR (DMSO-*d*₆, 600 MHz) δ (ppm)** 12.92(1H, s), 10.28(1H, s), 7.62(2H, d, *J =* 8.4 Hz), 6.97(2H, d, *J* = 8.4 Hz), 4.14(1H, d, *J* = 7.8 Hz), 3.69(3H, s), 3.00-3.08(2H, m), 2.97(1H, q), 2.47(1H, d, *J=* 16.2 Hz), 1.60-1.67(2H, m), 0.96(3H, t, *J=* 7.2 Hz);
**¹³CNMR (DMSO-*d*₆, 150 MHz) δ (ppm)** 170.78, 155.17, 143.53, 137.85 × 2, 129.52 × 2, 97.93, 92.64, 38.50, 33.37, 31.93, 22.99, 13.72;
**MS (ESI+) m/z** 442.0 [M+H] +.

### Example 25:

### Preparation of 2-propylmercapto-5-(4-methoxyphenyl)-5,8-dihydropyrido[2,3-d]pyrimidin-4,7(3H,6H)-dione (LSL-9-21)

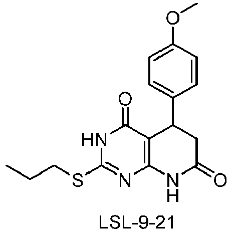

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-methoxybenzaldehyde (173 mg, 1.0 mmol) were dissolved in about 5 mL of ethylene glycol, and heated to react under an oil bath at 210°C for about 3 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 65%), 71 mg of pure product was obtained with a yield of 20.6%.
**¹HNMR (DMSO-*d*₆, 600 MHz) δ (ppm)** 12.70(1H, s), 10.39(1H, s), 7.06(2H, d, *J =* 9.0 Hz), 6.83(2H, d, *J=* 9.0 Hz), 4.13(1H, d, *J=* 7.2 Hz), 3.69(3H, s), 3.03-3.12(2H, m), 2.97(1H, q), 2.48(1H, d, *J=* 16.8 Hz), 1.62-1.69(2H, m), 0.97(3H, t, *J=* 7.2 Hz);
**¹³CNMR (DMSO-*d*₆, 150 MHz) δ (ppm)** 171.03, 158.46, 127.96 × 2, 114.41 × 2, 100.00, 55.51, 38.80, 32.73, 31.96, 22.90, 13.65;
**MS (ESI+) m/z** 346.1 [M+H] +.

### Example 26:

### Preparation of 2-propylmercapto-5-(2,3-dihydroxy-4-methoxyphenyl)-5,8-dihydropyrido[2,3-d]pyrimidin-4,7(3H,6H)-dione (LSL-9-23)

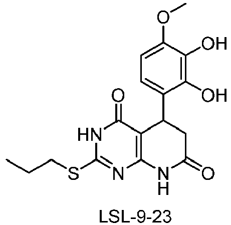

2-propylmercapto-6-aminopyrimidin-4(*3H*)-one (185 mg, 1.0 mmol), 2,2-dimethyl-1,3-dioxane-4,6-dione (173 mg, 1.2 mmol) and 2,3-dihydroxy-4-methoxybenzaldehyde (168 mg, 1.0 mmol) were dissolved in about 5 mL of ethylene glycol, and heated to react under an oil bath at 210°C for about 3 hours, and TLC detected (petroleum ether/ethyl acetate = 1/3) that the raw material points disappeared. The reaction solution was poured into an appropriate amount of ice water, so that a flocculent precipitate was produced, which was filtered to obtain a crude product. After separation and purification by a flash column (petroleum ether/ethyl acetate: 70%), 120 mg of pure product was obtained with a yield of 31.8%.
**¹HNMR (DMSO-*d*₆, 600 MHz) δ (ppm)** 12.56(1H, s), 10.38(1H, s), 9.02(1H, s), 8.39(1H, s), 6.31(1H, d, *J* = 8.4 Hz), 6.09(1H, d, *J* =8.4 Hz), 4.32(1H, d, *J* = 8.4 Hz), 3.69(3H, s), 3.05-3.14(2H, m), 2.89(1H, q), 2.47(1H, d, *J=* 16.8 Hz), 1.63-1.70(2H, m), 0.98(3H, t, *J=* 7.2 Hz);
**¹³CNMR (DMSO-*d*₆, 150 MHz) δ (ppm)** 171.32, 147.69, 143.93, 135.10, 115.54, 103.40, 100.00, 56.27, 37.57, 31.99, 28.01, 22.90, 13.65;
**MS (ESI+) m/z** 378.1 [M+H] +.

### Example 27:

### Preparation of 2-propylmercapto-5-(3-hydroxy-4-propoxyphenyl)-5,8-dihydropyrido[2,3-d]pyrimidin-4,7(3H,6H)-dione (LSL-9-25)

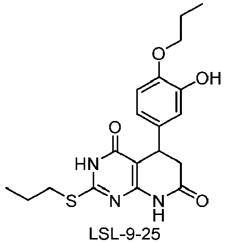

2-propylmercapto-6-aminopyrimidin-4(*3H*)-one (185 mg, 1.0 mmol), 2,2-dimethyl-1,3-dioxane-4,6-dione (173 mg, 1.2 mmol) and 3-hydroxy-4-propoxybenzaldehyde (152 mg, 1.0 mmol) were dissolved in about 5 mL of ethylene glycol, and heated to react under an oil bath at 180°C for about 3.5 hours, and TLC detected (petroleum ether/ethyl acetate = 1/3) that the raw material points disappeared. The reaction solution was poured into an appropriate amount of ice water, and a flocculent precipitate was produced, which was filtered to obtain a crude product. After separation and purification by a flash column (dichloromethane/methanol: 2.5%), 110 mg of pure product was obtained with a yield of 28.3%.
**¹HNMR (DMSO-*d*₆, 600 MHz) δ (ppm)** 12.55(1H, s), 10.40(1H, s), 8.78(1H, s), 6.78(1H, d, *J =* 8.4 Hz), 6.58(1H, d, *J* =2.4 Hz), 6.51(1H, dd, *J* =8.4, 2.4 Hz),4.04(1H, d, *J =* 7.8 Hz), 3.84(2H, t, *J* = 6.6 Hz), 3.03-3.14(2H, m), 2.94(1H, q), 2.45(1H, d, *J =* 16.2 Hz), 1.62-1.72(4H, m), 0.97(3H, t, *J* = 7.2 Hz), 0.95(3H, t, *J* = 7.2 Hz);
**¹³CNMR (DMSO-*d*₆, 150 MHz) δ (ppm)** 171.04, 147.27, 146.11, 135.78, 117.49, 114.47, 100.00, 70.50, 38.83, 32.86, 31.96, 22.89, 22.62, 13.65, 10.88;
**MS (ESI+) m/z** 390.1 [M+H] +.

### Example 28:

### Preparation of 2-propylmercapto-5-(4-bromophenyl)-5,8-dihydropyrido[2,3-d]pyrimidin-4,7(3H,6H)-dione (LSL-9-26)

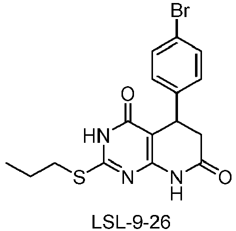

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-bromobenzaldehyde (185 mg, 1.0 mmol) were dissolved in about 5 mL of ethylene glycol, and heated to react under an oil bath at 200°C for about 2.5 hours, and TLC detected (petroleum ether/ethyl acetate = 1/3) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 65%), 135 mg of pure product was obtained with a yield of 34.4%.
**¹HNMR (DMSO-*d*₆, 600 MHz) δ (ppm)** 12.63(1H, s), 10.48(1H, s), 7.47(2H, dt, *J* = 8.4, 1.8 Hz), 7.11(2H, dt, *J* = 8.4, 1.8 Hz), 4.18(1H, d, *J* = 7.8 Hz), 3.04-3.14(2H, m), 3.02(1H, q), 2.50(1H, d, *J=* 16.2 Hz), 1.62-1.69(2H, m), 0.97(3H, t, *J=* 7.2 Hz);
**¹³CNMR (DMSO-*d*₆, 150 MHz) δ (ppm)** 170.74, 142.63, 131.91 × 2, 129.28 × 2, 120.15, 38.35, 33.11, 31.99, 22.87, 13.65;
**MS (ESI+) m/z** 394.0 [M+H] +.

### Example 29:

### Preparaiton of 2-propylmercapto-5-(4-fluorophenyl)-5,8-dihydropyrido[2,3-d]pyrimidin-4,7(3H,6H)-dione (LSL-9-27)

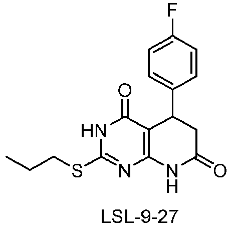

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-fluorobenzaldehyde (124 mg, 1.0 mmol) were dissolved in about 5 mL of ethylene glycol, and heated to react under an oil bath at 180°C for about 2.5 hours, and TLC detected (petroleum ether/ethyl acetate = 1/3) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 65%), 125 mg of pure product was obtained with a yield of 37.5%.
**¹HNMR (DMSO-*d*₆, 600 MHz) δ (ppm)** 12.59(1H, s), 10.48(1H, s), 7.17-7.21(2H, m), 7.08-7.12(2H, m), 4.20(1H, d, *J=* 7.8 Hz), 3.04-3.14(2H, m), 3.02(1H, q), 2.51(1H, d, *J=* 16.8 Hz), 1.63-1.69(2H, m), 0.97(3H, t, *J=* 7.2 Hz);
**¹³CNMR (DMSO-*d*₆, 150 MHz) δ (ppm)** 170.84, 160.66, 139.29, (128.85, 128.80, *J* _{C-F}= 7.95 Hz) × 2, (115.80, 115.66, *J* _{C-F}= 2.10 Hz) × 2, 38.61, 32.85, 31.98, 22.86, 13.64;
**MS (ESI+) m/z** 334.1 [M+H] +.

### Example 30:

### Preparation of 2-propylmercapto-5-(2-hydroxy-4-propoxyphenyl)-5,8-dihydropyrido[2,3-d]pyrimidin-4,7(3H,6H)-dione (LSL-9-29)

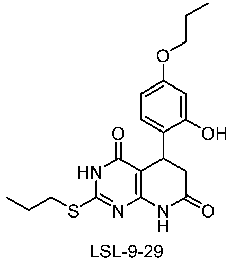

2-propylmercapto-6-aminopyrimidin-4(*3H*)-one (185 mg, 1.0 mmol), 2,2-dimethyl-1,3-dioxane-4,6-dione (173 mg, 1.2 mmol) and 2-hydroxy-4-propoxybenzaldehyde (152 mg, 1.0 mmol) were dissolved in about 5 mL of ethylene glycol, and heated to react under an oil bath at 185°C for about 5 hours, and TLC detected (petroleum ether/ethyl acetate = 1/3) that the raw material points disappeared. The reaction solution was poured into an appropriate amount of ice water, and a flocculent precipitate was produced, which was filtered to obtain a crude product. After separation and purification by a flash column (dichloromethane/methanol: 2%), 120 mg of pure product was obtained with a yield of 30.8%.
**¹HNMR (DMSO-*d*₆, 600 MHz) δ (ppm)** 12.48(1H, s), 10.36(1H, s), 9.96(1H, s), 6.53(1H, d, *J =* 8.4 Hz), 6.37(1H, d, *J* =1.8 Hz), 6.23(1H, dd, *J* =8.4, 1.8 Hz), 4.28(1H, d, *J =* 7.8 Hz), 3.79(2H, t, *J* = 6.6 Hz), 3.04-3.14(2H, m), 2.88(1H, q), 2.46(1H, d, *J* = 16.2 Hz), 1.63-1.70(4H, m), 0.98(3H, t, *J=* 7.2 Hz), 0.93(3H, t, *J=* 7.2 Hz);
**¹³CNMR (DMSO-*d*₆, 150 MHz) δ (ppm)** 171.29, 158.90, 105.49, 105.15, 103.27, 102.97, 102.12, 69.22, 37.57, 31.98, 27.93, 22.91, 22.49, 13.66, 10.86;
**MS (ESI+) m/z** 390.1 [M+H] +.

### Example 31:

### Preparation of 2-propylmercapto-5-(4-benzyloxyphenyl)-5,8-dihydropyrido[2,3-d]pyrimidin-4,7(3H,6H)-dione (LSL-9-31)

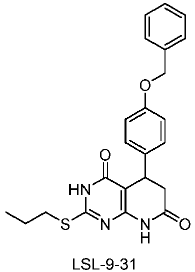

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-benzyloxybenzaldehyde (212 mg, 1.0 mmol) were dissolved in about 5 mL of ethylene glycol, and heated to react under an oil bath at 200°C for about 2.5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 50%), 70 mg of pure product was obtained with a yield of 19.3%.
**¹HNMR (DMSO-*d*₆, 600 MHz) δ (ppm)** 12.14(1H, s), 10.07(1H, s), 7.87(2H, dt, *J* = 9.0, 1.8 Hz), 7.47(2H, d, *J* = 7.2 Hz), 7.41(2H, t, *J* = 7.2 Hz), 7.35(1H, t, *J* = 7.2 Hz), 7.21(2H, dt, *J* = 9.0, 1.8 Hz), 5.03(2H, s), 4.09(1H, d, *J=* 7.8 Hz), 2.95-3.01(2H, m), 2.86(1H, q), 2.45(1H, d, *J=* 16.8 Hz), 1.59-1.66(2H, m), 0.95(3H, t, *J=* 7.2 Hz);
**¹³CNMR (DMSO-*d*₆, 150 MHz) δ (ppm)** 170.76, 163.77, 136.80, 132.27 × 2, 130.28, 128.99 × 2, 128.55, 128.32 × 2, 115.78 × 2, 70.15, 38.47, 33.22, 31.86, 23.09, 13.78;
**MS (ESI+) m/z** 422.1 [M+H] +.

### Example 32:

### Preparatiom of 2-propylmercapto-5-(4-phenoxyphenyl)-5,8-dihydropyrido[2,3-d]pyrimidin-4,7(3H,6H)-dione (LSL-9-32)

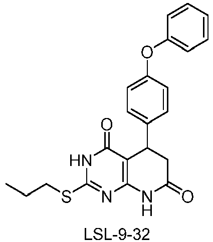

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-phenoxybenzaldehyde (212 mg, 1.0 mmol) were dissolved in about 5 mL of ethylene glycol, and heated to react under an oil bath at 190°C for about 7 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 65%), 120 mg of pure product was obtained with a yield of 28.5%.
**¹HNMR (DMSO-*d*₆, 600 MHz) δ (ppm)** 12.14(1H, s), 10.07(1H, s), 7.35(2H, t, *J =* 8.4 Hz), 7.18(2H, d, *J* = 8.4 Hz), 7.10(1H, t, *J* = 7.6 Hz), 6.95(2H, d, *J* = 7.8 Hz), 6.90(2H, d, *J* = 8.4 Hz), 4.17(1H, d, *J =* 7.8 Hz), 2.98-3.04(2H, m), 2.93(1H, q), 2.50(1H, d, *J =* 16.8 Hz), 1.59-1.66(2H, m), 0.95(3H, t, *J=* 7.2 Hz);
**¹³CNMR (DMSO-*d*₆, 150 MHz) δ (ppm)** 171.03, 157.41, 130.43 × 2, 128.65 × 2, 123.59, 119.22 × 2, 118.67 × 2, 114.17, 100.00, 38.47, 33.22, 31.86, 23.09, 13.78;
**MS (ESI+) m/z** 408.1 [M+H] +.

### Example 33:

### Preparation of 2-propylmercapto-5-(3,4,5-trihydroxyphenyl)-5,8-dihydropyrido[2,3-d]pyrimidin-4,7(3H,6H)-dione (LSL-9-33)

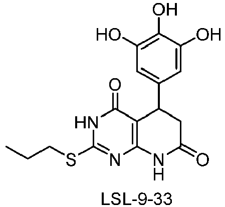

2-propylmercapto-6-aminopyrimidin-4(*3H*)-one (185 mg, 1.0 mmol), 2,2-dimethyl-1,3-dioxane-4,6-dione (173 mg, 1.2 mmol) and 3,4,5-trihydroxybenzaldehyde (154mg, 1.0 mmol) were dissolved in about 5 mL of ethylene glycol, and heated to react under an oil bath at 200°C for about 3 hours, and TLC detected (petroleum ether/ethyl acetate = 1/3) that the raw material points disappeared. The reaction solution was poured into an appropriate amount of ice water, and a flocculent precipitate was produced, which was filtered to obtain a crude product. After separation and purification by a flash column (dichloromethane/methanol: 5%), 50 mg of pure product was obtained with a yield of 13.8%.
**¹HNMR (DMSO-*d*₆, 600 MHz) δ (ppm)** 12.50(1H, s), 10.43(1H, s), 8.70(2H, s), 7.89(1H, s), 6.07(2H, s), 3.94(1H, d, *J* = 7.8 Hz), 3.04-3.17(2H, m), 2.92(1H, q), 2.42(1H, d, *J* = 16.2 Hz), 1.63-1.70(2H, m), 0.97(3H, t, *J=* 7.2 Hz);
**¹³CNMR (DMSO-*d*₆, 150 MHz) δ (ppm)** 171.07, 146.42 × 2, 133.00, 132.20, 105.71 × 2, 38.97, 32.97, 31.96, 22.86, 13.64;
**MS (ESI+) m/z** 364.1 [M+H] +.

### Example 34:

### Preparation of 2-propylmercapto-5-(4-trifluoromethoxyphenyl)-5,8-dihydropyrido[2,3-d]pyrimidin-4,7(3H,6H)-dione (LSL-9-35)

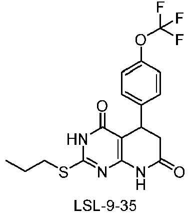

2-propylmercapto-6-aminopyrimidin-4(3H)-one (185 mg, 1.0 mmol), 2,2-dimethyl-1,3-dioxane-4,6-dione (173 mg, 1.2 mmol) and 4-trifluoromethoxybenzaldehyde (190 mg, 1.0 mmol) were dissolved in about 5 mL of ethylene glycol, and heated to react under an oil bath at 210°C for about 6 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 60%), 130 mg of pure product was obtained with a yield of 32.6%.
**¹HNMR (DMSO-*d*₆, 600 MHz) δ (ppm)** 12.58(1H, s), 10.18(1H, s), 7.28(2H, d, *J =* 8.4 Hz), 7.25(2H, d, *J=* 8.4 Hz), 4.20(1H, d, *J=* 7.8 Hz), 2.99-3.06(2H, m), 2.97(1H, q), 2.50(1H, d, *J=* 16.8 Hz), 1.60-1.66(2H, m), 0.95(3H, t, *J=* 7.2 Hz);
**¹³CNMR (DMSO-*d*₆, 150 MHz) δ (ppm)** 170.81, 147.33, 128.90 × 2, 121.50 × 2, 100.00, 97.94, 38.68, 33.32, 31.89, 23.06, 13.76;
**MS (ESI+) m/z** 400.1 [M+H] +.

### Example 35:

### Preparation of 2-propylmercapto-5-(quinolin-6-yl)-5,8-dihydropyrido[2,3-d]pyrimidin-4,7(3H,6H)-dione (LSL-9-36)

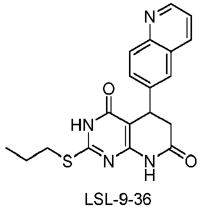

2-propylmercapto-6-aminopyrimidin-4(*3H*)-one (185 mg, 1.0 mmol), 2,2-dimethyl-1,3-dioxane-4,6-dione (173 mg, 1.2 mmol) and 6-formylquinoline (157 mg, 1.0 mmol) were dissolved in about 5 mL of ethylene glycol, and heated to react under an oil bath at 200°C for about 2 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (dichloromethane/methanol: 2.3%), 60 mg of pure product was obtained with a yield of 16.4%.
**¹HNMR (DMSO-*d*₆, 600 MHz) δ (ppm)** 12.59(1H, s), 10.60(1H, s), 8.84(1H, dd, *J* = 4.2, 1.2 Hz), 8.31(1H, d, *J* = 7.8 Hz), 7.97(1H, d, *J* = 8.4 Hz), 7.65(1H, dd, *J* = 3.0, 1.2 Hz), 7.62(1H, s), 7.47-7.50(1H, q), 4.41(1H, d, *J* = 7.8 Hz), 3.12-3.19(2H, m), 3.09(1H, q), 2.66(1H, d, *J* = 16.8 Hz), 1.65-1.72(2H, m), 0.98(3H, t, *J=* 7.2 Hz);
**¹³CNMR (DMSO-*d*₆, 150 MHz) δ (ppm)** 170.80, 150.71, 147.38, 141.16, 136.33, 129.98, 129.64, 128.20, 124.89, 122.09, 38.46, 33.62, 32.03, 22.83, 13.65;
**MS (ESI+) m/z** 367.1 [M+H] +.

### Example 36:

### Preparation of 2-propylmercapto-5-(4-methylphenyl)-5,8-dihydropyrido[2,3-d]pyrimidin-4,7(3H,6H)-dione (LSL-9-37)

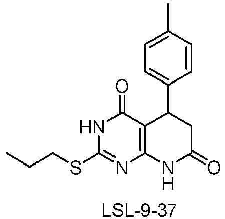

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-methylbenzaldehyde (120 mg, 1.0 mmol) were dissolved in about 5 mL of ethylene glycol, and heated to react under an oil bath at 200°C for about 6 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 60%), 70 mg of pure product was obtained with a yield of 19.3%.
**¹HNMR (DMSO-*d*₆, 600 MHz) δ (ppm)** 12.44(1H, s), 10.19(1H, s), 7.06(2H, d, *J =* 8.4 Hz), 7.03(2H, d, *J=* 7.8 Hz), 4.13(1H, d, *J=* 7.2 Hz), 2.99-3.08(2H, m), 2.94(1H, q), 2.47(1H, d, *J=* 16.2 Hz), 1.62-1.66(2H, m), 0.96(3H, t, *J=* 7.2 Hz);
**¹³CNMR (DMSO-*d*₆, 150 MHz) δ (ppm)** 171.03, 135.91, 129.42 × 2, 126.90 × 2, 38.89, 33.31, 31.90, 23.00, 21.02, 13.72;
**MS (ESI+) m/z** 330.1 [M+H] +.

### Example 37:

### Preparation of 2-propylmercapto-5-(4-trifluoromethylphenyl)-5,8-dihydropyrido[2,3-d]pyrimidin-4,7(3H,6H)-dione (LSL-9-38)

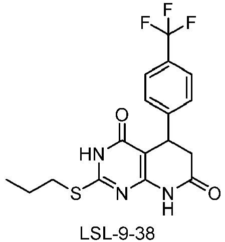

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-trifluoromethylbenzaldehyde (174 mg, 1.0 mmol) were dissolved in about 5 mL of ethylene glycol, and heated to react under an oil bath at 200°C for about 6 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (petroleum ether/ethyl acetate: 60%), 104 mg of pure product was obtained with a yield of 27.2%.
**¹HNMR (DMSO-*d*₆, 600 MHz) δ (ppm)** 12.63(1H, s), 10.62(1H, s), 7.66(2H, d, *J =* 8.4 Hz), 7.38(2H, d, *J=* 8.4 Hz), 4.30(1H, d, *J=* 7.8 Hz), 3.09-3.16(2H, m), 3.08(1H, q), 2.55(1H, d, *J=* 16.2 Hz), 1.64-1.70(2H, m), 0.97(3H, t, *J=* 7.2 Hz);
**¹³CNMR (DMSO-*d*₆, 150 MHz) δ (ppm)** 170.63, 147.86, 127.87 × 2, 126.07, 38.17, 33.51, 32.00, 22.81, 13.62;
**MS (ESI+) m/z** 384.1 [M+H] +.

### Example 38:

### Preparation of 2-propylmercapto-5-(4-ethoxyphenyl)-5,8-dihydropyrido[2,3-d]pyrimidin-4,7(3H,6H)-dione (LSL-9-39)

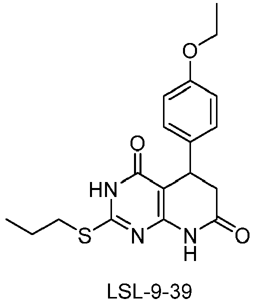

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-ethoxybenzaldehyde (150 mg, 1.0 mmol) were dissolved in about 5 mL of ethylene glycol, and heated to react under an oil bath at 200°C for about 6 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (dichloromethane/methanol: 2%), 50 mg of pure product was obtained with a yield of 13.9%.
**¹HNMR (DMSO-*d*₆, 600 MHz) δ (ppm)** 12.54(1H, s), 10.49(1H, s), 7.04(2H, d, *J =* 8.4 Hz), 6.82(2H, d, *J=* 7.8 Hz), 4.13(1H, d, *J=* 7.2 Hz), 3.96(2H, q), 3.06-3.15(2H, m), 2.99(1H, q), 2.50(1H, d, *J=* 16.2 Hz), 1.63-1.72(2H, m), 1.29(3H, t, *J=* 6.6 Hz), 0.97(3H, t, *J=* 7.2 Hz);
**¹³CNMR (DMSO-*d*₆, 150 MHz) δ (ppm)** 171.04, 157.78, 134.73, 127.93 × 2, 114.95 × 2, 63.41, 38.71, 32.64, 31.99, 22.84, 15.11, 13.62;
**MS (ESI+) m/z** 360.1 [M+H] +.

### Example 39:

### Preparation of 2-propylmercapto-5-(4-methoxy-3-nitrophenyl)-5,8-dihydropyrido[2,3-d]pyrimidin-4,7(3H,6H)-dione (LSL-9-40)

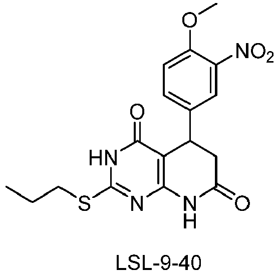

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-methoxy-3-nitrobenzaldehyde (181 mg, 1.0 mmol) were dissolved in about 5 mL of ethylene glycol, and heated to react under an oil bath at 200°C for about 5 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (dichloromethane/methanol: 1.5%), 70 mg of pure product was obtained with a yield of 17.9%.
**¹HNMR (DMSO-*d*₆, 600 MHz) δ (ppm)** 12.62(1H, s), 10.60(1H, s), 7.65(1H, d, *J =* 2.4 Hz), 7.42(1H, dd, *J=* 8.4, 1.8 Hz), 7.30(1H, d, *J=* 8.4 Hz), 4.248(1H, d, *J=* 7.8 Hz), 3.88(3H, s), 3.05-3.16(2H, m), 3.05(1H, q), 2.56(1H, d, *J* = 16.8 Hz), 1.63-1.70(2H, m), 0.97(3H, t, *J* = 7.8 Hz);
**¹³CNMR (DMSO-*d*₆, 150 MHz) δ (ppm)** 170.66, 151.34, 139.56, 135.40, 132.83, 123.29, 115.22, 57.16, 38.18, 32.40, 32.02, 22.81, 13.62;
MS (ESI+) m/z 391.1 [M+H] +.

### Example 40:

### Preparation of 2-propylmercapto-5-(4-methoxy-2-nitrophenyl)-5,8-dihydropyrido[2,3-d]pyrimidin-4,7(3H,6H)-dione (LSL-9-41)

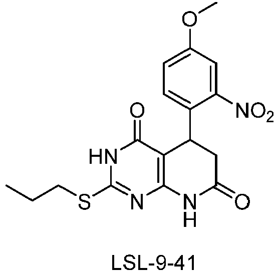

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-methoxy-2-nitrobenzaldehyde (181 mg, 1.0 mmol) were dissolved in about 5 mL of ethylene glycol, and heated to react under an oil bath at 200°C for about 2 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (dichloromethane/methanol: 1.3%), 50 mg of pure product was obtained with a yield of 12.8%.
¹HNMR (DMSO-*d*₆, 600 MHz) δ (ppm) 12.60(1H, s), 10.70(1H, s), 7.49(1H, d, *J* = 2.4 Hz), 7.21(1H, dd, *J =* 8.4, 2.4 Hz), 7.02(1H, d, *J =* 8.4 Hz), 4.48(1H, d, *J =* 8.4 Hz), 3.81(3H, s), 3.20(1H, q), 3.07-3.17(2H, m), 2.43(1H, d, *J* = 16.8 Hz), 1.65-1.72(2H, m), 0.97(3H, t, *J=* 7.2 Hz);
¹³CNMR (DMSO-*d*₆, 150 MHz) δ (ppm) 170.14, 158.82, 149.67, 129.35, 128.74, 120.50, 110.06, 56.40, 38.34, 32.04, 29.24, 22.82, 13.64;
MS (ESI+) m/z 391.1 [M+H] +.

### Example 41:

### Preparation of 2-propylmercapto-5-(3-methoxyphenyl)-5,8-dihydropyrido[2,3-d]pyrimidin-4,7(3H,6H)-dione (LSL-9-47)

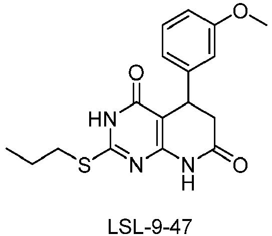

2-propylmercapto-6-aminopyrimidin-4(3H)-one (185 mg, 1.0 mmol), 2,2-dimethyl-1,3-dioxane-4,6-dione (173 mg, 1.2 mmol) and 3-methoxybenzaldehyde (136 mg, 1.0 mmol) were dissolved in about 5 mL of ethylene glycol, and heated to react under an oil bath at 200°C for about 4 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (dichloromethane/methanol: 1.5%), 100 mg of pure product was obtained with a yield of 29.0%.
**¹HNMR (DMSO-*d*₆, 600 MHz) δ (ppm)** 12.57(1H, s), 10.51(1H, s), 7.19(1H, t, *J =* 8.4 Hz), 6.78(1H, dd, *J=* 8.4, 1.8 Hz), 6.73(1H, t, *J=* 1.8 Hz), 6.70(1H, d, *J=* 7.8 Hz), 4.17(1H, d, *J* = 7.8 Hz), 3.70(3H, s), 3.06-3.15(2H, m), 3.02(1H, q), 2.52(1H, d, *J=* 16.8 Hz), 1.63-1.70(2H, m), 0.97(3H, t, *J* = 7.2 Hz);
**¹³CNMR (DMSO-*d*₆, 150 MHz) δ (ppm)** 170.96, 159.89, 144.62, 130.16, 118.89, 113.32, 112.09, 55.41, 38.45, 33.45, 32.00, 22.83, 13.61;
MS (ESI+) m/z 346.1 [M+H] +.

### Example 42: Preparation of 2-propylmercapto-5-(2-methoxyphenyl)-5,8-dihydropyrido[2,3-d]pyrimidin-4,7(3H,6H)-dione (LSL-9-48)

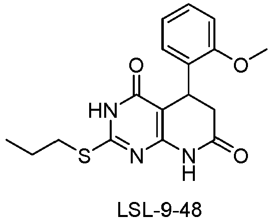

2-propylmercapto-6-aminopyrimidin-4(*3H*)-one (185 mg, 1.0 mmol), 2,2-dimethyl-1,3-dioxane-4,6-dione (173 mg, 1.2 mmol) and 2-methoxybenzaldehyde (136 mg, 1.0 mmol) were dissolved in about 5 mL of ethylene glycol, and heated to react under an oil bath at 200°C for about 5 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (dichloromethane/methanol: 1.3%), 70 mg of pure product was obtained with a yield of 20.3%.
**¹HNMR (DMSO-*d*₆, 600 MHz) δ (ppm)** 12.53(1H, s), 10.45(1H, s), 7.21(1H, td, *J* = 7.8, 1.8 Hz), 7.00(1H, d, *J* = 8.4 Hz), 6.81(1H, t, *J* = 7.8 Hz), 6.72(1H, d, *J* = 7.2 Hz), 4.41(1H, d, *J* = 8.4 Hz), 3.83(3H, s), 3.08-3.18(2H, m), 2.95(1H, q), 2.39(d, J = 16.8 Hz), 1.65-1.72(2H, m), 0.99(3H, t, *J* = 7.8 Hz);
**¹³CNMR (DMSO-*d*₆, 150 MHz) δ (ppm)** 170.86, 157.05, 129.65, 128.55, 126.87, 120.66, 111.59, 55.76, 37.54, 32.01, 28.62, 22.86, 13.65;
MS (ESI+) m/z 346.1 [M+H] +.

### Example 43:

### Preparation of 2-propylmercapto-5-(3-hydroxyphenyl)-5,8-dihydropyrido[2,3-d]pyrimidin-4,7(3H,6H)-dione (LSL-9-49)

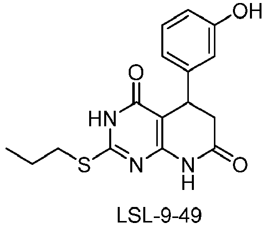

2-propylmercapto-6-aminopyrimidin-4(3H)-one (185 mg, 1.0 mmol), 2,2-dimethyl-1,3-dioxane-4,6-dione (173 mg, 1.2 mmol) and 3-hydroxybenzaldehyde (122 mg, 1.0 mmol) were dissolved in about 5 mL of ethylene glycol, and heated to react under an oil bath at 200°C for about 5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (dichloromethane/methanol: 3%), 70 mg of pure product was obtained with a yield of 22.4%.
**¹HNMR (DMSO-*d*₆, 600 MHz) δ (ppm)** 12.57(1H, s), 10.51(1H, s), 9.31(1H, s), 7.06(1H, t, *J* = 7.8 Hz), 6.60(1H, d, *J* = 7.8 Hz), 6.58(1H, dd, *J* = 7.8, 2.4 Hz), 6.53(1H, t, *J* = 2.4 Hz), 4.10(1H, d, *J=* 7.8 Hz), 3.06-3.17(2H, m), 3.00(1H, q), 2.48(1H, d, *J=* 16.8 Hz), 1.63-1.70(2H, m), 0.97(3H, t, *J* = 7.2 Hz);
**¹³CNMR (DMSO-*d*₆, 150 MHz) δ (ppm)** 170.96, 157.94, 144.46, 130.03, 117.67, 114.09, 113.72, 38.61, 33.43, 31.98, 22.85, 13.63;
MS (ESI+) m/z 332.1 [M+H] +.

### Example 44:

### Preparation of 2-propylmercapto-5-(3-cyanophenyl)-5,8-dihydropyrido[2,3-d]pyrimidin-4,7(3H,6H)-dione (LSL-10-02)

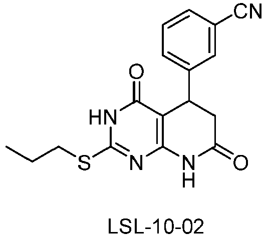

2-propylmercapto-6-aminopyrimidin-4(*3H*)-one (185 mg, 1.0 mmol), 2,2-dimethyl-1,3-dioxane-4,6-dione (173 mg, 1.2 mmol) and 3-cyanobenzaldehyde (131mg, 1.0 mmol) were dissolved in about 5 mL of ethylene glycol, and heated to react under an oil bath at 210°C for about 2 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (dichloromethane/methanol: 2.0%), 40 mg of pure product was obtained with a yield of 11.8%.
**¹HNMR (DMSO-*d*₆, 600 MHz) δ (ppm)** 12.62(1H, s), 10.61(1H, s), 7.70(1H, d, *J =* 7.2 Hz), 7.62(1H, s), 7.52(1H, t, *J=* 7.2 Hz), 7.48(1H, d, *J=* 7.8 Hz), 4.28(1H, d, *J=* 7.8 Hz), 3.10-3.17(2H, m), 3.07(1H, q), 2.58(1H, d, *J=* 16.2 Hz), 1.64-1.70(2H, m), 0.97(3H, t, *J=* 7.2 Hz);
**¹³CNMR (DMSO-*d*₆, 150 MHz) δ (ppm)** 170.58, 144.63, 131.92, 131.13, 130.78, 130.47, 119.22, 112.04, 38.11, 33.24, 32.04, 22.80, 13.63;
MS (ESI+) m/z 341.1 [M+H] +.

### Example 45:

### Preparation of 2-propylmercapto-5-(2-nitrophenyl)-5,8-dihydropyrido[2,3-d]pyrimidin-4,7(3H,6H)-dione (LSL-10-03)

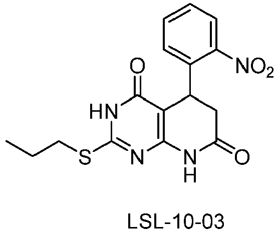

2-propylmercapto-6-aminopyrimidin-4(*3H*)-one (185 mg, 1.0 mmol), 2,2-dimethyl-1,3-dioxane-4,6-dione (173 mg, 1.2 mmol) and 2-nitrobenzaldehyde (151 mg, 1.0 mmol) were dissolved in about 5 mL of ethylene glycol, and heated to react under an oil bath at 200°C for about 3 hours, and TLC detected (petroleum ether/ethyl acetate = 1/2) that the raw material points disappeared. The reaction solution was poured into an appropriate amount of ice water, and a flocculent precipitate was produced, which was filtered to obtain a crude product. After separation and purification by a flash column (dichloromethane/methanol: 1.5%), 100 mg of pure product was obtained with a yield of 27.8%.
**¹HNMR (DMSO-*d*₆, 600 MHz) δ (ppm)** 12.61(1H, s), 10.73(1H, s), 7.95(1H, d, *J =* 8.4 Hz), 7.63(1H, d, *J* = 7.2 Hz), 7.50(1H, t, *J* = 7.2 Hz), 7.15(1H, d, *J* = 7.2 Hz), 4.58(1H, d, *J* = 8.4 Hz), 3.25(1H, q), 3.08-3.18(2H, m), 2.47(1H, d, *J* = 16.8 Hz), 1.67-1.71(2H, m), 0.99(3H, t, *J* = 7.2 Hz);
**¹³CNMR (DMSO-*d*₆, 150 MHz) δ (ppm)** 170.02, 149.09, 131.92, 137.14, 134.38, 128.88, 128.38, 125.31, 38.11, 32.05, 29.84, 22.81, 13.65;
MS (ESI+) m/z 361.1 [M+H] +.

### Example 46:

### Preparation of 2-propylmercapto-5-(4-hydroxy-3-nitrophenyl)-5,8-dihydropyrido[2,3-d]pyrimidin-4,7(3H,6H)-dione (LSL-10-04)

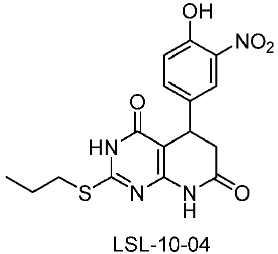

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-hydroxy-3-nitrobenzaldehyde (167 mg, 1.0 mmol) were dissolved in about 5 mL of ethylene glycol, and heated to react under an oil bath at 210°C for about 3 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (dichloromethane/methanol: 2.2%), 60 mg of pure product was obtained with a yield of 16.0%.
**¹HNMR (DMSO-*d*₆, 600 MHz) δ (ppm)** 12.61(1H, s), 10.85(1H, s), 10.59(1H, s), 7.64(1H, d, *J* = 2.4 Hz), 7.35(1H, dd, *J* = 8.4, 2.4 Hz), 7.07(1H, d, *J* = 8.4 Hz), 4.20(1H, d, *J* = 7.8 Hz), 3.06-3.16(2H, m), 3.03(1H, q), 2.55(1H, d, *J* = 16.2 Hz), 1.63-1.70(2H, m), 0.97(3H, t, *J* = 7.2 Hz);
**¹³CNMR (DMSO-*d*₆, 150 MHz) δ (ppm)** 170.71, 151.45, 136.92, 134.13, 134.04, 123.04, 120.03, 38.16, 32.35, 32.02, 22.81, 13.62;
MS (ESI+) m/z 377.1 [M+H] +.

### Example 47:

### Preparation of 2-propylmercapto-5-phenyl-5,8-dihydropyrido[2,3-d]pyrimidin-4,7(3H,6H)-dione (LSL-10-05)

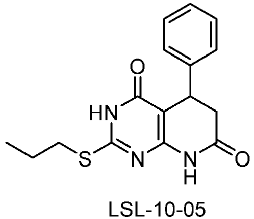

2-propylmercapto-6-aminopyrimidin-4(*3H*)-one (185 mg, 1.0mmol), 2,2-dimethyl-1,3-dioxane-4,6-dione (173 mg, 1.2 mmol) and benzaldehyde (106 mg, 1.0 mmol) were dissolved in about 5 mL of ethylene glycol, and heated to react under an oil bath at 170°C for about 2 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (dichloromethane/methanol: 2.5%), 80 mg of pure product was obtained with a yield of 25.4%.
**¹HNMR (DMSO-*d*₆, 600 MHz) δ (ppm)** 12.58(1H, s), 10.53(1H, s), 7.27-7.33(2H, m), 7.18-7.21(1H, m), 7.10-7.16(2H, m), 4.20(1H, d, *J* = 6.0 Hz), 3.10-3.16(2H, m), 3.05(1H, q), 2.53(1H, d, *J* = 16.2 Hz), 1.65-1.71(2H, m), 0.97(3H, t, *J* = 6.0 Hz);
**¹³CNMR (DMSO-*d*₆, 150 MHz) δ (ppm)** 170.94, 143.04, 129.09 × 2, 127.17, 126.93 × 2, 38.58, 33.49, 31.99, 22.84, 13.83;
MS (ESI+) m/z 316.1 [M+H] +.

### Example 48:

### Preparation of 2-propylmercapto-5-(quinolin-4-yl)-5,8-dihydropyrido[2,3-d]pyrimidin-4,7(3H,6H)-dione (LSL-10-06)

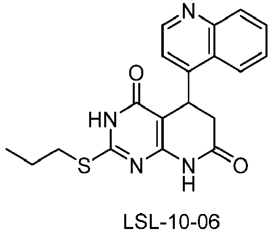

2-propylmercapto-6-aminopyrimidin-4(*3H*)-one (185 mg, 1.0 mmol), 2,2-dimethyl-1,3-dioxane-4,6-dione (173 mg, 1.2 mmol) and 4-quinolinecarboxaldehyde (157 mg, 1.0 mmol) were dissolved in about 5 mL of ethylene glycol, and heated to react under an oil bath at 170°Cfor about 2 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product. After separation and purification by a flash column (dichloromethane/methanol: 3.5%), 80 mg of pure product was obtained with a yield of 21.9%.
¹HNMR (DMSO-*d*₆, 600 MHz) δ (ppm) 12.64(1H, s), 10.68(1H, s), 8.75(1H, d, *J* = 4.8 Hz), 8.34(1H, d, *J* = 8.4 Hz), 8.07(1H, d, *J* = 8.4 Hz), 7.81(1H, td, *J* = 7.2, 0.6 Hz), 7.71(1H, td, *J* = 7.2, 0.6 Hz), 6.96(1H, d, *J* = 4.8 Hz), 5.06(1H, d, *J* = 9.0 Hz), 3.30(1H, q), 3.12-3.22(2H, m), 2.46(1H, d, *J* = 16.8 Hz), 1.69-1.76(2H, m), 1.01(3H, t, *J* = 7.2 Hz);
¹³CNMR (DMSO-*d*₆, 150 MHz) δ (ppm) 170.07, 150.97, 148.75, 147.64, 130.52, 129.83, 127.42, 126.13, 124.03, 118.21, 38.20, 32.09, 29.68, 22.85, 13.67;
MS (ESI+) m/z 367.1 [M+H] +.

### Example 49:

### Preparation of 2-propylmercapto-5-(quinolin-5-yl)-5,8-dihydropyrido[2,3-d]pyrimidin-4,7(3H,6H)-dione (LSL-10-07)

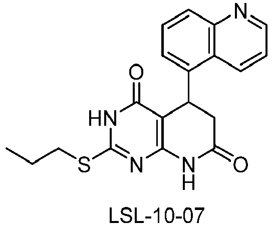

2-propylmercapto-6-aminopyrimidin-4(*3H*)-one (185 mg, 1.0 mmol), 2,2-dimethyl-1,3-dioxane-4,6-dione (173 mg, 1.2 mmol) and 5-quinolinecarboxaldehyde (157 mg, 1.0 mmol) were dissolved in about 5 mL of ethylene glycol, and heated to react under an oil bath at 170°C for about 0.5 hours, and TLC detected (petroleum ether/ethyl acetate = 1/1) that the raw material points disappeared. The reaction solution was poured into an appropriate amount of ice water, and a flocculent precipitate was produced, which was filtered to obtain a crude product was obtained. After separation and purification by a flash column (dichloromethane/methanol: 4.0%), 154 mg of pure product was obtained with a yield of 42.1%.
**¹HNMR (DMSO-*d*₆, 600 MHz) δ (ppm)** 12.60(1H, s), 10.64(1H, s), 8.95(1H, dd, *J* = 4.2, 1.8 Hz), 8.75(1H, d, *J* = 8.4 Hz), 7.91(1H, d, *J* = 8.4 Hz), 7.63(1H, d, *J* = 7.2 Hz), 7.62(1H, dd, *J* = 8.4, 3.6 Hz), 7.06(1H, d, *J* = 7.2 Hz), 5.05(1H, d, *J* = 8.4 Hz), 3.24(1H, q), 3.12-3.21(2H, m), 2.42(1H, d, *J* = 16.8 Hz), 1.69-1.75(2H, m), 1.01(3H, t, *J* = 7.2 Hz);
**¹³CNMR (DMSO-*d*₆, 150 MHz) δ (ppm)** 170.36, 150.71, 149.10, 138.91, 132.33, 129.56, 128.91, 125.94, 123.41, 121.97, 38.97, 32.07, 29.50, 22.86, 13.68;
MS (ESI+) m/z 367.1 [M+H] +.

### Example 50:

### Preparation of 2-propylmercapto-5-(4-methoxyphenyl)-pyrido[2,3-d]pyrimidin-4(3H)-one (LSL-11-02)

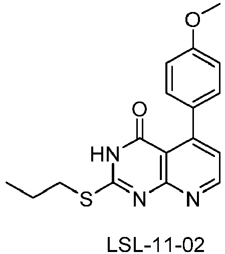

2-propylmercapto-6-aminopyrimidin-4(*3H*)-one (185 mg, 1.0mmol) and 3-dimethylamino-1-(4-methoxyphenyl)-2-propylene-1-one (205 mg, 1.0 mmol) were dissolved in about 10 mL of glacial acetic acid, and heated to reflux under an oil bath for about 2 hours, and TLC detected (petroleum ether/ethyl acetate = 1/1) that the raw material points disappeared. The reaction solution was poured into an appropriate amount of ice water to form a suspension, and extracted three times with ethyl acetate. After the organic phases were combined, they were washed with clear water and saturated saline respectively, dried over anhydrous sodium sulfate, filtered, concentrated, and separation and purification by a flash column (petroleum ether/ethyl acetate: 25%), 120 mg of pure product was obtained with a yield of 37%.
**¹HNMR (DMSO-*d*₆, 600 MHz) δ (ppm)** 12.75(1H, s), 8.39(1H, d, *J* = 7.8 Hz), 8.18(2H, d, *J* = 8.4 Hz), 7.95(1H, d, *J* = 8.4 Hz), 7.10(2H, d, *J* = 9.0 Hz), 3.85(3H, s), 3.28(2H, t, *J* = 6.6 Hz), 1.72-1.79(2H, m), 1.03(3H, t, *J* = 7.2 Hz);
**¹³CNMR (DMSO-*d*₆, 150 MHz) δ (ppm)** 162.04, 161.73, 161.71, 160.64, 158.55, 136.94, 130.49, 129.55 × 2, 117.73, 114.79 × 2, 113.61, 55.84, 32.14, 22.42, 13.67;
**MS (ESI+) m/z** 328.1 [M+H] +.

### Example 51:

### Preparation of 2-propylmercapto-5-(quinolin-6-yl)-pyrido[2,3-d]pyrimidin-4(3H)-one (LSL-11-08)

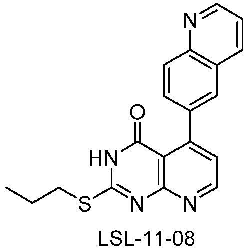

2-propylmercapto-6-aminopyrimidin-4(*3H*)-one (185 mg, 1.0 mmol) and 3-dimethylamino-1-(quinolin-6-yl)-2-propylene-1-one (171 mg, 1.0 mmol) were dissolved in about 10 mL of glacial acetic acid, and heated to reflux under an oil bath for about 2 hours, and TLC detected (petroleum ether/ethyl acetate = 1/1) that the raw material points disappeared. The reaction solution was concentrated and poured into an appropriate amount of ice water, and a yellow solid was produced. The crude product was obtained by filtration, and was separation and purification by a flash column (dichloromethane/methanol: 3.0%) to obtain130 mg of pure product with a yield of 37.4%.
**¹HNMR (DMSO-*d*₆, 600 MHz) δ (ppm)** 12.85(1H, s), 8.99(1H, dd, *J* = 4.2, 1.8 Hz), 8.87(1H, d, *J* = 1.8 Hz), 8.57-8.61(2H, m), 8.53(1H, d, *J* = 8.4 Hz), 8.20(1H, d, *J =* 8.4 Hz), 8.18(1H, d, *J* = 9.0 Hz), 7.63(1H, q), 3.31(2H, t, *J* = 6.6 Hz), 1.75-1.81(2H, m), 1.05(3H, t, *J* = 7.2 Hz);
**¹³CNMR (DMSO-*d*₆, 150 MHz) δ (ppm)** 152.14, 148.80, 137.71, 137.48, 135.98, 129.93, 128.72, 128.36, 128.21, 122.06, 118.91, 32.20, 22.40, 13.68;
MS (ESI+) m/z 349.1 [M+H] +.

### Example 52:

### Preparation of 2-propylmercapto-5-(4-methoxyphenyl)-pyrido[2,3-d]pyrimidin-4,7(3H,6H)-dione (LSL-11-09)

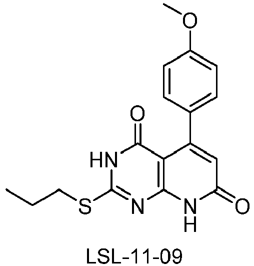

2-propylmercapto-6-aminopyrimidin-4(*3H*)-one (185 mg, 1.0 mmol) and acetic anhydride (204 mg, 2.0 mmol) were dissolved in about 5 mL of toluene. After stirring for about 5 minutes at room temperature, *p*-toluenesulfonic acid monohydrate (190 mg, 1.0 mmol), ethyl p-methoxycinnamate (1.03 g, 5.0 mmol), sodium persulfate (714 mg, 3.0 mmol) and palladium acetate (22.45 mg, 0.1 mmol) were added into the above reaction solution in one step, and heated to reflux under an oil bath for 36 hours, and TLC detected (petroleum ether/ethyl acetate = 1/1) that the raw material points disappeared. The reaction solution was filtered and added into an appropriate amount of ice water, and extracted three times with ethyl acetate. After the organic phases were combined, they were washed with clear water and saturated saline respectively, dried over anhydrous sodium sulfate, filtered, concentrated, and separation and purification by a flash column (petroleum ether/ethyl acetate: 25%), 50 mg of pure product was obtained with a yield of 14.6%.
**¹HNMR (DMSO-*d*₆, 600 MHz) δ (ppm)** 12.70(1H, s), 10.39(1H, s), 7.06(2H, d, *J* = 9.0 Hz), 6.83(2H, d, *J* = 9.0 Hz), 4.13(1H, s), 3.69(3H, s), 3.03-3.12(2H, m), 1.62-1.69(2H, m), 0.97(3H, t, *J* = 7.2 Hz);
**¹³CNMR (DMSO-*d*₆, 150 MHz) δ (ppm)** 171.03, 158.46, 133.5, 127.96 × 2, 115.1, 114.41 × 2, 100.00, 55.51, 31.96, 22.90, 13.65;
**MS (ESI+) m/z** 344.1 [M+H] +.

### General Synthesis Method in Examples 53 to 91:

2-propylmercapto-6-aminopyrimidin-4(*3H*)-one (185 mg, 1.0 mmol) or 2-methylmercapto-6-aminopyrimidin-4(*3H*)-one (157 mg, 1.0 mmol), 2,2-dimethyl-1,3-dioxane-4,6-dione (173 mg, 1.2 mmol) and various aldehyde group-containing reagents (1.0 mmol) were dissolved in about 5 mL of ethylene glycol, and heated to react under an oil bath at 150°C to 210°Cfor about 1 to 3 hours, and TLC detected that the raw material points disappeared. The reaction solution was poured into an appropriate amount of ice water, and a flocculent precipitate was produced, which was filtered to obtain a crude product. After separation and purification by a flash column (dichloromethane/methanol), the pure target compound was obtained.

### Example 53:

### Preparaiton of 2-propylmercapto-5-(3-amino-4-methoxyphenyl)-5,8-dihydropyrido[2,3-d]pyrimidin-4,7(3H,6H)-dione (LSL-9-42)

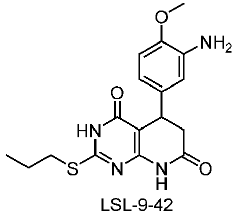

2-propylmercapto-6-aminopyrimidin-4(*3H*)-one (185 mg, 1.0 mmol) and 3-amino-4-methoxybenzaldehyde (151 mg, 1.0 mmol) reacted at 160°C for about 1 hour. After separation and purification by a flash column (dichloromethane/methanol: 2.0% to 3.0%), 70 mg of pure product was obtained with a yield of 19.4%.
**¹HNMR (CD₃OD, 600 MHz) δ (ppm)** 6.74(1H, d, *J* = 8.4 Hz), 6.64(1H, d, *J* = 1.8 Hz), 6.55(1H, dd, *J* = 8.4, 1.8 Hz), 4.20(1H, d, *J* = 7.2 Hz), 3.79(3H, s), 3.16-3.25(2H, m), 3.00(1H, q), 2.66(1H, dd, *J* = 16.8, 0.6 Hz), 1.73-1.80(2H, m), 1.05(3H, t, *J* = 7.2 Hz);
**¹³CNMR (CD₃OD, 150 MHz) δ (ppm)** 172.15, 147.04, 135.99, 134.48, 116.50, 113.81, 110.44, 54.72, 38.13, 32.95, 31.91, 22.31, 12.06;
**MS (ESI+) m/z** 361.1 [M+H] +.

### Example 54:

### Preparation of 2-propylmercapto-5-(3-amino-4-chlorophenyl)-5,8-dihydropyrido[2,3-d]pyrimidin-4,7(3H,6H)-dione (LSL-9-43)

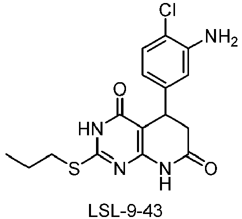

2-propylmercapto-6-aminopyrimidin-4(*3H*)-one (185 mg, 1.0 mmol) and 2-amino-4-chrolo-benzaldehyde (155 mg, 1.0 mmol) reacted at 160 °C for about 1 hour. After separation and purification by a flash column (dichloromethane/methanol: 2.0% to 3.0%), 94 mg of pure product was obtained with a yield of 25.8%.
**¹HNMR (DMSO-*d*₆, 600 MHz) δ (ppm)** 12.53(1H, s), 10.47(1H, s), 7.07(1H, d, *J* = 8.4 Hz), 6.54(1H, d, *J* = 1.8 Hz), 6.34(1H, dd, *J* = 8.4, 1.8 Hz), 5.27(2H, s), 4.05(1H, d, *J* = 7.2 Hz), 3.05-3.16(2H, m), 2.99(1H, q), 2.44(1H, d, *J* = 16.2Hz), 1.64-1.70(2H, m), 0.98(3H, t, *J* = 7.2 Hz);
**¹³CNMR (DMSO-*d*₆, 150 MHz) δ (ppm)** 170.76, 145.00, 142.67, 129.55, 115.83, 115.61, 113.74, 49.07, 38.75, 33.30, 32.00, 22.83, 13.64;
**MS (ESI+) m/z** 365.1 [M+H] +.

### Example 55:

### Preparation of 2-propylmercapto-5-(4-aminophenyl)-5,8-dihydropyrido[2,3-d]pyrimidin-4,7(3H,6H)-dione (LSL-9-44)

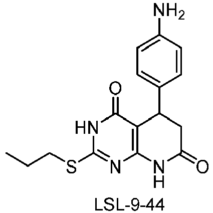

2-propylmercapto-6-aminopyrimidin-4(3*H*)-one (185 mg, 1.0 mmol) and 4-aminobenzaldehyde (121 mg, 1.0 mmol) reacted at 150°C for about 1 hour. After separation and purification by a flash column (dichloromethane/methanol: 2.0% to 3.0%), 67 mg of pure product was obtained with a yield of 20.3%.
**¹HNMR (DMSO-*d*₆, 600 MHz) δ (ppm)** 12.46(1H, s), 10.40(1H, s), 6.78(2H, d, *J* = 7.8 Hz), 6.45(2H, d, *J* = 7.8 Hz), 4.90(2H, s), 4.02(1H, d, *J* = 7.2 Hz), 3.05-3.18(2H, m), 2.92(1H, q), 2.45(1H, d, *J* = 16.2Hz), 1.63-1.70(2H, m), 0.97(3H, t, *J* = 7.2 Hz);
**¹³CNMR (DMSO-*d*₆, 150 MHz) δ (ppm)** 171.21, 147.79, 129.81, 127.36 × 2, 114.41 × 2, 38.94, 32.62, 31.99, 22.84, 13.62;
**MS (ESI+) m/z** 331.1 [M+H] +.

### Example 56:

### Preparation of 2-propylmercapto-5-(3-aminophenyl)-5,8-dihydropyrido[2,3-d]pyrimidin-4,7(3H,6H)-dione (LSL-9-45)

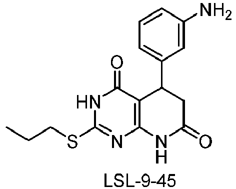

2-propylmercapto-6-aminopyrimidin-4(*3H*)-one (185 mg, 1.0 mmol) and 3-aminobenzaldehyde (121 mg, 1.0 mmol) reacted at 150°C for about 1 hour. After separation and purification by a flash column (dichloromethane/methanol: 2.0% to 3.0%), 70 mg of pure product was obtained with a yield of 21.2%.
**¹HNMR (DMSO-*d*₆, 600 MHz) δ (ppm)** 12.46(1H, s), 10.41(1H, s), 6.89(1H, t, *J* = 7.8 Hz), 6.36-6.39(1H, m), 6.28-6.32(2H, m), 4.97(2H, s), 4.02(1H, d, *J* = 7.8 Hz), 3.05-3.16(2H, m), 2.97(1H, q), 2.44(1H, d, *J* = 16.2Hz), 1.64-1.71(2H, m), 0.98(3H, t, *J* = 7.2 Hz);
**¹³CNMR (DMSO-*d*₆, 150 MHz) δ (ppm)** 170.97, 149.26, 143.65, 129.50, 114.42, 112.77, 112.30, 38.91, 33.67, 31.99, 22.85, 13.64;
**MS (ESI+) m/z** 331.1 [M+H] +.

### Example 57:

### Preparation of 2-propylmercapto-5-(2-amino-3-methoxyphenyl)-5,8-dihydropyrido[2,3-d]pyrimidin-4,7(3H,6H)-dione (LSL-9-46)

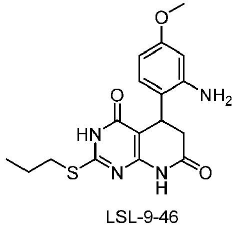

2-propylmercapto-6-aminopyrimidin-4(*3H*)-one (185 mg, 1.0 mmol) and 2-amino-4-methoxybenzaldehyde (151 mg, 1.0 mmol) reacted at 160°C for about 1.5 hours. After separation and purification by a flash column (dichloromethane/methanol: 2.0% to 3.0%), 68 mg of pure product was obtained with a yield of 18.9%.
**¹HNMR (DMSO-*d*₆, 600 MHz) δ (ppm)** 11.71(1H, s), 9.94(1H, s), 6.65(1H, d, *J* = 8.4 Hz), 6.46(1H, d, *J* = 2.4 Hz), 6.42(1H, dd, *J* = 8.4, 2.4 Hz), 6.22(2H, s), 4.27(1H, d, *J* = 7.8 Hz), 3.68(3H, s), 3.06-3.09(2H, m), 2.97(1H, q), 2.14(1H, dd, *J* = 16.2, 6.0 Hz), 1.62-1.69(2H, m), 0.97(3H, t, *J* = 7.2 Hz);
**¹³CNMR (DMSO-*d*₆, 150 MHz) δ (ppm)** 171.20, 158.84, 139.64, 123.29, 114.94, 107.35, 91.86, 55.53, 38.95, 34.18, 31.67, 22.83, 13.66;
**MS (ESI+) m/z** 361.1 [M+H] +.

### Example 58:

### Preparation of 2-propylmercapto-5-(2-cyanophenyl)-5,8-dihydropyrido[2,3-d]pyrimidin-4,7(3H,6H)-dione (LSL-9-50)

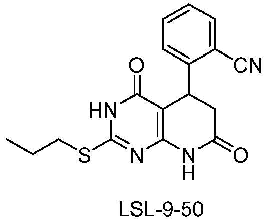

2-propylmercapto-6-aminopyrimidin-4(*3H*)-one (185 mg, 1.0 mmol) and 2-cyanobenzaldehyde (131 mg, 1.0 mmol) reacted at 170°C for about 2 hours. After separation and purification by a flash column (dichloromethane/methanol: 2.0% to 3.0%), 76 mg of pure product was obtained with a yield of 22.3%.
**¹HNMR (DMSO-*d*₆, 600 MHz) δ (ppm)** 12.58(1H, s), 10.45(1H, s), 7.21(1H, td, *J* = 7.8, 1.8 Hz), 7.00(1H, d, *J* = 8.4 Hz), 6.81(1H, t, *J* = 7.8 Hz), 6.72(1H, d, *J* = 7.2 Hz), 4.41(1H, d, *J* = 8.4 Hz), 3.08-3.18(2H, m), 2.95(1H, q), 2.39, d, *J* = 16.8 Hz), 1.65-1.72(2H, m), 0.99(3H, t, *J* = 7.8 Hz);
**¹³CNMR (DMSO-*d*₆, 150 MHz) δ (ppm)** 170.86, 157.05, 130.78, 129.65, 128.55, 126.87, 120.66, 111.59, 37.54, 32.01, 28.62, 22.86, 13.65;
**MS (ESI+) m/z** 341.1 [M+H] +

### Example 59:

### Preparation of 2-propylmercapto-5-(4-cyanophenyl)-5,8-dihydropyrido[2,3-d]pyrimidin-4,7(3H,6H)-dione (LSL-10-01)

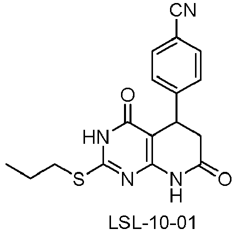

2-propylmercapto-6-aminopyrimidin-4(*3H*)-one (185 mg, 1.0 mmol) and 4-cyanobenzaldehyde (131 mg, 1.0 mmol) reacted at 170°C for about 1.5 hours. After separation and purification by a flash column (dichloromethane/methanol: 2.0% to 3.0%), 90 mg of pure product was obtained with a yield of 26.5%.
**¹HNMR (DMSO-*d*₆, 600 MHz) δ (ppm)** 12.60(1H, s), 10.26(1H, s), 7.73(2H, d, *J* = 8.4 Hz), 7.37(2H, d, *J* = 8.4 Hz), 4.27(1H, d, *J* = 7.2 Hz), 2.99-3.07(3H, m), 2.53(1H, d, *J* = 16.2Hz), 1.60-1.67(2H, m), 0.96(3H, t, *J* = 7.2 Hz);
**¹³CNMR (DMSO-*d*₆, 150 MHz) δ (ppm)** 170.61, 155.30, 149.70, 132.91 × 2, 128.19 × 2, 119.31, 109.77, 97.33, 38.17, 34.02, 31.94, 23.01, 13.72;
**MS (ESI+) m/z** 341.1 [M+H] +.

Example 60:

### Preparation of 2-methylmercapto-5-(4-methoxyphenyl)-5,8-dihydropyrido[2,3-d]pyrimidin-4,7(3H,6H)-dione (LSL-10-08)

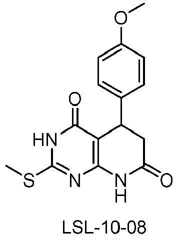

2-methylmercapto-6-aminopyrimidin-4(*3H*)-one (157 mg, 1.0 mmol) and 4-methoxybenzaldehyde (136 mg, 1.0 mmol) reacted at 170°C for about 1 hour. After separation and purification by a flash column (dichloromethane/methanol: 1.5% to 2.5%), 103 mg of pure product was obtained with a yield of 32.5%.
**¹HNMR (DMSO-*d*₆, 600 MHz) δ (ppm)** 12.56(1H, s), 10.47(1H, s), 7.06(2H, d, *J* = 8.4 Hz), 6.83(2H, d, *J* = 8.4 Hz), 4.15(1H, d, *J* = 7.2 Hz), 3.70(3H, s), 2.98(1H, q), 2.49(1H, d, *J* = 16.2 Hz), 2.49(3H, s);
**¹³CNMR (DMSO-*d*₆, 150 MHz) δ (ppm)** 170.96, 158.54, 134.84, 127.94 × 2, 114.48 × 2, 55.53, 38.75, 32.67, 13.18;
**MS (ESI+) m/z** 318.1 [M+H] +.

### Example 61:

### Preparation of 2-butylmercapto-5-(4-methoxyphenyl)-5,8-dihydropyrido[2,3-d]pyrimidin-4,7(3H,6H)-dione (LSL-10-19)

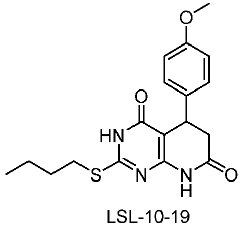

2-butylmercapto-6-aminopyrimidin-4(*3H*)-one (199 mg, 1.0 mmol) and 4-methoxybenzaldehyde (136 mg, 1.0 mmol) reacted at 170°C for about 1 hour. After separation and purification by a flash column (dichloromethane/methanol: 1.5% to 2.5%), 95 mg of pure product was obtained with a yield of 26.5%.
**¹HNMR (DMSO-*d*₆, 600 MHz) δ (ppm)** 12.54(1H, s), 10.24(1H, s), 7.07(2H, d, *J* = 8.0 Hz), 6.82(2H, d, *J* = 8.0 Hz), 4.12(1H, d, *J* = 7.2 Hz), 3.70(3H, s), 3.02-3.12(2H, m), 2.93(1H, q), 2.48(1H, d, *J* = 16.0 Hz), 1.53-1.68(2H, m), 1.35-1.45(2H, m), 0.91(3H, t, *J* = 7.2 Hz);
**¹³CNMR (DMSO-*d*₆, 150 MHz) δ (ppm)** 171.09, 158.34, 154.91, 149.75, 135.51, 130.12, 128.00 × 2, 114.28 × 2, 98.85, 55.48, 38.95, 32.84, 31.64, 29.71, 21.89, 14.02;
**MS (ESI+) m/z** 360.1 [M+H] +.

### Example 62:

### Preparation of 2-butylmercapto-5-(3-nitro-4-hydroxyphenyl)-5,8-dihydropyrido[2,3-d]pyrimidin-4,7(3H,6H)-dione (LSL-10-20)

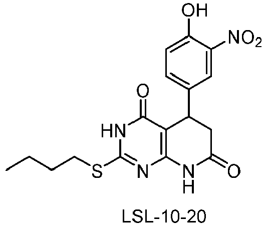

2-butylmercapto-6-aminopyrimidin-4(*3H*)-one (199 mg, 1.0 mmol) and 3-nitro-4-hydroxybenzaldehyde (167 mg, 1.0 mmol) reacted at 170°C for about 2 hours. After separation and purification by a flash column (dichloromethane/methanol: 1.5% to 2.5%), 65 mg of pure product was obtained with a yield of 16.7%.
**¹HNMR (DMSO-*d*₆, 600 MHz) δ (ppm)** 12.58(1H, s), 10.83(1H, s), 10.57(1H, s), 7.64(1H, d, *J* = 2.4 Hz), 7.35(1H, dd, *J* = 8.4, 2.4 Hz), 7.08(1H, d, *J* = 8.4 Hz), 4.20(1H, d, *J* = 7.8 Hz), 3.09-3.19(2H, m), 3.03(1H, q), 2.54(1H, d, *J* = 16.2 Hz), 1.60-1.66(2H, m), 1.36-1.43(2H, m), 0.91(3H, t, *J* = 7.2 Hz);
**¹³CNMR (DMSO-*d*₆, 150 MHz) δ (ppm)** 170.71, 151.46, 136.92, 134.16, 134.06, 123.02, 120.04, 38.15, 32.37, 31.38, 29.90, 21.80, 13.93;
**MS (ESI+) m/z** 391.1 [M+H] +.

### Example 63:

### Preparation of 2-butylmercapto-5-(4-propoxyphenyl)-5,8-dihydropyrido[2,3-d]pyrimidin-4,7(3H,6H)-dione (LSL-10-23)

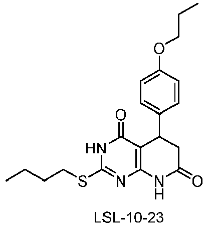

2-butylmercapto-6-aminopyrimidin-4(*3H*)-one (199 mg, 1.0 mmol) and 4-propoxybenzaldehyde (164 mg, 1.0 mmol) reacted at 170°C for about 1.5 hours. After separation and purification by a flash column (dichloromethane/methanol: 1.5% to 2.5%), 92 mg of pure product was obtained with a yield of 23.8%.
**¹HNMR (DMSO-*d*₆, 600 MHz) δ (ppm)** 12.35(1H, s), 10.09(1H, s), 7.06(2H, d, *J* = 8.4 Hz), 6.80(2H, d, *J* = 8.4 Hz), 4.12(1H, d, *J* =7.8 Hz), 3.86(2H, t, *J* =6.6 Hz), 3.00-3.10(2H, m), 2.90(1H, q), 2.47(1H, d, *J* = 16.2 Hz), 1.66-1.72(2H, m), 1.57-1.63(2H, m), 1.36-1.43(2H, m), 0.95(3H, t, *J* =7.2 Hz), 0.90(3H, t, *J* =7.2 Hz);
**¹³CNMR (DMSO-*d*₆, 150 MHz) δ (ppm)** 171.09, 157.24, 128.02 × 2, 114.79 × 2, 69.38, 39.04, 32.95, 31.72, 29.71, 22.50, 21.90, 14.01, 10.84;
**MS (ESI+) m/z** 388.2 [M+H] +.

### Example 64:

### Preparation of 2-butylmercapto-5-(3-hydroxy-4-methoxyphenyl)-5,8-dihydropyrido[2,3-d]pyrimidin-4,7(3H,6H)-dione (LSL-10-24)

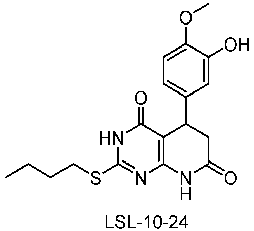

2-butylmercapto-6-aminopyrimidin-4(*3H*)-one (199 mg, 1.0 mmol) and 3-hydroxy-4-methoxybenzaldehyde (152 mg, 1.0 mmol) reacted at 170°C for about 1.5 hours. After separation and purification by a flash column (dichloromethane/methanol: 1.5% to 2.5%), 81 mg of pure product was obtained with a yield of 21.6%.
**¹HNMR (DMSO-*d*₆, 600 MHz) δ (ppm)** 12.45(1H, s), 10.33(1H, s), 8.04(1H, s), 6.79(1H, d, *J* = 8.4 Hz), 6.58(1H, d, *J* = 2.4 Hz), 6.53(1H, dd, *J* = 8.4, 2.4 Hz), 4.05(1H, d, *J* = 7.8 Hz), 3.70(3H, s), 3.05-3.15(2H, m), 2.93(1H, q), 2.46(1H, d, *J* = 16.2 Hz), 1.59-1.64(2H, m), 1.37-1.43 (2H, m), 0.90(3H, t, *J* = 7.2 Hz);
**¹³CNMR (DMSO-*d*₆, 150 MHz) δ (ppm)** 171.05, 146.95, 146.86, 135.94, 117.47, 114.45, 113.07, 56.23, 38.87, 32.91, 31.52, 29.80, 21.84, 13.97;
**MS (ESI+) m/z** 376.1 [M+H] +.

### Example 65:

### Preparation of 2-butylmercapto-5-(quinolin-6-yl)-5,8-dihydropyrido[2,3-d]pyrimidin-4,7(3H,6H)-dione (LSL-10-25)

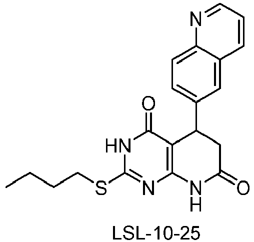

2-butylmercapto-6-aminopyrimidin-4(*3H*)-one (199 mg, 1.0 mmol) and 6-quinolinebenzaldehyde (157 mg, 1.0 mmol) reacted at 170°C for about 2 hours. After separation and purification by a flash column (dichloromethane/methanol: 1.5% to 2.5%), 97 mg of pure product was obtained with a yield of 25.5%.
**¹HNMR (DMSO-*d*₆, 600 MHz) δ (ppm)** 12.55(1H, s), 10.57(1H, s), 8.84(1H, d, *J* = 2.4 Hz), 8.30(1H, d, *J* = 8.4 Hz), 7.97(1H, d, *J* = 9.0 Hz), 7.65(1H, d, *J* = 9.0 Hz), 7.62(1H, s), 7.47-7.50(1H, m), 4.41(1H, d, *J* = 7.8 Hz), 3.09-3.21(3H, m), 2.66(1H, d, *J* = 16.8 Hz), 1.62-1.67(2H, m), 1.37-1.44(2H, m), 0.92(3H, t, *J* = 7.2 Hz);
**¹³CNMR (DMSO-*d*₆, 150 MHz) δ (ppm)** 170.79, 150.70, 147.40, 141.20, 136.31, 129.97, 129.63, 128.21, 124.90, 122.08, 38.46, 33.64, 31.40, 29.91, 21.84, 13.95;
**MS (ESI+) m/z** 381.1 [M+H] +.

### Example 66:

### Preparation of 2-isopropylmercapto-5-(4-methoxyphenyl)-5,8-dihydropyrido[2,3-d]pyrimidin-4,7(3H,6H)-dione (LSL-10-38)

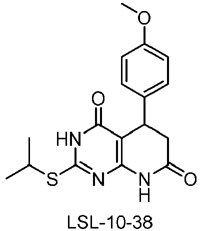

2-isopropylmercapto-6-aminopyrimidin-4(*3H*)-one (185 mg, 1.0 mmol) and 4-methoxybenzaldehyde (136 mg, 1.0 mmol) reacted at 170°C for about 2 hours. After separation and purification by a flash column (dichloromethane/methanol: 1.5% to 2.5%), 115 mg of pure product was obtained with a yield of 33.3%.
**¹HNMR (DMSO-*d*₆, 400 MHz) δ (ppm)** 12.53(1H, s), 10.51(1H, s), 7.07(2H, d, *J* = 8.4 Hz), 6.85(2H, d, *J* = 8.4 Hz), 4.15(1H, d, *J* = 7.2 Hz), 3.89-3.96(1H, m), 3.70(3H, s), 3.01(1H, q), 2.49(1H, d, *J* = 16.4 Hz), 1.38(3H, s), 1.36(3H, s);
**¹³CNMR (DMSO-*d*₆, 100 MHz) δ (ppm)** 171.00, 158.50, 134.83, 127.93 × 2, 114.46 × 2, 55.50, 38.70, 36.25, 32.63, 23.36, 23.11;
**MS (ESI+) m/z** 346.1 [M+H] +.

### Example 67:

### Preparation of 2-propylmercapto-5-(4-morpholinophenyl)-5,8-dihydropyrido[2,3-d]pyrimidin-4,7(3H,6H)-dione (LSL-10-43)

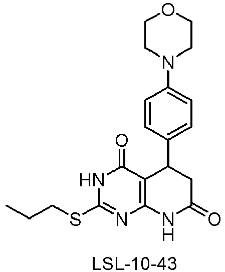

2-propylmercapto-6-aminopyrimidin-4(*3H*)-one (185 mg, 1.0 mmol) and 4-morpholinobenzaldehyde (174 mg, 1.0 mmol) reacted at 170 °C for about 2 hours. After separation and purification by a flash column (dichloromethane/methanol: 1.5% to 2.5%), 102 mg of pure product was obtained with a yield of 25.5%.
**¹HNMR (DMSO-*d*₆, 400 MHz) δ (ppm)** 12.29(1H, s), 9.72(1H, s), 7.03(2H, d, *J* = 8.8 Hz), 6.80(2H, d, *J* = 8.8 Hz), 4.05(1H, d, *J* = 7.2 Hz), 3.69-3.73(4H, m), 2.99-3.73(4H, m), 2.92-2.97(2H, m), 2.80(1H, q), 2.43(1H, d, *J* = 15.6 Hz), 1.55-1.65(2H, m), 0.95(3H, t, *J* = 7.2 Hz);
**¹³CNMR (DMSO-*d*₆, 100 MHz) δ (ppm)** 171.24, 154.69, 149.99, 127.67 × 2, 115.62 × 2, 98.32, 66.56 × 2, 49.33 × 2, 33.15, 31.76, 23.27, 13.88;
**MS (ESI+) m/z** 401.1 [M+H] +.

### Example 68:

### Preparation of 2-propylmercapto-5-(4-(4-methylpiperazine)phenyl)-5,8-dihydropyrido[2,3-d]pyrimidin-4,7(3H,6H)-dione (LSL-10-47)

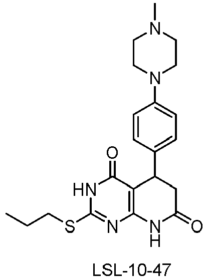

2-propylmercapto-6-aminopyrimidin-4(*3H*)-one (185 mg, 1.0 mmol) and 4-(4-methylpiperazine)benzaldehyde (204 mg, 1.0 mmol) reacted at 170°C for about 2 hours. After separation and purification by a flash column (dichloromethane/methanol: 1.5% to 2.5%), 96 mg of pure product was obtained with a yield of 23.2%.
**¹HNMR (DMSO-*d*₆, 400 MHz) δ (ppm)** 12.46(1H, s), 10.49(1H, s), 6.98(2H, d, *J* = 8.8 Hz), 6.85(2H, d, *J* = 8.8 Hz), 4.10(1H, d, *J* = 7.2 Hz), 3.04-3.16(2H, m), 3.04-3.08(4H, m), 2.98(1H, q), 2.46(1H, d, *J* = 16.0 Hz), 2.42-2.45(4H, m), 2.21(3H, s), 1.62-1.71(2H, m), 0.97(3H, t, *J* = 7.2 Hz);
**¹³CNMR (DMSO-*d*₆, 100 MHz) δ (ppm)** 171.13, 162.31, 161.56, 154.94, 150.27, 133.11, 127.41 × 2, 116.02 × 2, 99.36, 54.99 × 2, 48.60 × 2, 46.16, 38.67, 32.57, 31.94, 22.84, 13.63;
**MS (ESI+) m/z** 414.2 [M+H] +.

### Example 69:

### Preparation of 2-propylmercapto-5-(4-(piperidin-1-yl)phenyl)-5,8-dihydropyrido[2,3-d]pyrimidin-4,7(3H,6H)-dione (LSL-10-48)

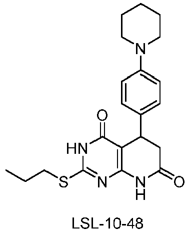

2-propylmercapto-6-aminopyrimidin-4(3*H*)-one (185 mg, 1.0 mmol) and 4-piperidin-1-ylbenzaldehyde (189 mg, 1.0 mmol) reacted at 170°C for about 2 hours. After separation and purification by a flash column (dichloromethane/methanol: 1.5% to 2.5%), 88 mg of pure product was obtained with a yield of 22.1%.
**¹HNMR (DMSO-*d*₆, 400 MHz) δ (ppm)** 12.50(1H, s), 10.49(1H, s), 6.97(2H, d, *J* = 8.8 Hz), 6.82(2H, d, *J* = 8.8 Hz), 4.09(1H, d, *J* = 7.6 Hz), 3.05-3.17(2H, m), 3.04-3.09(4H, m), 2.97(1H, q), 2.49(1H, d, *J* = 16.0 Hz), 1.61-1.71(2H, m), 1.55-1.64(4H, m), 1.48-1.52(2H, m), 0.97(3H, t, *J* = 7.2 Hz);
**¹³CNMR (DMSO-*d*₆, 100 MHz) δ (ppm)** 171.15, 150.97, 132.69, 127.38 × 2, 116.51 × 2, 50.16, 38.68, 32.55, 31.94, 25.68 × 2, 24.35 × 2, 22.84, 13.63;
**MS (ESI+) m/z** 399.2 [M+H] +.

### Example 70:

### Preparation of 2-propylmercapto-5-(4-thiomorpholinophenyl)-5,8-dihydropyrido[2,3-d]pyrimidin-4,7(3H,6H)-dione (LSL-10-50)

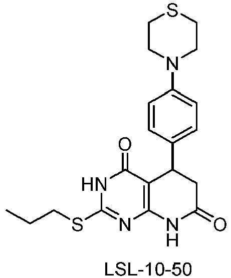

2-propylmercapto-6-aminopyrimidin-4(*3H*)-one (185 mg, 1.0 mmol) and 4-thiomorpholinobenzaldehyde (207 mg, 1.0 mmol) reacted at 170 °C for about 2 hours. After separation and purification by a flash column (dichloromethane/methanol: 1.5% to 2.5%), 89 mg of pure product was obtained with a yield of 21.4%.
**¹HNMR (DMSO-*d*₆, 400 MHz) δ (ppm)** 12.32(1H, s), 10.06(1H, s), 7.00(2H, d, *J* = 8.4 Hz), 6.79(2H, d, *J* = 8.4 Hz), 4.07(1H, d, *J* = 7.2 Hz), 3.42(4H, br s), 2.96-3.06(2H, m), 2.87(1H, q), 2.63(4H, br s), 2.45(1H, d, *J* = 16.4 Hz), 1.56-1.68(2H, m), 0.95(3H, t, *J* = 7.2 Hz);
**¹³CNMR (DMSO-*d*₆, 100 MHz) δ (ppm)** 171.22, 154.88, 149.75, 134.35, 127.76 × 2, 116.94 × 2, 98.72, 51.78 × 2, 39.02, 32.90, 31.86, 26.08 × 2, 23.08, 13.78;
**MS (ESI+) m/z** 417.1 [M+H] +.

### Example 71:

### Preparation of 2-propylmercapto-5-(benzofuran-5-yl)-5,8-dihydropyrido[2,3-d]pyrimidin-4,7(3H,6H)-dione (LSL-11-17)

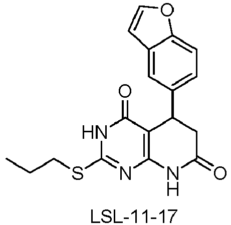

2-propylmercapto-6-aminopyrimidin-4(3*H*)-one (185 mg, 1.0 mmol) and benzofuran-5-carbaldehyde (146 mg, 1.0 mmol) reacted at 180°C for about 2 hours. After separation and purification by a flash column (dichloromethane/methanol: 1.5% to 2.5%), 92 mg of pure product was obtained with a yield of 25.9%.
**¹HNMR (DMSO-*d*₆, 400 MHz) δ (ppm)** 12.59(1H, s), 10.57(1H, s), 7.95(1H, d, *J* = 2.0 Hz), 7.52(1H, d, *J* = 8.4 Hz), 7.36(1H, d, *J* = 1.6 Hz), 7.17(1H, dd, *J* = 8.4, 2.0 Hz), 6.92(1H, dd, *J* = 2.0, 1.2 Hz), 4.31(1H, d, *J* = 7.6 Hz), 3.05-3.16(3H, m), 2.57(1H, d, *J* = 16.4 Hz), 1.63-1.73(2H, m), 0.97(3H, t, *J* = 7.2 Hz);
**¹³CNMR (DMSO-*d*₆, 100 MHz) δ (ppm)** 170.99, 153.27, 146.82, 137.68, 127.80, 123.76, 118.95, 111.86, 107.19, 39.12, 33.42, 31.97, 22.84, 13.64;
**MS (ESI+) m/z** 356.1 [M+H] +.

### Example 72:

### Preparation of 2-propylmercapto-5-(benzothiazol-2-yl)-5,8-dihydropyrido[2,3-d]pyrimidin-4,7(3H,6H)-dione (LSL-11-18)

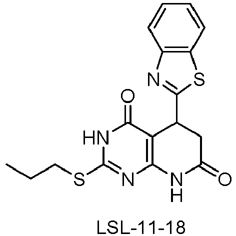

2-propylmercapto-6-aminopyrimidin-4(3*H*)-one (185 mg, 1.0 mmol) and benzothiazine-2-carbaldehyde (163 mg, 1.0 mmol) reacted at 170°C for about 2 hours. After separation and purification by a flash column (dichloromethane/methanol: 1.5% to 2.5%), 87 mg of pure product was obtained with a yield of 23.3%.
**¹HNMR (DMSO-*d*₆, 400 MHz) δ (ppm)** 12.65(1H, s), 10.65(1H, s), 7.86(2H, d, *J* = 8.4 Hz), 7.43(2H, d, *J* = 8.4 Hz), 4.32(1H, d, *J* = 7.6 Hz), 3.05-3.17(3H, m), 2.56(1H, d, *J* = 16.4 Hz), 1.63-1.73(2H, m), 0.98(3H, t, *J* = 7.2 Hz);
**¹³CNMR (DMSO-*d*₆, 100 MHz) δ (ppm)** 170.59, 149.02, 139.76, 127.99 × 2, 127.94 × 2, 44.01, 38.11, 33.60, 32.00, 22.81, 13.63;
**MS (ESI+) m/z** 373.1 [M+H] +.

### Example 73:

### Preparation of 2-propylmercapto-5-(4-phenylthiophenyl)-5,8-dihydropyrido[2,3-d]pyrimidin-4,7(3H,6H)-dione (LSL-11-19)

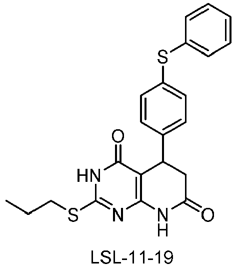

2-propylmercapto-6-aminopyrimidin-4(*3H*)-one (185 mg, 1.0 mmol) and 4-phenylthiobenzaldehyde (214 mg, 1.0 mmol) reacted at 170°C for about 2 hours. After separation and purification by a flash column (dichloromethane/methanol: 1.5% to 2.5%), 80 mg of pure product was obtained with a yield of 18.9%.
**¹HNMR (DMSO-*d*₆, 400 MHz) δ (ppm)** 12.65(1H, s), 10.44(1H, s), 7.34-7.38(2H, m), 7.26-7.30(5H, m), 7.18(2H, d, *J* = 8.4 Hz), 4.20(1H, d, *J* = 7.6 Hz), 3.02-3.13(2H, m), 3.02(1H, q), 2.52(1H, d, *J* = 16.4 Hz), 1.61-1.70(2H, m), 0.97(3H, t, *J* = 7.2 Hz);
**¹³CNMR (DMSO-*d*₆, 100 MHz) δ (ppm)** 170.86, 155.22, 142.95, 135.63, 132.91, 131.86 × 2, 130.71 × 2, 129.99 × 2, 128.29 × 2, 127.65, 98.27, 38.46, 33.25, 31.94, 22.92, 13.69;
**MS (ESI+) m/z** 424.1 [M+H] +.

### Example 74:

### Preparation of 2-propylmercapto-5-(4-methanesulfonylphenyl)-5,8-dihydropyrido[2,3-d]pyiimidin-4,7(3H,6H)-dione (LSL-11-20)

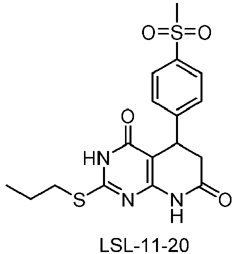

2-propylmercapto-6-aminopyrimidin-4(*3H*)-one (185 mg, 1.0 mmol) and 4-methanesulfonylbenzaldehyde (184 mg, 1.0 mmol) reacted at 170°C for about 2 hours. After separation and purification by a flash column (dichloromethane/methanol: 1.5% to 2.5%), 95 mg of pure product was obtained with a yield of 24.2%.
**¹HNMR (DMSO-*d*₆, 400 MHz) δ (ppm)** 12.78(1H, s), 10.31(1H, s), 7.83(2H, d, *J* = 8.0 Hz), 7.43(2H, d, *J* = 8.0 Hz), 4.30(1H, d, *J* = 8.0 Hz), 3.17(3H, s), 2.98-3.09(3H, m), 2.54(1H, d, *J* = 16.4 Hz), 1.59-1.69(2H, m), 0.97(3H, t, *J* = 7.6 Hz);
**¹³CNMR (DMSO-*d*₆, 100 MHz) δ (ppm)** 170.68, 155.34, 149.95, 139.46, 128.05 × 2, 127.74 × 2, 97.43, 44.07, 38.34, 33.89, 31.91, 23.03, 13.75;
**MS (ESI+) m/z** 394.1 [M+H] +.

### Example 75:

### Preparation of 2-propylmercapto-5-(4-methylthiophenyl)-5,8-dihydropyrido[2,3-d]pyrimidin-4,7(3H,6H)-dione (LSL-11-21)

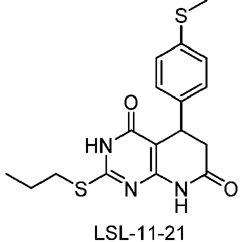

2-propylmercapto-6-aminopyrimidin-4(3*H*)-one (185 mg, 1.0 mmol) and 4-methylthiobenzaldehyde (152 mg, 1.0 mmol) reacted at 170 °C for about 2 hours. After separation and purification by a flash column (dichloromethane/methanol: 1.5% to 2.5%), 94 mg of pure product was obtained with a yield of 26.0%.
**¹HNMR (DMSO-*d*₆, 400 MHz) δ (ppm)** 12.59(1H, s), 10.56(1H, s), 7.19(2H, d, *J* = 8.4 Hz), 7.10(2H, d, *J* = 8.4 Hz), 4.17(1H, d, *J* = 7.6 Hz), 3.06-3.16(2H, m), 3.03(1H, q), 2.50(1H, d, *J* = 16.0 Hz), 2.43(3H, s), 1.63-1.72(2H, m), 0.96(3H, t, *J* = 7.2 Hz);
**¹³CNMR (DMSO-*d*₆, 100 MHz) δ (ppm)** 170.92, 139.71, 136.73, 127.55 × 2, 126.91 × 2, 38.47, 32.97, 31.97, 22.84, 15.35, 13.63;
**MS (ESI+) m/z** 362.1 [M+H] +.

### Example 76:

### Preparation of 2-propylmercapto-5-(3-nitro-4-bromophenyl)-5,8-dihydropyrido[2,3-d]pyiimidin-4,7(3H,6H)-dione (LSL-11-22)

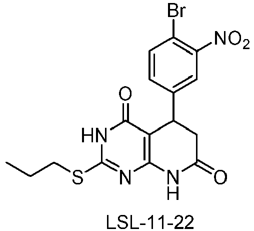

2-propylmercapto-6-aminopyrimidin-4(*3H*)-one (185 mg, 1.0 mmol) and 3-nitro-4-bromobenzaldehyde (229 mg, 1.0 mmol) reacted at 170°C for about 2 hours. After separation and purification by a flash column (dichloromethane/methanol: 1.5% to 2.5%), 115 mg of pure product was obtained with a yield of 26.2%.
**¹HNMR (DMSO-*d*₆, 400 MHz) δ (ppm)** 12.86(1H, s), 10.67(1H, s), 7.85(1H, d, *J* = 2.0 Hz), 7.84(1H, d, *J* = 8.4 Hz), 7.36(1H, dd, *J* = 8.4, 2.0 Hz), 4.30(1H, d, *J* = 7.2 Hz), 3.05-3.18(3H, m), 2.59(1H, d, *J* = 16.0 Hz), 1.63-1.72(2H, m), 0.98(3H, t, *J* = 7.2 Hz);
**¹³CNMR (DMSO-*d*₆, 100 MHz) δ (ppm)** 170.38, 150.22, 144.73, 135.40, 132.28, 124.24, 111.53, 37.87, 32.97, 32.00, 22.80, 13.63;
**MS (ESI+) m/z** 440.1 [M+H] +.

### Example 77:

### Preparation of 2-propylmercapto-5-(4-carboxyphenyl)-5,8-dihydropyrido[2,3-d]pyrimidin-4,7(3H,6H)-dione (LSL-11-24)

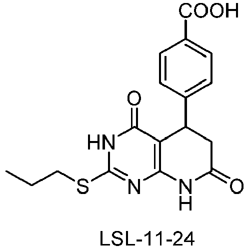

2-propylmercapto-6-aminopyrimidin-4(3*H*)-one (185 mg, 1.0 mmol) and 4-carboxybenzaldehyde (150 mg, 1.0 mmol) reacted at 170°C for about 2 hours. After separation and purification by a flash column (dichloromethane/methanol: 1.5% to 2.5%), 74 mg of pure product was obtained with a yield of 20.6%.
**¹HNMR (DMSO-*d*₆, 400 MHz) δ (ppm)** 12.72(2H, s), 10.61(1H, s), 7.87(2H, d, *J* = 8.4 Hz), 7.28(2H, d, *J* = 8.4 Hz), 4.28(1H, d, *J* = 8.0 Hz), 3.06-3.18(3H, m), 2.55(1H, d, *J* = 16.0 Hz), 1.63-1.73(2H, m), 0.98(3H, t, *J* = 7.2 Hz);
**¹³CNMR (DMSO-*d*₆, 100 MHz) δ (ppm)** 170.72, 167.53, 148.11, 130.22 × 2, 129.80, 127.21 × 2, 38.26, 33.60, 31.99, 22.82, 13.64;
**MS (ESI+) m/z** 360.1 [M+H] +.

### Example 78:

### Preparation of 2-propylmercapto-5-(2-nitro-4-bromophenyl)-5,8-dihydropyrido[2,3-d]pyrimidin-4,7(3H,6H)-dione (LSL-11-25)

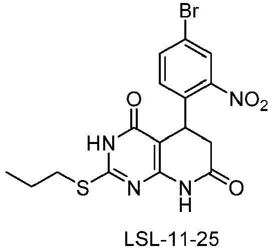

2-propylmercapto-6-aminopyrimidin-4(3*H*)-one (185 mg, 1.0 mmol) and 2-nitro-4-bromobenzaldehyde (229 mg, 1.0 mmol) reacted at 170°C for about 2 hours. After separation and purification by a flash column (dichloromethane/methanol: 1.5% to 2.5%), 83 mg of pure product was obtained with a yield of 18.9%.
**¹HNMR (DMSO-*d*₆, 400 MHz) δ (ppm)** 12.64(1H, s), 10.78(1H, s), 8.19(1H, d, *J* = 2.0 Hz), 7.84(1H, dd, *J* = 8.4, 2.0 Hz), 7.10(1H, d, *J* = 8.4 Hz),4.50(1H, d, *J* = 8.0 Hz), 3.26(1H, q), 3.06-3.20(2H, m), 2.47(1H, d, *J* = 16.0 Hz), 1.64-1.74(2H, m), 0.99(3H, t, *J* = 7.2 Hz);
**¹³CNMR (DMSO-*d*₆, 100 MHz) δ (ppm)** 169.81, 149.71, 137.05, 136.48, 130.46, 127.72, 120.59, 37.69, 32.03, 29.75, 22.80, 13.65;
**MS (ESI+) m/z** 440.1 [M+H] +.

### Example 79:

### Preparation of 2-propylmercapto-5-(pyridin-2-yl)-5,8-dihydropyrido[2,3-d]pyrimidin-4,7(3H,6H)-dione (LSL-11-26)

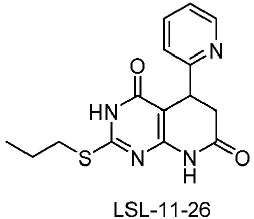

2-propylmercapto-6-aminopyrimidin-4(*3H*)-one (185 mg, 1.0 mmol) and pyridin-2-carbaldehyde (107 mg, 1.0 mmol) reacted at 170 °C for about 2 hours. After separation and purification by a flash column (dichloromethane/methanol: 1.5% to 2.5%), 86 mg of pure product was obtained with a yield of 27.2%.
**¹HNMR (DMSO-*d*₆, 400 MHz) δ (ppm)** 12.34(1H, s), 10.29(1H, s), 8.41(1H, d, *J* = 3.6 Hz), 7.68(1H, td, *J* = 7.6, 2.0 Hz), 7.29(1H, d, *J* = 7.6 Hz), 7.16-7.22(1H, m), 4.25(1H, d, *J* = 7.6 Hz), 3.02-3.09(2H, m), 2.92(1H, q), 2.65(1H, d, *J* = 16.4 Hz), 1.59-1.68(2H, m), 0.95(3H, t, *J* = 7.2 Hz);
**¹³CNMR (DMSO-*d*₆, 100 MHz) δ (ppm)** 170.93, 162.17, 149.54, 137.15, 122.57, 122.14, 36.23, 35.96, 31.92, 22.88, 13.63;
**MS (ESI+) m/z** 317.1 [M+H] +.

### Example 80:

### Preparation of 2-propylmercapto-5-(4-isobutoxyphenyl)-5,8-dihydropyrido[2,3-d]pyiimidin-4,7(3H,6H)-dione (LSL-11-27)

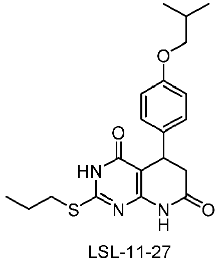

2-propylmercapto-6-aminopyrimidin-4(3*H*)-one (185 mg, 1.0 mmol) and 4-isobutoxybenzaldehyde (178 mg, 1.0 mmol) reacted at 170°C for about 1 hour. After separation and purification by a flash column (dichloromethane/methanol: 1.5% to 2.5%), 90 mg of pure product was obtained with a yield of 23.3%.
**¹HNMR (DMSO-*d*₆, 400 MHz) δ (ppm)** 12.34(1H, s), 10.37(1H, s), 7.05(2H, d, *J* = 8.4 Hz), 6.82(2H, d, *J* = 8.4 Hz), 4.13(1H, d, *J* = 7.2 Hz), 3.68(2H, d, *J* = 6.4 Hz), 3.02-3.13(2H, m), 2.96(1H, q), 2.49(1H, d, *J* = 16.0 Hz), 1.92-2.03(1H, m), 1.61-1.70(2H, m), 0.97(3H, t, *J* = 7.2 Hz), 0.96(3H, s), 0.95(3H, s);
**¹³CNMR (DMSO-*d*₆, 100 MHz) δ (ppm)** 171.07, 157.96, 155.03, 135.09, 129.99, 127.97 × 2, 114.94 × 2, 114.12, 99.02, 74.19, 38.86, 32.74, 31.92, 28.14, 22.93, 19.52 × 2, 13.68;
**MS (ESI+) m/z** 388.1 [M+H] +.

### Example 81:

### Preparation of 2-propylmercapto-5-(pyridin-3-yl)-5,8-dihydropyrido[2,3-d]pyrimidin-4,7(3H,6H)-dione (LSL-11-28)

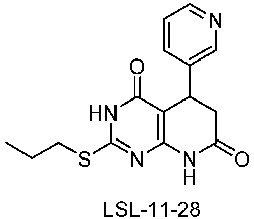

2-propylmercapto-6-aminopyrimidin-4(*3H*)-one (185 mg, 1.0 mmol) and pyridin-3-carbaldehyde (107 mg, 1.0 mmol) were heated to react at 200 °C for about 6 hours. After separation and purification by a flash column (dichloromethane/methanol: 60%), 104 mg of pure product was obtained with a yield of 27.2%.
**¹HNMR (DMSO-*d*₆, 400 MHz) δ (ppm)** 12.61(1H, s), 10.65(1H, s), 8.43(2H, s), 7.52(1H, d, *J* = 7.6 Hz), 7.30-7.34(1H, m), 4.26(1H, d, *J* = 7.6 Hz), 3.07-3.18(3H, m), 2.57(1H, d, *J* = 16.4 Hz), 1.63-1.72(2H, m), 0.98(3H, t, *J* = 7.2 Hz);
**¹³CNMR (DMSO-*d*₆, 100 MHz) δ (ppm)** 170.68, 162.04, 155.25, 148.60, 148.51, 138.49, 134.43, 124.23, 97.99, 38.04, 32.00, 31.40, 22.82, 13.63;
**MS (ESI+) m/z** 317.1 [M+H] +.

### Example 82:

### Preparation of 2-propylmercapto-5-(pyridin-4-yl)-5,8-dihydropyrido[2,3-d]pyrimidin-4,7(3H,6H)-dione (LSL-11-29)

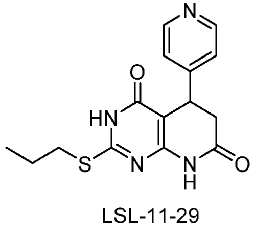

2-propylmercapto-6-aminopyrimidin-4(*3H*)-one (185 mg, 1.0 mmol) and pyridin-4-carbaldehyde (107 mg, 1.0 mmol) were heated to react at 170 °C for about 1 hour. After separation and purification by a flash column (dichloromethane/methanol: 1.5% to 2.5%), 80 mg of pure product was obtained with a yield of 25.3%.
**¹HNMR (DMSO-*d*₆, 400 MHz) δ (ppm)** 12.63(1H, s), 10.64(1H, s), 8.47(2H, d, *J* = 5.6 Hz), 7.18(2H, d, *J* = 5.6 Hz), 4.22(1H, d, *J* = 7.6 Hz), 3.06-3.18(3H, m), 2.57(1H, d, *J* = 16.4 Hz), 1.63-1.72(2H, m), 0.98(3H, t, *J* = 7.2 Hz);
**¹³CNMR (DMSO-*d*₆, 100 MHz) δ (ppm)** 170.58, 151.64, 150.40 × 2, 122.38 × 2, 97.45, 37.51, 32.97, 32.00, 22.82, 13.63;
**MS (ESI+) m/z** 317.1 [M+H] +.

### Example 83:

### Preparation of 2-propylmercapto-5-cyclohexyl-5,8-dihydropyrido[2,3-d]pyrimidin-4,7(3H,6H)-dione (LSL-11-30)

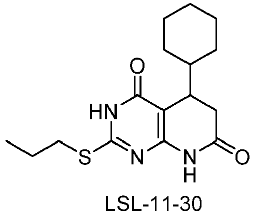

2-propylmercapto-6-aminopyrimidin-4(*3H*)-one (185 mg, 1.0 mmol) and cyclohexanecarbaldehyde (112 mg, 1.0 mmol) reacted at 150 °C for about 1 hour. After separation and purification by a flash column (dichloromethane/methanol: 1.0% to 2.0%), 112 mg of pure product was obtained with a yield of 34.9%.
**¹HNMR (DMSO-*d*₆, 400 MHz) δ (ppm)** 12.46(1H, s), 10.29(1H, s), 3.02-3.14(2H, m), 2.77(1H, t, *J* = 6.8 Hz), 2.58(1H, q), 2.40(1H, d, *J* = 16.4 Hz), 1.60-1.70(4H, m), 1.52-1.58(2H, m), 1.34-1.48(2H, m), 1.10-1.17(2H, m), 1.02-1.10(2H, m), 0.97(3H, t, *J* = 7.2 Hz) , 0.80-0.90(1H, m);
**¹³CNMR (DMSO-*d*₆, 100 MHz) δ (ppm)** 171.95, 41.46, 33.89, 33.10, 31.89, 30.38, 28.84, 26.44, 26.40, 26.35, 22.84, 13.63;
**MS (ESI+) m/z** 322.1 [M+H] +.

### Example 84:

### Preparation of 2-propylmercapto-5-cyclopentyl-5,8-dihydropyrido[2,3-d]pyrimidin-4,7(3H,6H)-dione (LSL-11-31)

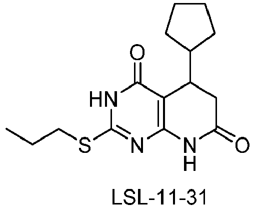

2-propylmercapto-6-aminopyrimidin-4(*3H*)-one (185 mg, 1.0 mmol) and cyclopentanecarbaldehyde (98 mg, 1.0 mmol) were heated to react at 150°C for about 1.5 hours. After separation and purification by a flash column (dichloromethane/methanol: 1.0% to 2.0%), 76 mg of pure product was obtained with a yield of 24.8%.
**¹HNMR (DMSO-*d*₆, 400 MHz) δ (ppm)** 12.46(1H, s), 10.34(1H, s), 3.02-3.14(2H, m), 2.84(1H, t, *J* = 7.2 Hz), 2.63(1H, q), 2.35(1H, d, *J* = 16.4 Hz), 1.76-1.84(1H, m), 1.62-1.68(3H, m), 1.50-1.59(2H, m),1.38-1.44(3H, m), 1.27-1.37(1H, m), 1.08-1.14(1H, m), 0.97(3H, t, *J* = 7.2 Hz);
**¹³CNMR (DMSO-*d*₆, 100 MHz) δ (ppm)** 171.82, 43.92, 35.53, 32.42, 31.89, 30.05, 29.84, 24.98, 24.86, 22.84, 13.63;
**MS (ESI+) m/z** 308.1 [M+H] +.

### Example 85:

### Preparation of 2-propylmercapto-5-(1,4-benzodioxan-6-yl)-5,8-dihydropyrido[2,3-d]pyrimidin-4,7(3H,6H)-dione (LSL-11-32)

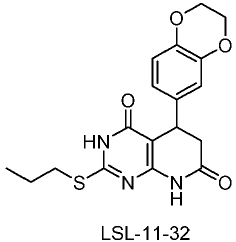

2-propylmercapto-6-aminopyrimidin-4(*3H*)-one (185 mg, 1.0 mmol) and 1,4-benzodioxan-6-carbaldehyde (164 mg, 1.0 mmol) reacted at 170°C for about 2 hours. After separation and purification by a flash column (dichloromethane/methanol: 1.0% to 2.0%), 78 mg of pure product was obtained with a yield of 20.9%.
**¹HNMR (DMSO-*d*₆, 400 MHz) δ (ppm)** 12.46(1H, s), 10.42(1H, s), 6.75(1H, d, *J* = 8.8 Hz), 6.60-6.63(2H, m), 4.18(4H, s), 4.09(1H, t, *J* = 7.2 Hz), 3.04-3.13(2H, m), 2.95(1H, q), 2.48(1H, d, *J* = 16.4 Hz), 1.61-1.71(2H, m), 0.97(3H, t, *J* = 7.2 Hz);
**¹³CNMR (DMSO-*d*₆, 100 MHz) δ (ppm)** 171.05, 155.02, 143.59, 142.57, 136.20, 119.62, 117.47, 115.47, 98.98, 64.53, 64.43, 38.63, 32.75, 31.94, 22.90, 13.66;
**MS (ESI+) m/z** 374.1 [M+H] +.

### Example 86:

### Preparation of 2-propylmercapto-5-(2,3-dihydrobenzofuran-5-yl)-5,8-dihydropyrido[2,3-d]pyrimidin-4,7(3H,6H)-dione (LSL-11-33)

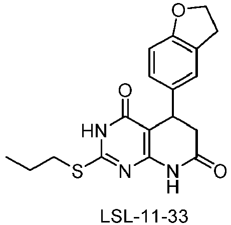

2-propylmercapto-6-aminopyrimidin-4(*3H*)-one (185 mg, 1.0 mmol) and 2,3-dihydrobenzofuran-5-carbaldehyde (148 mg, 1.0 mmol) reacted at 170°C for about 2 hours. After separation and purification by a flash column (dichloromethane/methanol: 1.0% to 2.0%), 73 mg of pure product was obtained with a yield of 20.4%.
**¹HNMR (DMSO-*d*₆, 400 MHz) δ (ppm)** 12.51(1H, s), 10.47(1H, s), 7.00(1H, d, *J* = 1.2 Hz), 6.85(1H, dd, *J* = 8.0, 1.2 Hz), 6.65(1H, d, *J* = 8.0 Hz), 4.47(2H, t, *J* = 8.8 Hz), 4.12(1H, d, *J* = 7.6 Hz), 3.11(2H, t, *J* = 8.8 Hz), 3.04-3.15(2H, m), 2.98(1H, q), 2.48(1H, d, *J* = 16.4 Hz), 1.62-1.72(2H, m), 0.98(3H, t, *J* = 7.2 Hz);
**¹³CNMR (DMSO-*d*₆, 100 MHz) δ (ppm)** 171.08, 158.97, 134.94, 128.03, 126.29, 123.56, 109.18, 71.34, 38.99, 32.96, 31.95, 29.57, 22.85, 13.65;
**MS (ESI+) m/z** 358.1 [M+H] +.

### Example 87:

### Preparation of 2-propylmercapto-5-(quinoxalin-6-yl)-5,8-dihydropyrido[2,3-d]pyrimidin-4,7(3H,6H)-dione (LSL-11-34)

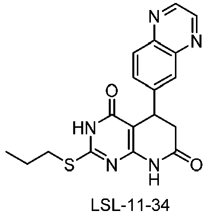

2-propylmercapto-6-aminopyrimidin-4(3*H*)-one (185 mg, 1.0 mmol) and quinoxalin-6-carbaldehyde (174 mg, 1.0 mmol) reacted at 170 °C for about 2 hours. After separation and purification by a flash column (dichloromethane/methanol: 1.5% to 2.5%), 104 mg of pure product was obtained with a yield of 28.3%.
**¹HNMR (DMSO-*d*₆, 400 MHz) δ (ppm)** 12.68(1H, s), 10.69(1H, s), 8.90-8.92(2H, m), 8.07(1H, d, *J* = 8.8 Hz), 7.81(1H, dd, *J* = 8.8, 2.0 Hz), 7.71(1H, d, *J* = 2.0 Hz), 4.49(1H, d, *J* = 8.0 Hz), 3.18(1H, q), 3.07-3.17(2H, m), 2.72(1H, d, *J* = 16.4 Hz), 1.64-1.73(2H, m), 0.98(3H, t, *J* = 7.2 Hz);
**¹³CNMR (DMSO-*d*₆, 100 MHz) δ (ppm)** 170.79, 146.36, 145.95, 145.29, 142.61, 141.85, 130.51, 130.11, 125.78, 38.02, 33.56, 32.02, 22.82, 13.63;
**MS (ESI+) m/z** 368.1 [M+H] +.

### Example 88:

### Preparation of 2-propylmercapto-5-(6-methoxypyridin-3-yl)-5,8-dihydropyrido[2,3-d]pyrimidin-4,7(3H,6H)-dione (LSL-11-35)

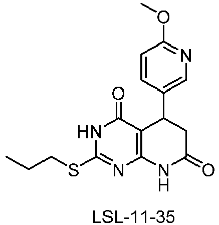

2-propylmercapto-6-aminopyrimidin-4(*3H*)-one (185 mg, 1.0 mmol) and 6-methoxypyridin-3-carbaldehyde (137 mg, 1.0 mmol) reacted at 180°C for about 1 hour. After separation and purification by a flash column (dichloromethane/methanol: 1.5% to 2.5%), 85 mg of pure product was obtained with a yield of 24.6%.
**¹HNMR (DMSO-*d*₆, 400 MHz) δ (ppm)** 12.68(1H, s), 10.40(1H, s), 7.93(1H, d, *J* = 2.4 Hz), 7.47(1H, dd, *J* = 8.8, 2.4 Hz), 6.73(1H, d, *J* = 2.4 Hz), 4.17(1H, d, *J* = 7.2 Hz), 3.79(3H, s), 3.01-3.11(2H, m), 2.99(1H, q), 2.51(1H, d, *J* = 16.4 Hz), 1.59-1.69(2H, m), 0.96(3H, t, *J* = 7.2 Hz);
**¹³CNMR (DMSO-*d*₆, 100 MHz) δ (ppm)** 170.85, 162.94, 155.09, 145.01, 138.07, 131.82, 110.86, 98.17, 53.51, 38.34, 31.93, 30.70, 22.95, 13.69;
**MS (ESI+) m/z** 347.1 [M+H] +.

### Example 89:

### Preparation of 2-propylmercapto-5-(quinolin-3-yl)-5,8-dihydropyrido[2,3-d]pyrimidin-4,7(3H,6H)-dione (LSL-11-36)

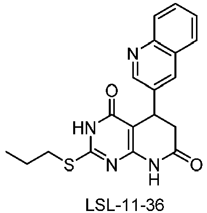

2-propylmercapto-6-aminopyrimidin-4(*3H*)-one (185 mg, 1.0 mmol) and quinolin-3-carbaldehyde (157 mg, 1.0 mmol) reacted at 170°C for about 2 hours. After separation and purification by a flash column (dichloromethane/methanol: 1.5% to 2.5%), 91 mg of pure product was obtained with a yield of 24.9%.
**¹HNMR (DMSO-*d*₆, 400 MHz) δ (ppm)** 12.61(1H, s), 10.68(1H, s), 8.87(1H, d, *J* = 2.4 Hz), 7.98(1H, t, *J* = 8.0 Hz), 7.94(2H, s), 7.72(1H, td, *J* = 8.0, 2.0 Hz), 7.58(1H, t, *J* = 7.6 Hz), 4.47(1H, d, *J* = 7.6 Hz), 3.07-3.22(3H, m), 2.71(1H, d, *J* = 16.0 Hz), 1.64-1.74(2H, m), 0.99(3H, t, *J* = 7.2 Hz);
**¹³CNMR (DMSO-*d*₆, 100 MHz) δ (ppm)** 170.64, 151.22, 147.10, 135.92, 132.32, 129.66, 129.04, 128.40, 127.88, 127.31, 38.08, 32.03, 31.63, 22.83, 13.66;
**MS (ESI+) m/z** 367.1 [M+H] +.

### Example 90:

### Preparation of 2-propylmercapto-5-(furan-3-yl)-5,8-dihydropyrido[2,3-d]pyrimidin-4,7(3H,6H)-dione (LSL-11-38)

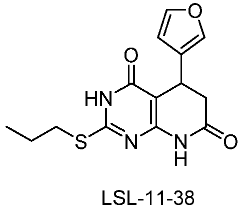

2-propylmercapto-6-aminopyrimidin-4(*3H*)-one (185 mg, 1.0 mmol) and furan-3-carbaldehyde (96 mg, 1.0 mmol) reacted at 160 °C for about 2 hours. After separation and purification by a flash column (dichloromethane/methanol: 1.5% to 2.5%), 85 mg of pure product was obtained with a yield of 27.9%.
**¹HNMR (DMSO-*d*₆, 400 MHz) δ (ppm)** 12.49(1H, s), 10.42(1H, s), 7.55(1H, s), 7.30(1H, s), 6.34(1H, s), 4.06(1H, d, *J* = 7.2 Hz), 3.02-3.12(2H, m), 2.90(1H, q), 2.57(1H, d, *J* = 16.0 Hz), 1.60-1.71(2H, m), 0.96(3H, t, *J* = 7.2 Hz);
**¹³CNMR (DMSO-*d*₆, 100 MHz) δ (ppm)** 171.09, 154.61, 144.07, 139.00, 126.67, 110.37, 98.81, 37.16, 31.94, 25.24, 22.86, 13.65;
**MS (ESI+) m/z** 306.1 [M+H] +.

### Example 91:

### Preparation of 2-propylmercapto-5-(4-heptoxyphenyl)-5,8-dihydropyrido[2,3-d]pyrimidin-4,7(3H,6H)-dione (LSL-11-39)

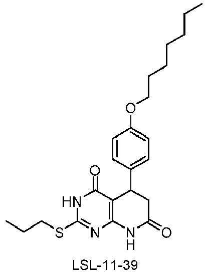

2-propylmercapto-6-aminopyrimidin-4(*3H*)-one (185 mg, 1.0 mmol) and 4-heptoxybenzaldehyde (220 mg, 1.0 mmol) reacted at 170°C for about 1 hour. After separation and purification by a flash column (dichloromethane/methanol: 1.5% to 2.5%), 107 mg of pure product was obtained with a yield of 24.9%.
**¹HNMR (DMSO-*d*₆, 400 MHz) δ (ppm)** 12.52(1H, s), 10.36(1H, s), 7.04(2H, d, *J* = 8.0 Hz), 6.81(2H, d, *J* = 8.0 Hz), 4.12(1H, d, *J* = 7.2 Hz), 3.89(2H, t, *J* = 7.2 Hz), 3.02-3.12(2H, m), 2.95(1H, q), 2.48(1H, d, *J* = 16.4 Hz), 1.57-1.74(4H, m), 1.18-1.44(8H, m), 0.96(3H, t, *J* = 7.2 Hz), 0.86(3H, t, *J* = 7.2 Hz);
**¹³CNMR (DMSO-*d*₆, 100 MHz) δ (ppm)** 170.62, 157.40, 134.59, 127.51 × 2, 114.43 × 2, 98.63, 67.35, 38.39, 32.29, 31.47, 31.25, 28.70, 28.44, 25.51, 22.48, 22.06, 13.97, 13.23;
**MS (ESI+) m/z** 430.1 [M+H] +.

### Experimental Example 1: Bioactivity Test

### Bacteriostasis Experiment

The strain MTB H37Rv (ATCC 27294) is preserved in the bacterial immunity rooms of Beijing Chest Hospital, Capital Medical University & Beijing Tuberculosis and Thoracic Tumor Research Institute. The culture medium 7H9 and 7H10 and the additive OADC for culturing mycobacteria were purchased from BD.

MTB H37Rv preserved at -80°C was dipped using an inoculating loop and streaked on the solid culture medium 7H10 (containing 10% OADC), and stood for culture at 37°C until single colonies grew; and a single colony was chosen and inoculated in 5 mL of culture medium 7H9 (containing 10% OADC and 0.05% Tween80), and stood for culture for 10 to 14 days to late logarithmic phase.

Minimum inhibitory concentrations (MIC) of the compounds against Mtb H37Rv were determined in a 96-well plate using the two-fold dilution method. 200 µL of culture medium 7H9 was added to the first row and the eighth row to prevent drying. In the log phase, the strains were inoculated into the fresh culture medium 7H9 to a final concentration of OD600 of 0.005. The highest concentration of the tested samples were 64 µg/mL and the lowest concentration 0.125 µg/mL (three parallel samples). The solvent DMSO was the negative control, while INH (isonicotinyl hydrazide) was the positive control. The 96-well plate was put into an incubator at 37°C to stand for culture for about 10 days, and the growth of the strain was observed. Drug concentrations where there was no obvious change in the light absorption of the culture solution were the minimum inhibitory concentrations.

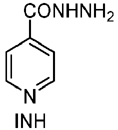

**Table 1: Inhibitory Activity Data of Part of Compounds against Mycobacterium Tuberculosis MIC Unit: µg/ml)**

| Compound | MTB H37Rv | Compound | MTB H37Rv | Compound | MTB H37Rv |
|---|---|---|---|---|---|
| LSL-7-30 | 0.5 | LSL-7-36 | 32 | LSL-7-38 | 1 |
| LSL-7-39 | 16 | LSL-7-42 | 32 | LSL-7-43 | >64 |
| LSL-7-44 | 16 | LSL-7-45 | 64 | LSL-7-46 | 8 |
| LSL-7-48 | 2 | LSL-7-49 | 64 | LSL-7-50 | 64 |
| LSL-9-01 | 8 | LSL-9-02 | 0.5 | LSL-9-06 | >64 |
| LSL-9-07 | 64 | LSL-9-08 | 64 | LSL-9-09 | 64 |
| LSL-9-10 | 4 | LSL-9-12 | 32 | LSL-9-14 | 1 |
| LSL-9-15 | 2 | LSL-9-16 | 2 | LSL-9-17 | 8 |
| LSL-9-19 | >64 | LSL-9-21 | 0.125 | LSL-9-23 | 64 |
| LSL-9-25 | 4 | LSL-9-26 | 2 | LSL-9-27 | >64 |
| LSL-9-28 | 8 | LSL-9-29 | 0.5 | LSL-9-30 | >64 |
| LSL-9-31 | 16 | LSL-9-32 | >64 | LSL-9-33 | 64 |
| LSL-9-35 | 64 | LSL-9-36 | 0.125 | LSL-9-37 | 2 |
| LSL-9-38 | 16 | LSL-9-39 | 0.5 | LSL-9-40 | 4 |
| LSL-9-41 | 0.125 | LSL-9-43 | 4 | LSL-9-44 | 16 |
| LSL-9-46 | 16 | LSL-9-47 | 4 | LSL-9-48 | 16 |
| LSL-9-49 | 16 | LSL-10-01 | 16 | LSL-10-02 | 6 |
| LSL-10-03 | 8 | LSL-10-04 | 0.625 | LSL-10-05 | 8 |
| LSL-10-06 | 4 | LSL-10-07 | 16 | LSL-10-08 | 8 |
| LSL-10-15 | 8 | LSL-10-19 | 0.625 | LSL-10-20 | 2 |
| LSL-10-21 | 4 | LSL-10-23 | 16 | LSL-10-24 | 2 |
| LSL-10-25 | 2 | LSL-10-38 | 2 | LSL-10-43 | 1 |
| LSL-10-47 | 2 | LSL-10-48 | 2 | LSL-10-50 | 1 |
| LSL-11-02 | 0.5 | LSL-11-08 | 0.25 | LSL-11-09 | 0.25 |
| LSL-11-17 | 4 | LSL-11-18 | 2 | LSL-11-19 | 8 |
| LSL-11-20 | 1 | LSL-11-21 | 0.5 | LSL-11-22 | 1 |
| LSL-11-24 | 2 | LSL-11-25 | 4 | LSL-11-26 | 2 |
| LSL-11-27 | 1 | LSL-11-28 | 2 | LSL-11-29 | 8 |
| LSL-11-30 | 16 | LSL-11-31 | 16 | LSL-11-32 | 2 |
| LSL-11-33 | 2 | LSL-11-34 | 4 | LSL-11-35 | 1 |
| LSL-11-36 | 8 | LSL-11-38 | 1 | LSL-11-39 | 0.625 |
| INH | 0.125-0.0625 | | | | |

It can be known from the data in Table 1 that the compounds of the present invention have good anti-tuberculosis activity. For example, the anti-mycobacterium tuberculosis MICs of the compounds LSL-7-30, LSL-9-02, LSL-9-21, LSL-9-29, LSL-9-36, LSL-9-39, LSL-9-41, LSL-10-04, LSL-10-19, LSL-11-02, LSL-11-08, LSL-11-09, LSL-11-21 and LSL-11-39, particularly LSL-9-21, LSL-9-36 and LSL-9-41, are equivalent to that of the positive control drug INH.

Finally, it should be noted that, the above examples are only used to illustrate the technical solutions of the present invention, but not to limit the same; although the present invention is described in detail with reference to the examples described above, it should be understood by those skilled in the art that, the technical solutions in the examples described above can still be modified, or some or all of the technical features can be equivalently replaced; and these modifications or replacements do not make the technical solutions corresponding thereto depart from the scope of the technical solutions in the examples of the present invention.

## Claims

1. A compound shown as formula I, an enantiomer, diastereomer, raceme or their mixture thereof, or a pharmaceutically acceptable salt thereof, 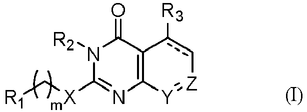
in the formula, R₁ is a saturated hydrocarbyl group, an unsaturated hydrocarbyl group, a carboxyl group, a hydroxyl group, a C₁-C₆ alkoxy group, a substituted or unsubstituted amino group, halogen, a nitro group, a cyano group, or a substituted or unsubstituted amide group, wherein a substituent in the substituted amino group is selected from hydrogen, a C₁-C₆ alkyl group, a carboxyl group, a hydroxyl group, an amino group, a C₁-C₆ alkoxy group, a nitro group, a cyano group, or a trifluoromethyl group;
m is 0, 1, or 2;
X is sulfur or oxygen;
if there is a single bond between Y and Z, Y is -CH₂- or -N(R)-, and R is selected from hydrogen, a saturated hydrocarbyl group, an unsaturated hydrocarbyl group, a carboxyl group, a hydroxyl group, a C1-C6 alkoxy group, a substituted or unsubstituted amino group, halogen,
a nitro group, a cyano group, or a substituted or unsubstituted amide group; Z is 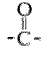 or -CH₂-; wherein the substituent in the substituted amino group
is selected from hydrogen, a C₁-C₆ alkyl group, a carboxyl group, a hydroxyl group, an amino group, a C₁-C₆ alkoxy group, a nitro group, a cyano group, or a trifluoromethyl group; if there is a double bond between Y and Z, Y is N and Z is CH;
R₂ is hydrogen, a saturated hydrocarbyl group, an unsaturated hydrocarbyl group, a carboxyl group, a hydroxyl group, a C₁-C₆ alkoxy group, a substituted or unsubstituted amino group, halogen, a nitro group, a cyano group, or a substituted or unsubstituted amide group; wherein a substituent in the substituted amino group is selected from hydrogen, a C₁-C₆ alkyl group, a carboxyl group, a hydroxyl group, an amino group, a C₁-C₆ alkoxy group, a nitro group, a cyano group, or a trifluoromethyl group;
R₃ is hydrogen, a substituted or unsubstituted C₁-C₆ alkyl group, a C₁-C₆ alkoxy group, a carboxyl group, a nitro group, halogen, a trifluoromethyl group, a cyano group, a protecting group-protected or -unprotected hydroxyl group, a protecting group-protected or -unprotected amino group, a substituted or unsubstituted phenyl group or fused ring group, or a substituted or unsubstituted heterocyclyl group; wherein a substituent in the substituted alkyl group is selected from a carboxyl group, a hydroxyl group, an amino group, halogen, a nitro group, a cyano group, or a mercapto group; wherein the substituted phenyl or fused ring group has 1, 2, 3, 4 or 5 substituents, the substituent being selected from hydrogen, a C₁-C₆ alkyl group, a carboxyl group, a hydroxyl group, an amino group, a C₁-C₆ alkoxy group, a nitro group, a cyano group, or a trifluoromethyl group; wherein the heterocyclyl group is a 3-membered, 4-membered or 5-membered heterocyclyl group; a heteroatom is O, S or N; if the heterocyclyl group has one or more substituents, the substituent is selected from hydrogen, a C₁-C₆ alkyl group, a carboxyl group, a hydroxyl group, an amino group, a C₁-C₆ alkoxy group, a nitro group, a cyano group, or a trifluoromethyl group; wherein the protecting group is selected from a *tert*-butoxycarbonyl group, a *p*-methoxybenzyl group, a dibenzyl group, a benzyl group, a *tert*-butyldimethylsilyl group, a *tert*-butyldiphenylsilyl group, an allyl group, a methoxymethyl group, a methylthiomethyl group, a methoxyethoxymethyl group, or a benzyloxymethyl group.

2. The compound, the enantiomer, diastereomer, raceme or their mixture thereof or the pharmaceutically acceptable salt thereof according to claim 1, **characterized in that**, the configuration of each chiral carbon in the compound shown as formula I is independently an R configuration or an S configuration.

3. The compound, the enantiomer, diastereomer, raceme or their mixture thereof or the pharmaceutically acceptable salt thereof according to claim 1, **characterized in that**, the pharmaceutically acceptable salt is hydrochloride, hydrobromide, sulfate, nitrate, phosphate, citrate, mesylate, trifluoroacetate, acetate, oxalate, succinate, malate, tosylate, tartrate, fumarate, glutamate, glucuronate, lactate, glutarate, arginine salt, or maleate.

4. The compound, the enantiomer, diastereomer, raceme or their mixture thereof or the pharmaceutically acceptable salt thereof according to claim 1, **characterized in that**,
in the formula, R₁ is a methyl group or a vinyl group;
m is 0, 1, or 2;
X is sulfur;
if there is a single bond between Y and Z, Y is -NH-; Z is 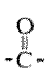 or -CH₂-; if there is a double bond between Y and Z, Y is N; Z is CH;
R₂ is hydrogen; and
R₃ is a 4-propoxyphenyl group, a 3-hydroxy-4-methoxyphenyl group, a 3,4-dihydroxyphenyl group, a 3,4,5-trimethoxyphenyl group, a 3,4-dimethoxyphenyl group, a 3-nitro-4-chlorophenyl group, a 4-nitrophenyl group, a 2-hydroxy-4-methoxyphenyl group, a 4-hydroxyphenyl group, a 2,6-dimethylphenyl group, a 3-nitrophenyl group, a 2,4-dihydroxyphenyl group, a 4-propoxyphenyl group, a 4-iodophenyl group, a 4-methoxyphenyl group, a 2,3-dihydroxy-4-methoxyphenyl group, a 3-hydroxy-4-propoxyphenyl group, a 4-bromophenyl group, a 4-fluorophenyl group, a 2-hydroxy-4-propoxyphenyl group, a 4-benzyloxyphenyl group, a 4-phenoxyphenyl group, a 3,4,5-trihydroxyphenyl group, a 4-trifluoromethoxyphenyl group, a 4-ethoxyphenyl group, a 4-methoxy-3-nitrophenyl group, a 4-methoxy-2-nitrophenyl group, a 3-methoxyphenyl group, a 2-methoxyphenyl group, a 3-hydroxyphenyl group, a 3-cyanophenyl group, a 2-nitrophenyl group, a 4-hydroxy-3-nitrophenyl group, a phenyl group, a quinolin-4-yl group, a quinolin-5-yl group, a quinolin-6-yl group, or a 4-methoxyphenyl group.

5. The compound, the enantiomer, diastereomer, raceme or their mixture thereof or the pharmaceutically acceptable salt thereof according to claim 1, **characterized in that**, the compound shown as formula I is:
2-propylmercapto-5-(4-propoxyphenyl)-5,8-dihydropyrido[2,3-*d*]pyrimidin-4,7(*3H,6H*)-dione;
2-methylmercapto-5-(4-propoxyphenyl)-5,8-dihydropyrido[2,3-*d*]pyrimidin-4,7(*3H,6H*)-dione;
2-propylmercapto-5-(3-hydroxy-4-methoxyphenyl)-5,8-dihydropyrido[2,3-*d*]pyrimidin-4,7(*3H*,*6H*)-dione;
2-methylmercapto-5-(3-hydroxy-4-methoxyphenyl)-5,8-dihydropyrido[2,3-*d*]pyrimidin-4,7(*3H*,*6H*)-dione;
2-propylmercapto-5-(3,4-dihydroxyphenyl)-5,8-dihydropyrido[2,3-*d*]pyrimidin-4,7(*3H,6H*)-dione;
2-methylmercapto-5-(3,4,5-trimethoxyphenyl)-5,8-dihydropyrido[2,3-*d*]pyrimidin-4,7(*3H,6H*)-dione;
+2-propylmercapto-5-(3,4,5-trimethoxyphenyl)-5,8-dihydropyrido[2,3-*d*]pyrimidin-4,7(*3H,6H*)-dione;
2-propylmercapto-5-(3,4-dimethoxyphenyl)-5,8-dihydropyrido[2,3-*d*]pyrimidin-4,7(*3H,6H*)-dione;
2-propylmercapto-5-(3-nitro-4-chlorophenyl)-5,8-dihydropyrido[2,3-*d*]pyrimidin-4,7(*3H,6H*)-dione;
2-methylmercapto-5-(4-nitrophenyl)-5,8-dihydropyrido[2,3-*d*]pyrimidin-4,7(*3H,6H*)-dione;
2-propylmercapto-5-(4-nitrophenyl)-5,8-dihydropyrido[2,3-*d*]pyrimidin-4,7(*3H,6H*)-dione;
2-methylmercapto-5-(2-hydroxy-4-methoxyphenyl)-5,8-dihydropyrido[2,3-*d*]pyrimidin-4,7(*3H,6H*)-dione;
2-propylmercapto-5-(2-hydroxy-4-methoxyphenyl)-5,8-dihydropyrido[2,3-*d*]pyrimidin-4,7(*3H,6H*)-dione;
2-propylmercapto-5-(4-hydroxyphenyl)-5,8-dihydropyrido[2,3-*d*]pyrimidin-4,7(*3H,6H*)-dione;
2-methylmercapto-5-(2,6-dimethylphenyl)-5,8-dihydropyrido[2,3-*d*]pyrimidin-4,7(*3H,6H*)-dione;
2-propylmercapto-5-(2,6-dimethylphenyl)-5,8-dihydropyrido[2,3-*d*]pyrimidin-4,7(*3H,6H*)-dione;
2-methylmercapto-5-(3-nitrophenyl)-5,8-dihydropyrido[2,3-*d*]pyrimidin-4,7(*3H,6H*)-dione;
2-propylmercapto-5-(3-nitrophenyl)-5,8-dihydropyrido[2,3-*d*]pyrimidin-4,7(*3H,6H*)-dione;
2-propylmercapto-5-(2,4-dihydroxyphenyl)-5,8-dihydropyrido[2,3-*d*]pyrimidin-4,7(*3H,6H*)-dione;
2-allylmercapto-5-(4-propoxyphenyl)-5,8-dihydropyrido[2,3-*d*]pyrimidin-4,7(*3H,6H*)-dione;
2-allylmercapto-5-(3-hydroxy-4-oxyphenyl)-5,8-dihydropyrido[2,3-*d*]pyrimidin-4,7(*3H,6H*)-dione;
2-allylmercapto-5-(2-hydroxy-4-propoxyphenyl)-5,8-dihydropyrido[2,3-*d*]pyrimidin-4,7(*3H,6H*)-dione;
2-allylmercapto-5-(3,4-dimethoxyphenyl)-5,8-dihydropyrido[2,3-*d*]pyrimidin-4,7(*3H,6H*)-dione;
2-propylmercapto-5-(4-iodophenyl)-5,8-dihydropyrido[2,3-*d*]pyrimidin-4,7(*3H,6H*)-dione;
2-propylmercapto-5-(4-methoxyphenyl)-5,8-dihydropyrido[2,3-*d*]pyrimidin-4,7(*3H,6H*)-dione;
2-propylmercapto-5-(2,3-dihydroxy-4-methoxyphenyl)-5,8-dihydropyrido[2,3-*d*]pyrimidin-4,7(*3H,6H*)-dione;
2-propylmercapto-5-(3-hydroxy-4-propoxyphenyl)-5,8-dihydropyrido[2,3-*d*]pyrimidin-4,7(*3H,6H*)-dione;
2-propylmercapto-5-(4-bromophenyl)-5,8-dihydropyrido[2,3-*d*]pyrimidin-4,7(*3H,6H*)-dione;
2-propylmercapto-5-(4-fluorophenyl)-5,8-dihydropyrido[2,3-*d*]pyrimidin-4,7(*3H,6H*)-dione;
2-propylmercapto-5-(2-hydroxy-4-propoxyphenyl)-5,8-dihydropyrido[2,3-*d*]pyrimidin-4,7(*3H,6H*)-dione;
2-propylmercapto-5-(4-benzyloxyphenyl)-5,8-dihydropyrido[2,3-*d*]pyrimidin-4,7(*3H,6H*)-dione;
2-propylmercapto-5-(4-phenoxyphenyl)-5,8-dihydropyrido[2,3-*d*]pyrimidin-4,7(*3H,6H*)-dione;
2-propylmercapto-5-(3,4,5-trihydroxyphenyl)-5,8-dihydropyrido[2,3-*d*]pyrimidin-4,7(*3H,6H*)-dione;
2-propylmercapto-5-(4-trifluoromethoxyphenyl)-5,8-dihydropyrido[2,3-*d*]pyrimidin-4,7(*3H,6H*)-dione;
2-propylmercapto-5-(quinolin-6-yl)-5,8-dihydropyrido[2,3-*d*]pyrimidin-4,7(*3H,6H*)-dione;
2-propylmercapto-5-(4-methylphenyl)-5,8-dihydropyrido[2,3-*d*]pyrimidin-4,7(*3H,6H*)-dione;
2-propylmercapto-5-(4-trifluoromethylphenyl)-5,8-dihydropyrido[2,3-*d*]pyrimidin-4,7(*3H,6H*)-dione;
2-propylmercapto-5-(4-ethoxyphenyl)-5,8-dihydropyrido[2,3-*d*]pyrimidin-4,7(*3H,6H*)-dione;
2-propylmercapto-5-(3-nitro-4-methoxyphenyl)-5,8-dihydropyrido[2,3-*d*]pyrimidin-4,7(*3H,6H*)-dione;
2-propylmercapto-5-(2-nitro-4-methoxyphenyl)-5,8-dihydropyrido[2,3-*d*]pyrimidin-4,7(*3H,6H*)-dione;
2-propylmercapto-5-(3-methoxyphenyl)-5,8-dihydropyrido[2,3-*d*]pyrimidin-4,7(*3H,6H*)-dione;
2-propylmercapto-5-(2-methoxyphenyl)-5,8-dihydropyrido[2,3-*d*]pyrimidin-4,7(*3H,6H*)-dione;
2-propylmercapto-5-(3-hydroxyphenyl)-5,8-dihydropyrido[2,3-*d*]pyrimidin-4,7(*3H,6H*)-dione;
2-propylmercapto-5-(3-cyanophenyl)-5,8-dihydropyrido[2,3-*d*]pyrimidin-4,7(*3H,6H*)-dione;
2-propylmercapto-5-(2-nitrophenyl)-5,8-dihydropyrido[2,3-*d*]pyrimidin-4,7(*3H,6H*)-dione;
2-propylmercapto-5-(3-nitro-4-hydroxyphenyl)-5,8-dihydropyrido[2,3-*d*]pyrimidin-4,7(*3H,6H*)-dione;
2-propylmercapto-5-phenyl-5,8-dihydropyrido[2,3-*d*]pyrimidin-4,7(*3H,6H*)-dione;
2-propylmercapto-5-(quinolin-4-yl)-5,8-dihydropyrido[2,3-*d*]pyrimidin-4,7(*3H,6H*)-dione;
2-propylmercapto-5-(quinolin-5-yl)-5,8-dihydropyrido[2,3-*d*]pyrimidin-4,7(*3H,6H*)-dione;
2-propylmercapto-5-(4-methoxyphenyl)-pyrido[2,3-*d*]pyrimidin-4(*3H*)-one;
2-propylmercapto-5-(quinolin-6-yl)-pyrido[2,3-*d*]pyrimidin-4(*3H*)-one;
2-propylmercapto-5-(4-methoxyphenyl)-pyrido[2,3-*d*]pyrimidin-4,7(*3H,6H*)-dione;
2-propylmercapto-5-(3-amino-4-methoxyphenyl)-5,8-dihydropyrido[2,3-*d*]pyrimidin-4,7(*3H,6H*)-dione;
2-propylmercapto-5-(3-amino-4-chlorophenyl)-5,8-dihydropyrido[2,3-*d*]pyrimidin-4,7(*3H,6H*)-dione;
2-propylmercapto-5-(4-aminophenyl)-5,8-dihydropyrido[2,3-*d*]pyrimidin-4,7(*3H*,*6H*)-dione;
2-propylmercapto-5-(3-aminophenyl)-5,8-dihydropyrido[2,3-*d*]pyrimidin-4,7(*3H,6H*)-dione;
2-propylmercapto-5-(2-amino-3-methoxyphenyl)-5,8-dihydropyrido[2,3-*d*]pyrimidin-4,7(*3H,6H*)-dione;
2-propylmercapto-5-(2-cyanophenyl)-5,8-dihydropyrido[2,3-*d*]pyrimidin-4,7(*3H,6H*)-dione;
2-propylmercapto-5-(4-cyanophenyl)-5,8-dihydropyrido[2,3-*d*]pyrimidin-4,7(*3H,6H*)-dione;
2-methylmercapto-5-(4-methoxyphenyl)-5,8-dihydropyrido[2,3-*d*]pyrimidin-4,7(*3H,6H*)-dione;
2-butylmercapto-5-(4-methoxyphenyl)-5,8-dihydropyrido[2,3-*d*]pyrimidin-4,7(*3H,6H*)-dione;
2-butylmercapto-5-(3-nitro-4-hydroxyphenyl)-5,8-dihydropyrido[2,3-*d*]pyrimidin-4,7(*3H,6H*)-dione;
2-butylmercapto-5-(4-propoxyphenyl)-5,8-dihydropyrido[2,3-*d*]pyrimidin-4,7(*3H,6H*)-dione;
2-butylmercapto-5-(3-hydroxy-4-methoxyphenyl)-5,8-dihydropyrido[2,3-*d*]pyrimidin-4,7(*3H,6H*)-dione;
2-butylmercapto-5-(quinolin-6-yl)-5,8-dihydropyrido[2,3-*d*]pyrimidin-4,7(*3H,6H*)-dione;
2-isopropylmercapto-5-(4-methoxyphenyl)-5,8-dihydropyrido[2,3-*d*]pyrimidin-4,7(*3H,6H*)-dione;
2-propylmercapto-5-(4-morpholinophenyl)-5,8-dihydropyrido[2,3-*d*]pyrimidin-4,7(*3H,6H*)-dione;
2-propylmercapto-5-(4-(4-methylpiperazine)phenyl)-5,8-dihydropyrido[2,3-d]pyrimidin-4,7(*3H*,*6H*)-dione;
2-propylmercapto-5-(4-(piperidin-1-yl)phenyl)-5, 8-dihydropyrido[2,3-d]pyrimidin-4,7(*3H,6H*)-dione;
2-propylmercapto-5-(4-thiomorpholinophenyl)-5,8-dihydropyrido[2,3-*d*]pyrimidin-4,7(*3H,6H*)-dione;
2-propylmercapto-5-(benzofuran-5-yl)-5,8-dihydropyrido[2,3-*d*]pyrimidin-4,7(*3H,6H*)-dione;
2-propylmercapto-5-(benzothiazol-2-yl)-5,8-dihydropyrido[2,3-*d*]pyrimidin-4,7(*3H,6H*)-dione;
2-propylmercapto-5-(4-phenylthiophenyl)-5,8-dihydropyrido[2,3-*d*]pyrimidin-4,7(*3H,6H*)-dione;
2-propylmercapto-5-(4-methanesulfonylphenyl)-5,8-dihydropyrido[2,3-*d*]pyrimidin-4,7(*3H,6H*)-dione;
2-propylmercapto-5-(4-methylthiophenyl)-5,8-dihydropyrido[2,3-*d*]pyrimidin-4,7(*3H,6H*)-dione;
2-propylmercapto-5-(3-nitro-4-bromophenyl)-5,8-dihydropyrido[2,3-*d*]pyrimidin-4,7(*3H,6H*)-dione;
2-propylmercapto-5-(4-carboxyphenyl)-5,8-dihydropyrido[2,3-*d*]pyrimidin-4,7(*3H,6H*)-dione;
2-propylmercapto-5-(2-nitro-4-bromophenyl)-5,8-dihydropyrido[2,3-*d*]pyrimidin-4,7(*3H,6H*)-dione;
2-propylmercapto-5-(pyridin-2-yl)-5,8-dihydropyrido[2,3-*d*]pyrimidin-4,7(*3H,6H*)-dione;
2-propylmercapto-5-(4-isobutoxyphenyl)-5,8-dihydropyrido[2,3-*d*]pyrimidin-4,7(*3H,6H*)-dione;
2-propylmercapto-5-(pyridin-3-yl)-5,8-dihydropyrido[2,3-*d*]pyrimidin-4,7(*3H,6H*)-dione;
2-propylmercapto-5-(pyridin-4-yl)-5,8-dihydropyrido[2,3-*d*]pyrimidin-4,7(*3H,6H*)-dione;
2-propylmercapto-5-cyclohexyl-5, 8-dihydropyrido[2,3-*d*]pyrimidin-4,7(3H, *6H*)-dione;
2-propylmercapto-5-cyclopentyl-5,8-dihydropyrido[2,3-*d*]pyrimidin-4,7(*3H,6H*)-dione;
2-propylmercapto-5-(1,4-benzodioxan-6-yl)-5,8-dihydropyrido[2,3-*d*]pyrimidin-4,7(*3H,6H*)-dione;
2-propylmercapto-5-(2,3-dihydrobenzofuran-5-yl)-5,8-dihydropyrido[2,3-d]pyrimidin-4,7(*3H,6H*)-dione;
2-propylmercapto-5-(quinoxalin-6-yl)-5,8-dihydropyrido[2,3-*d*]pyrimidin-4,7(*3H,6H*)-dione;
2-propylmercapto-5-(6-methoxypyridin-3-yl)-5,8-dihydropyrido[2,3-*d*]pyrimidin-4,7(*3H,6H*)-dione;
2-propylmercapto-5-(quinolin-3-yl)-5,8-dihydropyrido[2,3-*d*]pyrimidin-4,7(*3H,6H*)-dione;
2-propylmercapto-5-(furan-3-yl)-5,8-dihydropyrido[2,3-*d*]pyrimidin-4,7(*3H,6H*)-dione; or
2-propylmercapto-5-(4-heptoxyphenyl)-5,8-dihydropyrido[2,3-*d*]pyrimidin-4,7(*3H,6H*)-dione.

6. A preparation method for the compound according to claim 1, **characterized in that**, when Y is -N(R)- and Z is 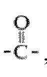
, the compound shown as formula I is: 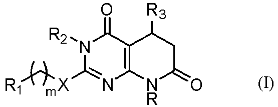
the method comprising the following steps: 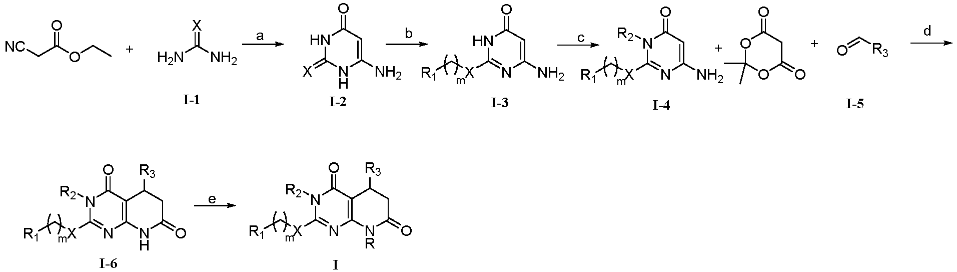
(a) ethyl cyanoacetate reacts with a compound of formula I-1 to give a compound of formula I-2;
(b) the compound of formula I-2 reacts with an R₁-substituted halide to give a compound of formula I-3;
(c) the compound of formula I-3 reacts with methanol, ethanol, haloalkane, unsaturated haloalkane, cyanogen bromide, *N*-chlorosuccinimide, *N*-bromosuccinimide, 1-cyano-1-methylethyl nitrate, urea, methyl isocyanate and chloramine to give a compound of formula I-4;
(d) the compound of formula I-4 reacts with 2,2-dimethyl-1,3-dioxane-4,6-dione and a compound of formula I-5 by a "one-pot" method to give a compound of formula I-6, wherein R₃ is hydrogen, a substituted or unsubstituted C₁-C₆ alkyl group, a carboxyl group, a nitro group, halogen, a trifluoromethyl group, a substituted or unsubstituted phenyl group or fused ring group, or a substituted or unsubstituted heterocyclyl group;
the compound obtained in step (d) is further subjected to hydroxylation, reduction, cyanation and cyan-hydrolysis reaction in sequence to give the compound of formula I-6, wherein R₃ is a hydroxyl group, an amino group, a cyano group, or an amide group;
(e) the compound of formula I-6 is subjected to substitution reaction to give the compound shown as formula I;
in the formula, X, m, R₁, R₂, R and substituents are defined as those in claim 1; or
the method comprises the following steps: 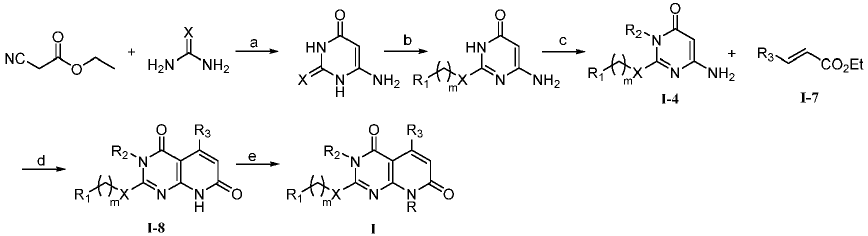
steps (a), (b) and (c) are the same as shown above, obtaining the compound of formula I-4 by reaction;
(d) the compound of formula I-4 reacts with a compound of formula I-7 to give a compound of formula I-8, wherein R₃ is hydrogen, a substituted or unsubstituted C₁-C₆ alkyl group, a carboxyl group, a nitro group, halogen, a trifluoromethyl group, a substituted or unsubstituted phenyl group or fused ring group, or a substituted or unsubstituted heterocyclyl group;
the compound obtained in step (d) is further subjected to hydroxylation, reduction, cyanation and cyanohydrolysis reaction in sequence to give the compound of formula I-8, wherein R₃ is a hydroxyl group, an amino group, a cyano group, or an amide group;
(e) the compound of formula I-8 is subjected to substitution reaction to give the compound shown as formula I;
in the formula, X, m, R₁, R₂, R and the substituents are defined as those in claim 1.

7. The preparation method for the compound of formula I according to claim 1, wherein if Y is N and Z is CH, the compound of formula I is: 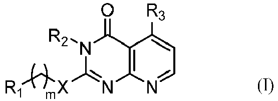
the method comprises the following steps: 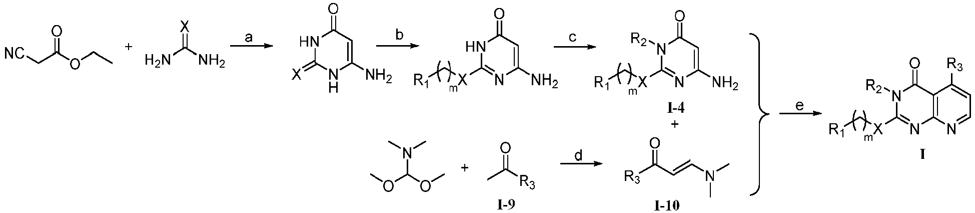
steps (a), (b) and (c) are described as claim 6, obtaining the compound of formula I-4 by reaction;
(d) *N,N*-dimethylformamide dimethyl acetal (DMFDMA) reacts with a compound of formula I-9 to give a compound of formula I-10;
(e) the compound of formula I-4 reacts with the compound of formula I-10 to give a compound of formula I, wherein R₃ is hydrogen, a substituted or unsubstituted C₁-C₆ alkyl group, a carboxyl group, a nitro group, halogen, a trifluoromethyl group, a substituted or unsubstituted phenyl ring group or fused ring group, or a substituted or unsubstituted heterocyclyl group;
the compound obtained in step (e) is further subjected to hydroxylation, reduction, cyanation and cyan-hydrolysis reaction in sequence to give the compound shown as formula I, wherein R₃ is a hydroxyl group, an amino group, a cyano group, or an amide group; and
in the formula, X, m, R₁, R₂, R and the substituents are defined as those in claim 1.

8. A pharmaceutical composition, **characterized in that**, the pharmaceutical composition comprising:
the compound, the enantiomer, diastereomer, raceme or their mixture thereof or the pharmaceutically acceptable salt thereof according to any one of claims 1 to 5; and a pharmaceutically acceptable carrier.

9. Use of the compound, the enantiomer, diastereomer, raceme or their mixture thereof or the pharmaceutically acceptable salt thereof according to any one of claims 1 to 5, **characterized in that**, used in
(1) the preparation of a drug for preventing and/or treating tuberculosis infection;
(2) the preparation of a drug for preventing and/or treating drug-resistant tuberculosis infection; or
(3) the preparation of a drug for inhibiting the growth of mycobacterium tuberculosis.

10. A method for decreasing the pathogenicity or toxicity of mycobacterium tuberculosis, **characterized in that**, comprises the following step:
mycobacterium tuberculosis is brought into contact with the compound, the enantiomer, diastereomer, raceme or their mixture thereof or the pharmaceutically acceptable salt thereof according to any one of claims 1 to 5 or the pharmaceutical composition according to claim 8, so as to decrease the pathogenicity or toxicity of mycobacterium tuberculosis.
